# EUROPEAN PATENT APPLICATION

(11) **EP 1 600 165 A1**
(43) Date of publication of application: **30.11.2005**
(21) Application number: 04717301.8
(22) Date of filing: 04.03.2004
(51) Int. Cl.: A61K 39/395, A61K 38/00, A61K 45/00, A61K 48/00, A61P 9/00, A61P 25/00, A61P 43/00, G01N 33/15, G01N 33/50

(54) **MEDICINAL USE OF MIP-3ALPHA INHIBITOR AND METHOD OF SCREENING BRAIN/NERVE CELL PROTECTIVE AGENT**

(30) Priority: 04.03.2003 JP 2003056885; 10.04.2003 JP 2003106247
(71) Applicant: Takeda Pharmaceutical Company Limited, Osaka 541-0045 (JP)
(72) Inventor: SHINTANI, Yasushi, c/o Takeda Pharm. Co.Ltd, Osaka-shi, Osaka 532-8686 (JP); OHTA, Hiroyuki, c/o Takeda Pharm. Co.Ltd, Osaka-shi, Osaka 532-8686 (JP); TERAO, Yasuko, c/o Takeda Pharm. Co.Ltd, Osaka-shi, Osaka 532-8686 (JP); KIYOTA, Yoshihiro, c/o Takeda Pharm. Co.Ltd, Osaka-shi, Osaka 532-8686 (JP)
(74) Representative: Lewin, John Harvey
(86) International application number: PCT/JP2004/002774
(87) International publication number: WO 2004/078208

(57) **Abstract**

The present invention provides a brain/nerve cell protective agent comprising a substance that reduces the expression and/or activity of MIP-3α, for example, an anti-MIP-3α antibody or an MIP-3α antisense nucleic acid, a diagnostic agent for a brain/nerve cell injury comprising a nucleic acid that encodes the antibody or MIP-3α^{,} a method for screening of a substance exhibiting brain/nerve cell protective action using MIP-3α and/or CCR6, a nucleic acid that encodes MIP-3α or CCR6, or an antibody against MIP-3α or CCR6, a kit therefor, and the like.

SEQUENCE LISTING

<110> Takeda Chemical Industries, Ltd.

<120> Pharmaceutical Use of MIP-3α Suppressing Substance and Screening for Brain or Nerve Cell
Protecting Substance

<130> 3158WOOP

<150> JP 2003-056885
<151> 2003-03-04

<150> JP 2003-106247
<151> 2003-04-10

<160> 21

<170> PatentIn version 3.1

<210> 1
<211> 288
<212> DNA
<213> Homo sapiens

<220>
<221> CDS
<222> (1)..(288)
<223>

<220>
<221> sig_peptide
<222> (1)..(78)
<223>

<220>
<221> mat_peptide
<222> (79)..()
<223>

<400> 1
atg tgc tgt acc aag agt ttg ctc ctg gct gct ttg atg tca gtg ctg 48Met Cys Cys Thr Lys Ser Leu Leu
Leu Ala Ala Leu Met Ser Val Leu -25 -20 -15
cta ctc cac ctc tgc ggc gaa tca gaa gca gca agc aac ttt gac tgc 96Leu Leu His Leu Cys Gly Glu
Ser Glu Ala Ala Ser Asn Phe Asp Cys -10 -5 -1 1 5
tgt ctt gga tac aca gac cgt att ctt cat cct aaa ttt att gtg ggc 144Cys Leu Gly Tyr Thr Asp
Arg Ile Leu His Pro Lys Phe Ile Val Gly 10 15 20
ttc aca cgg cag ctg gcc aat gaa ggc tgt gac atc aat gct atc atc 192Phe Thr Arg Gln Leu
Ala Asn Glu Gly Cys Asp Ile Asn Ala Ile Ile 25 30 35
ttt cac aca aag aaa aag ttg tct gtg tgc gca aat cca aaa cag act 240Phe His Thr Lys
Lys Lys Leu Ser Val Cys Ala Asn Pro Lys Gln Thr
40 45 50 tgg gtg aaa tat att gtg cgt ctc
ctc agt aaa aaa gtc aag aac atg 288Trp Val Lys Tyr Ile Val Arg Leu Leu Ser Lys Lys Val Lys Asn Met
55 60 65 70

<210> 2
<211> 96
<212> PRT
<213> Homo sapiens

<400> 2
Met Cys Cys Thr Lys Ser Leu Leu Leu Ala Ala Leu Met Ser Val Leu
-25 -20 -15
Leu Leu His Leu Cys Gly Glu Ser Glu Ala Ala Ser Asn Phe Asp Cys
-10 -5 -1 1 5
Cys Leu Gly Tyr Thr Asp Arg Ile Leu His Pro Lys Phe Ile Val Gly
10 15 20
Phe Thr Arg Gln Leu Ala Asn Glu Gly Cys Asp Ile Asn Ala Ile Ile
25 30 35
Phe His Thr Lys Lys Lys Leu Ser Val Cys Ala Asn Pro Lys Gln Thr
40 45 50
Trp Val Lys Tyr Ile Val Arg Leu Leu Ser Lys Lys Val Lys Asn Met
55 60 65 70

<210> 3
<211> 288
<212> DNA

<213>  Rattus norvegicus

<220>
<221>  CDS
<222>  (1)..(288)
<223>

<220>
<221>  sig_peptide
<222>  (1)..(75)
<223>

<220>
<221>  mat_peptide
<222>  (76)..()
<223>

<400>  3
atg gcc tgc aag cat ctg ccc ttc ctg gct ttg gcg ggg gta ctg ctg       48Met Ala Cys Lys His Leu Pro Phe
Leu Ala Leu Ala Gly Val Leu Leu          -25                    -20                    -15                    -10

gct tac ctc tgc agc cag tca gaa gca gca agc aac ttt gac tgc tgc       96Ala Tyr Leu Cys Ser Gln Ser Glu
Ala Ala Ser Asn Phe Asp Cys Cys                          -5                    -1  1                    5
     ctc acg tac aca aag aac gtg tat cat cat gcg aga aat ttt gtg ggt       144Leu Thr Tyr Thr Lys Asn Val
Tyr His His Ala Arg Asn Phe Val Gly                    10                    15                    20
          ttc aca aca cag atg gcc gac gaa gct tgt gac att aat gct atc atc       192Phe Thr Thr Gln Met Ala
Asp Glu Ala Cys Asp Ile Asn Ala Ile Ile                    25                    30                    35
               ttt cac ctg aag tcg aaa aga tcc gtg tgc gct gac cca aag cag atc       240Phe His Leu Lys Ser
Lys Arg Ser Val Cys Ala Asp Pro Lys Gln Ile          40                    45                    50
     55          tgg gtg aaa agg att ttg cac ctc ctc agc cta aga acc aag aag atg       288Trp Val Lys Arg
Ile Leu His Leu Leu Ser Leu Arg Thr Lys Lys Met                    60                    65
     70

<210>  4
<211>  96
<212>  PRT
<213>  Rattus norvegicus

<400>  4
Met Ala Cys Lys His Leu Pro Phe Leu Ala Leu Ala Gly Val Leu Leu
-25                 -20                 -15                 -10
Ala Tyr Leu Cys Ser Gln Ser Glu Ala Ala Ser Asn Phe Asp Cys Cys
                -5                  -1  1               5
Leu Thr Tyr Thr Lys Asn Val Tyr His His Ala Arg Asn Phe Val Gly
        10                  15                  20
Phe Thr Thr Gln Met Ala Asp Glu Ala Cys Asp Ile Asn Ala Ile Ile
    25                  30                  35
Phe His Leu Lys Ser Lys Arg Ser Val Cys Ala Asp Pro Lys Gln Ile
40                  45                  50                  55
Trp Val Lys Arg Ile Leu His Leu Leu Ser Leu Arg Thr Lys Lys Met
                60                  65                  70

<210>  5
<211>  291
<212>  DNA
<213>  Mus musculus

<220>
<221>  CDS
<222>  (1)..(291)
<223>

<220>
<221>  sig_peptide
<222>  (1)..(81)
<223>

<220>
<221>  mat_peptide
<222>  (82)..()
<223>

<400>  5
atg gcc tgc ggt ggc aag cgt ctg ctc ttc ctt gct ttg gca tgg gta       48Met Ala Cys Gly Gly Lys Arg Leu
Leu Phe Leu Ala Leu Ala Trp Val                    -25                    -20                    -15
     ctg ctg gct cac ctc tgc agc cag gca gaa gca gca agc aac tac gac       96Leu Leu Ala His Leu Cys Ser

Gln Ala Glu Ala Ala Ser Asn Tyr Asp -10 -5 -1 1 5
tgt tgc ctc tcg tac ata cag acg cct ctt cct tcc aga gct att gtg 144Cys Cys Leu Ser Tyr Ile
Gln Thr Pro Leu Pro Ser Arg Ala Ile Val 10 15
20 ggt ttc aca aga cag atg gcc gat gaa gct tgt gac att aat gct atc 192Gly Phe Thr Arg
Gln Met Ala Asp Glu Ala Cys Asp Ile Asn Ala Ile 25 30
35 atc ttt cac acg aag aaa aga aaa tct gtg tgc gct gat cca aag cag 240Ile Phe His
Thr Lys Lys Arg Lys Ser Val Cys Ala Asp Pro Lys Gln 40 45
50 aac tgg gtg aaa agg gct gtg aac ctc ctc agc cta aga gtc aag aag 288Asn Trp
Val Lys Arg Ala Val Asn Leu Leu Ser Leu Arg Val Lys Lys 55 60
65 atg 291Met
70

<210> 6
<211> 97
<212> PRT
<213> Mus musculus

<400> 6
Met Ala Cys Gly Gly Lys Arg Leu Leu Phe Leu Ala Leu Ala Trp Val
-25 -20 -15
Leu Leu Ala His Leu Cys Ser Gln Ala Glu Ala Ala Ser Asn Tyr Asp
-10 -5 -1 1 5
Cys Cys Leu Ser Tyr Ile Gln Thr Pro Leu Pro Ser Arg Ala Ile Val
10 15 20
Gly Phe Thr Arg Gln Met Ala Asp Glu Ala Cys Asp Ile Asn Ala Ile
25 30 35
Ile Phe His Thr Lys Lys Arg Lys Ser Val Cys Ala Asp Pro Lys Gln
40 45 50
Asn Trp Val Lys Arg Ala Val Asn Leu Leu Ser Leu Arg Val Lys Lys
55 60 65
Met
70

<210> 7
<211> 1122
<212> DNA
<213> Homo sapiens

<220>
<221> CDS
<222> (1)..(1122)
<223>

<400> 7
atg agc ggg gaa tca atg aat ttc agc gat gtt ttc gac tcc agt gaa 48Met Ser Gly Glu Ser Met Asn Phe
Ser Asp Val Phe Asp Ser Ser Glu 1 5 10 15
gat tat ttt gtg tca gtc aat act tca tat tac tca gtt gat tct gag 96Asp Tyr Phe Val Ser Val Asn
Thr Ser Tyr Tyr Ser Val Asp Ser Glu 20 25 30
atg tta ctg tgc tcc ttg cag gag gtc agg cag ttc tcc agg cta ttt 144Met Leu Leu Cys Ser Leu
Gln Glu Val Arg Gln Phe Ser Arg Leu Phe 35 40 45
gta ccg att gcc tac tcc ttg atc tgt gtc ttt ggc ctc ctg ggg aat 192Val Pro Ile Ala Tyr
Ser Leu Ile Cys Val Phe Gly Leu Leu Gly Asn 50 55 60
att ctg gtg gtg atc acc ttt gct ttt tat aag aag gcc agg tct atg 240Ile Leu Val Val
Ile Thr Phe Ala Phe Tyr Lys Lys Ala Arg Ser Met 65 70 75
80 aca gac gtc tat ctc ttg aac atg gcc att gca gac atc ctc ttt gtt 288Thr Asp Val
Tyr Leu Leu Asn Met Ala Ile Ala Asp Ile Leu Phe Val 85 90
95 ctt act ctc cca ttc tgg gca gtg agt cat gcc act ggt gcg tgg gtt 336Leu Thr
Leu Pro Phe Trp Ala Val Ser His Ala Thr Gly Ala Trp Val 100 105
110 ttc agc aat gcc acg tgc aag ttg cta aaa ggc atc tat gcc atc aac 384Phe
Ser Asn Ala Thr Cys Lys Leu Leu Lys Gly Ile Tyr Ala Ile Asn 115 120
125 ttt aac tgc ggg atg ctg ctc ctg act tgc att agc atg gac cgg tac
432Phe Asn Cys Gly Met Leu Leu Leu Thr Cys Ile Ser Met Asp Arg Tyr 130 135
140 atc gcc att gta cag gcg act aag tca ttc cgg ctc cga tcc aga aca
480Ile Ala Ile Val Gln Ala Thr Lys Ser Phe Arg Leu Arg Ser Arg Thr 145 150
155 160 cta ccg cgc agc aaa atc atc tgc ctt gtt gtg tgg ggg ctg tca
gtc 528Leu Pro Arg Ser Lys Ile Ile Cys Leu Val Val Trp Gly Leu Ser Val 165
170 175 atc atc tcc agc tca act ttt gtc ttc aac caa aaa tac aac
acc caa 576Ile Ile Ser Ser Ser Thr Phe Val Phe Asn Gln Lys Tyr Asn Thr Gln 180
185 190 ggc agc gat gtc tgt gaa ccc aag tac cag act gtc tcg
gag ccc atc 624Gly Ser Asp Val Cys Glu Pro Lys Tyr Gln Thr Val Ser Glu Pro Ile 195
200 205 agg tgg aag ctg ctg atg ttg ggg ctt gag cta ctc
ttt ggt ttc ttt 672Arg Trp Lys Leu Leu Met Leu Gly Leu Glu Leu Leu Phe Gly Phe Phe 210
215 220 atc cct ttg atg ttc atg ata ttt tgt tac acg
ttc att gtc aaa acc 720Ile Pro Leu Met Phe Met Ile Phe Cys Tyr Thr Phe Ile Val Lys Thr 225
230 235 240 ttg gtg caa gct cag aat tct aaa agg cac

aaa gcc atc cgt gta atc      768Leu Val Gln Ala Gln Asn Ser Lys Arg His Lys Ala Ile Arg Val Ile
            245                    250                    255                    ata gct gtg gtg ctt gtg ttt ctg gct
tgt cag att cct cat aac atg      816Ile Ala Val Val Leu Val Phe Leu Ala Cys Gln Ile Pro His Asn Met
            260                    265                    270                    gtc ctg ctt gtg acg gct gca aat
ttg ggt aaa atg aac cga tcc tgc      864Val Leu Leu Val Thr Ala Ala Asn Leu Gly Lys Met Asn Arg Ser Cys
            275                    280                    285                    cag agc gaa aag cta att ggc
tat acg aaa act gtc aca gaa gtc ctg      912Gln Ser Glu Lys Leu Ile Gly Tyr Thr Lys Thr Val Thr Glu Val
Leu                    290                    295                    300                    gct ttc ctg cac tgc
tgc ctg aac cct gtg ctc tac gct ttt att ggg      960Ala Phe Leu His Cys Cys Leu Asn Pro Val Leu Tyr Ala
Phe Ile Gly            305                    310                    315                    320        cag aag ttc
aga aac tac ttt ctg aag atc ttg aag gac ctg tgg tgt      1008Gln Lys Phe Arg Asn Tyr Phe Leu Lys Ile Leu
Lys Asp Leu Trp Cys                    325                    330                    335                    gtg
aga agg aag tac aag tcc tca ggc ttc tcc tgt gcc ggg agg tac      1056Val Arg Arg Lys Tyr Lys Ser Ser Gly
Phe Ser Cys Ala Gly Arg Tyr                    340                    345                    350
  tca gaa aac att tct cgg cag acc agt gag acc gca gat aac gac aat      1104Ser Glu Asn Ile Ser Arg Gln Thr
Ser Glu Thr Ala Asp Asn Asp Asn                    355                    360                    365
    gcg tcg tcc ttc act atg                                        1122Ala Ser Ser Phe Thr Met
                                        370

<210>  8
<211>  374
<212>  PRT
<213>  Homo sapiens

<400>  8
Met Ser Gly Glu Ser Met Asn Phe Ser Asp Val Phe Asp Ser Ser Glu
1               5                   10                  15
Asp Tyr Phe Val Ser Val Asn Thr Ser Tyr Tyr Ser Val Asp Ser Glu
            20                  25                  30
Met Leu Leu Cys Ser Leu Gln Glu Val Arg Gln Phe Ser Arg Leu Phe
        35                  40                  45
Val Pro Ile Ala Tyr Ser Leu Ile Cys Val Phe Gly Leu Leu Gly Asn
    50                  55                  60
Ile Leu Val Val Ile Thr Phe Ala Phe Tyr Lys Lys Ala Arg Ser Met
65                  70                  75                  80
Thr Asp Val Tyr Leu Leu Asn Met Ala Ile Ala Asp Ile Leu Phe Val
                85                  90                  95
Leu Thr Leu Pro Phe Trp Ala Val Ser His Ala Thr Gly Ala Trp Val
            100                 105                 110
Phe Ser Asn Ala Thr Cys Lys Leu Leu Lys Gly Ile Tyr Ala Ile Asn
        115                 120                 125
Phe Asn Cys Gly Met Leu Leu Leu Thr Cys Ile Ser Met Asp Arg Tyr
    130                 135                 140
Ile Ala Ile Val Gln Ala Thr Lys Ser Phe Arg Leu Arg Ser Arg Thr
145                 150                 155                 160
Leu Pro Arg Ser Lys Ile Ile Cys Leu Val Val Trp Gly Leu Ser Val
                165                 170                 175
Ile Ile Ser Ser Ser Thr Phe Val Phe Asn Gln Lys Tyr Asn Thr Gln
            180                 185                 190
Gly Ser Asp Val Cys Glu Pro Lys Tyr Gln Thr Val Ser Glu Pro Ile
        195                 200                 205
Arg Trp Lys Leu Leu Met Leu Gly Leu Glu Leu Leu Phe Gly Phe Phe
    210                 215                 220
Ile Pro Leu Met Phe Met Ile Phe Cys Tyr Thr Phe Ile Val Lys Thr
225                 230                 235                 240
Leu Val Gln Ala Gln Asn Ser Lys Arg His Lys Ala Ile Arg Val Ile
                245                 250                 255
Ile Ala Val Val Leu Val Phe Leu Ala Cys Gln Ile Pro His Asn Met
            260                 265                 270
Val Leu Leu Val Thr Ala Ala Asn Leu Gly Lys Met Asn Arg Ser Cys
        275                 280                 285
Gln Ser Glu Lys Leu Ile Gly Tyr Thr Lys Thr Val Thr Glu Val Leu
    290                 295                 300
Ala Phe Leu His Cys Cys Leu Asn Pro Val Leu Tyr Ala Phe Ile Gly
305                 310                 315                 320
Gln Lys Phe Arg Asn Tyr Phe Leu Lys Ile Leu Lys Asp Leu Trp Cys
                325                 330                 335
Val Arg Arg Lys Tyr Lys Ser Ser Gly Phe Ser Cys Ala Gly Arg Tyr
            340                 345                 350
Ser Glu Asn Ile Ser Arg Gln Thr Ser Glu Thr Ala Asp Asn Asp Asn
        355                 360                 365
Ala Ser Ser Phe Thr Met
    370

<210>  9

<211>  1101
<212>  DNA
<213>  Mus musculus

<220>
<221>  CDS
<222>  (1)..(1101)
<223>

<400>  9
atg aat tcc aca gag tcc tac ttt gga acg gat gat tat gac aac aca      48Met Asn Ser Thr Glu Ser Tyr Phe
Gly Thr Asp Asp Tyr Asp Asn Thr          1               5                   10                  15
gag tat tat tct att cct cca gac cat ggg cca tgc tcc cta gaa gag      96Glu Tyr Tyr Ser Ile Pro Pro
Asp His Gly Pro Cys Ser Leu Glu Glu                  20                  25                  30
gtc aga aac ttc acc aag gta ttt gtg cca att gcc tac tcc tta ata      144Val Arg Asn Phe Thr Lys
Val Phe Val Pro Ile Ala Tyr Ser Leu Ile              35                  40                  45
tgt gtc ttt ggc ctc ctg ggc aac att atg gtg gtg atg acc ttt gcc      192Cys Val Phe Gly Leu
Leu Gly Asn Ile Met Val Val Met Thr Phe Ala          50                  55                  60
ttc tac aag aaa gcc aga tcc atg act gac gtc tac ctg ttg aac atg      240Phe Tyr Lys Lys
Ala Arg Ser Met Thr Asp Val Tyr Leu Leu Asn Met      65                  70                  75
80          gcc atc aca gac ata ctc ttt gtc ctc acc cta ccg ttc tgg gca gtt      288Ala Ile Thr
Asp Ile Leu Phe Val Leu Thr Leu Pro Phe Trp Ala Val                      85                  90
95          act cat gcc acc aac act tgg gtt ttc agc gat gca ctg tgt aaa ctg      336Thr His
Ala Thr Asn Thr Trp Val Phe Ser Asp Ala Leu Cys Lys Leu                  100                 105
110         atg aaa ggc aca tat gcg gtc aac ttt aac tgt ggg atg ctg ctc ctg      384Met
Lys Gly Thr Tyr Ala Val Asn Phe Asn Cys Gly Met Leu Leu Leu              115                 120
125         gcc tgt atc agc atg gac cgg tac att gcc atc gtc cag gca acc aaa
432Ala Cys Ile Ser Met Asp Arg Tyr Ile Ala Ile Val Gln Ala Thr Lys          130                 135
140         tct ttc cgg gta cgc tcc aga aca ctg acg cac agt aag gtc atc tgt
480Ser Phe Arg Val Arg Ser Arg Thr Leu Thr His Ser Lys Val Ile Cys          145                 150
155         160         gtg gca gtg tgg ttc atc tcc atc atc atc tca agc cct aca ttt
atc      528Val Ala Val Trp Phe Ile Ser Ile Ile Ile Ser Ser Pro Thr Phe Ile          165
170         175         ttc aac aag aaa tac gag ctg cag gat cgt gat gtc tgt gag
cca cgg      576Phe Asn Lys Lys Tyr Glu Leu Gln Asp Arg Asp Val Cys Glu Pro Arg          180
185         190         tac agg tct gtc tca gag ccc atc acg tgg aag ctg ctg
ggt atg gga      624Tyr Arg Ser Val Ser Glu Pro Ile Thr Trp Lys Leu Leu Gly Met Gly          195
200         205         ctg gag ctg ttc ttt ggg ttc ttc acc cct ttg ctg
ttt atg gtg ttc      672Leu Glu Leu Phe Phe Gly Phe Phe Thr Pro Leu Leu Phe Met Val Phe          210
215         220         tgc tat ctg ttc att atc aag acc ttg gtg cag
gcc cag aac tcc aag      720Cys Tyr Leu Phe Ile Ile Lys Thr Leu Val Gln Ala Gln Asn Ser Lys          225
230         235         240         agg cac aga gcc atc cga gtc gtg atc gct
gtg gtt ctc gtg ttc ctg      768Arg His Arg Ala Ile Arg Val Val Ile Ala Val Val Leu Val Phe Leu
245         250         255         gct tgt cag atc cct cac aac atg gtc
ctc ctc gtg act gcg gtc aac      816Ala Cys Gln Ile Pro His Asn Met Val Leu Leu Val Thr Ala Val Asn
260         265         270         acg ggc aaa gtg ggc cgg agc tgc
agc acc gag aaa gtc ctc gcc tac      864Thr Gly Lys Val Gly Arg Ser Cys Ser Thr Glu Lys Val Leu Ala Tyr
275         280         285         acc agg aac gtg gcc gag gtc
ctg gct ttc ctg cat tgc tgc ctc aac      912Thr Arg Asn Val Ala Glu Val Leu Ala Phe Leu His Cys Cys Leu
Asn         290         295         300         ccc gtg ttg tat gcg
ttt att gga cag aaa ttc aga aac tac ttc atg      960Pro Val Leu Tyr Ala Phe Ile Gly Gln Lys Phe Arg Asn
Tyr Phe Met         305         310         315         320         aag atc atg
aag gat gtg tgg tgt atg aga agg aag aat aag atg cct      1008Lys Ile Met Lys Asp Val Trp Cys Met Arg Arg
Lys Asn Lys Met Pro         325         330         335         ggc
ttc ctc tgt gcc cgg gtt tac tcg gaa agc tac atc tcc agg cag      1056Gly Phe Leu Cys Ala Arg Val Tyr Ser
Glu Ser Tyr Ile Ser Arg Gln         340         345         350
acc agt gag acc gtc gaa aat gat aat gca tcg tcc ttt acc atg      1101Thr Ser Glu Thr Val Glu Asn Asp
Asn Ala Ser Ser Phe Thr Met         355         360         365


<210>  10
<211>  367
<212>  PRT
<213>  Mus musculus

<400>  10
Met Asn Ser Thr Glu Ser Tyr Phe Gly Thr Asp Asp Tyr Asp Asn Thr
1               5                   10                  15
Glu Tyr Tyr Ser Ile Pro Pro Asp His Gly Pro Cys Ser Leu Glu Glu
            20                  25                  30
Val Arg Asn Phe Thr Lys Val Phe Val Pro Ile Ala Tyr Ser Leu Ile
        35                  40                  45
Cys Val Phe Gly Leu Leu Gly Asn Ile Met Val Val Met Thr Phe Ala
    50                  55                  60
Phe Tyr Lys Lys Ala Arg Ser Met Thr Asp Val Tyr Leu Leu Asn Met
65                  70                  75                  80
Ala Ile Thr Asp Ile Leu Phe Val Leu Thr Leu Pro Phe Trp Ala Val

                85                  90                  95
Thr His Ala Thr Asn Thr Trp Val Phe Ser Asp Ala Leu Cys Lys Leu
            100                 105                 110
Met Lys Gly Thr Tyr Ala Val Asn Phe Asn Cys Gly Met Leu Leu Leu
        115                 120                 125
Ala Cys Ile Ser Met Asp Arg Tyr Ile Ala Ile Val Gln Ala Thr Lys
    130                 135                 140
Ser Phe Arg Val Arg Ser Arg Thr Leu Thr His Ser Lys Val Ile Cys
145                 150                 155                 160
Val Ala Val Trp Phe Ile Ser Ile Ile Ile Ser Ser Pro Thr Phe Ile
                165                 170                 175
Phe Asn Lys Lys Tyr Glu Leu Gln Asp Arg Asp Val Cys Glu Pro Arg
            180                 185                 190
Tyr Arg Ser Val Ser Glu Pro Ile Thr Trp Lys Leu Leu Gly Met Gly
        195                 200                 205
Leu Glu Leu Phe Phe Gly Phe Phe Thr Pro Leu Leu Phe Met Val Phe
    210                 215                 220
Cys Tyr Leu Phe Ile Ile Lys Thr Leu Val Gln Ala Gln Asn Ser Lys
225                 230                 235                 240
Arg His Arg Ala Ile Arg Val Val Ile Ala Val Val Leu Val Phe Leu
                245                 250                 255
Ala Cys Gln Ile Pro His Asn Met Val Leu Leu Val Thr Ala Val Asn
            260                 265                 270
Thr Gly Lys Val Gly Arg Ser Cys Ser Thr Glu Lys Val Leu Ala Tyr
        275                 280                 285
Thr Arg Asn Val Ala Glu Val Leu Ala Phe Leu His Cys Cys Leu Asn
    290                 295                 300
Pro Val Leu Tyr Ala Phe Ile Gly Gln Lys Phe Arg Asn Tyr Phe Met
305                 310                 315                 320
Lys Ile Met Lys Asp Val Trp Cys Met Arg Arg Lys Asn Lys Met Pro
                325                 330                 335
Gly Phe Leu Cys Ala Arg Val Tyr Ser Glu Ser Tyr Ile Ser Arg Gln
            340                 345                 350
Thr Ser Glu Thr Val Glu Asn Asp Asn Ala Ser Ser Phe Thr Met
        355                 360                 365

<210> 11
<211> 20
<212> DNA
<213> Artificial

<220>
<221> misc_feature
<223> Oligonucleotide designed to act as primer for amplifying fragment of rat MIP-3α gene
transcript.

<400> 11
agaatggcct gcaagcatct 20

<210> 12
<211> 21
<212> DNA
<213> Artificial

<220>
<221> misc_feature
<223> Oligonucleotide designed to act as primer for amplifying fragment of rat MIP-3α gene
transcript.

<400> 12
tgcagaggta agccagcagt a 21

<210> 13
<211> 1502
<212> DNA
<213> Rattus norvegicus (kidney)

<220>
<221> CDS
<222> (343)..(1443)
<223>

<400> 13
tgtattgaag acagaacact tgtggtaaga cacccacccc cgggagggcg aagaacaagc 60cacacactgc tttgaagagt ccagccccaa
gcagaactgc aagggcagac actgttctgg 120ccacctgcag tttgaagtca tcactttcaa tccccctgtg actagggcca gggtcttcac
180acctgcgaga ggaagcaaag atctaagcaa tctgaatttt aagagagaaa ctgcagctgt 240cggtttgtgg gccggaacat

tattggactg gagcctggac aagcactaag gcgggggtac    300ctggccagcc cacttcggag ctcagcgttt ccttgggaaa cg atg aat
ttc acc      354                                                    Met Asn Phe Thr
                                        1                    gag gcc aac tac gga atg gaa gat tat act ggc tca
gat tac tct atg      402Glu Ala Asn Tyr Gly Met Glu Asp Tyr Thr Gly Ser Asp Tyr Ser Met      5
        10                    15                    20              ttt cca gag acc gag cca tgc tct ctg caa gag
gtc aga gac ttc acc      450Phe Pro Glu Thr Glu Pro Cys Ser Leu Gln Glu Val Arg Asp Phe Thr
        25                    30                    35                    aag gtg ttc gtg cca atc gcc tac tcc tta
atc tgt gtc ttt ggc ctc      498Lys Val Phe Val Pro Ile Ala Tyr Ser Leu Ile Cys Val Phe Gly Leu
        40                    45                    50                          ctt ggc aat att atg gtg gtg ata acc
ttt gcc ttc tac aag aaa gcc      546Leu Gly Asn Ile Met Val Val Ile Thr Phe Ala Phe Tyr Lys Lys Ala
        55                    60                    65                                agg tcc atg act gac gtc tac cta
ttg aac atg gcc atc aca gac ata      594Arg Ser Met Thr Asp Val Tyr Leu Leu Asn Met Ala Ile Thr Asp Ile
        70                    75                    80                                      ctc ttt gtc ctc acc cta cca
ttc tgg gca gtt act cat gcc act gac      642Leu Phe Val Leu Thr Leu Pro Phe Trp Ala Val Thr His Ala Thr
Asp        85                    90                    95                    100          act tgg atc ttt ggc
aac acg atg tgt aaa ctg atg aaa ggc acg tat      690Thr Trp Ile Phe Gly Asn Thr Met Cys Lys Leu Met Lys
Gly Thr Tyr                          105                    110                    115                gcg gtc aac
ttt aac tgt ggg atg ctg ctc ctg gcc tgt atc agc atg      738Ala Val Asn Phe Asn Cys Gly Met Leu Leu Leu
Ala Cys Ile Ser Met                          120                    125                    130                    gac
cgg tac att gcc atc gtc cag gcg acc aaa tct ttc cgg gta cgc      786Asp Arg Tyr Ile Ala Ile Val Gln Ala
Thr Lys Ser Phe Arg Val Arg                          135                    140                    145
  tcc aga aca ctg acg cac agt aag gtc atc tgt ctg acg gtg tgg ttc      834Ser Arg Thr Leu Thr His Ser Lys
Val Ile Cys Leu Thr Val Trp Phe                          150                    155                    160
      gtt tcc atc atc atc tca agc ccc aca ttc ttc ttc aac aag caa tac      882Val Ser Ile Ile Ile Ser Ser
Pro Thr Phe Phe Phe Asn Lys Gln Tyr                    165                    170                    175
180          aag ctg cag ggc cgt gat gtc tgc gag cct cag tac aag ctc gtc tcg      930Lys Leu Gln Gly Arg
Asp Val Cys Glu Pro Gln Tyr Lys Leu Val Ser                                        185                    190
  195                    gag ccc atc acg tgg aaa ctg ctg ggc atg gga ctc gag ctg ctc ttt      978Glu Pro Ile Thr
Trp Lys Leu Leu Gly Met Gly Leu Glu Leu Leu Phe                                        200                    205
  210                    ggc ttc ttc atc cct ttg ctg ttt atg gtg ttc tgt tac ctg ttc atc      1026Gly Phe Phe
Ile Pro Leu Leu Phe Met Val Phe Cys Tyr Leu Phe Ile                                        215                    220
  225                          atc aag acc ttg gtg cag gcc cag aat tcc aag agg cac aga gcc atc      1074Ile Lys
Thr Leu Val Gln Ala Gln Asn Ser Lys Arg His Arg Ala Ile                                        230                    235
  240                                cga gtc gtg att gct gtg gtt ctc gtg ttc ctg gct tgt cag atc cct      1122Arg
Val Val Ile Ala Val Val Leu Val Phe Leu Ala Cys Gln Ile Pro                    245                    250
  255                    260              cac aac atg gtc ctc ctc gtg act gca gcc aac acg ggc aaa atg ggc
1170His Asn Met Val Leu Leu Val Thr Ala Ala Asn Thr Gly Lys Met Gly                                        265
        270                    275                    cgc agc tgc agc gcc gag aaa gcc ctc gcc tac gcc agg aat gtg gct
    1218Arg Ser Cys Ser Ala Glu Lys Ala Leu Ala Tyr Ala Arg Asn Val Ala                                        280
        285                    290                          gag gtc ctg gct ttc ctg cac tgc tgt ctc aac ccc gtg ttg tat
gcc      1266Glu Val Leu Ala Phe Leu His Cys Cys Leu Asn Pro Val Leu Tyr Ala                                        295
        300                    305                          ttc att gga cag aaa ttc aga agc tac ttc atg aag atc atg
aag gat      1314Phe Ile Gly Gln Lys Phe Arg Ser Tyr Phe Met Lys Ile Met Lys Asp                                        310
        315                    320                                gtg tgg tgt atg agg agg aag agc aag gtg cct acc ttc
ttc tgt gcc      1362Val Trp Cys Met Arg Arg Lys Ser Lys Val Pro Thr Phe Phe Cys Ala                    325
        330                    335                    340              cgg gtt tac tca gaa agc tac atc tcc agg cag acc
agt gag act gta      1410Arg Val Tyr Ser Glu Ser Tyr Ile Ser Arg Gln Thr Ser Glu Thr Val
        345                    350                    355                    gaa aat gac aac gca tcg tcc ttt acc atg taa
cacgagagca caaagcagca    1463Glu Asn Asp Asn Ala Ser Ser Phe Thr Met
        360                    365                                        tgccccgaaa gcctttgtga aacttgctat
tacatgtga                          1502

<210>  14
<211>  366
<212>  PRT
<213>  Rattus norvegicus

<400>  14
Met Asn Phe Thr Glu Ala Asn Tyr Gly Met Glu Asp Tyr Thr Gly Ser
1                    5                    10                    15
Asp Tyr Ser Met Phe Pro Glu Thr Glu Pro Cys Ser Leu Gln Glu Val
                    20                    25                    30
Arg Asp Phe Thr Lys Val Phe Val Pro Ile Ala Tyr Ser Leu Ile Cys
        35                    40                    45
Val Phe Gly Leu Leu Gly Asn Ile Met Val Val Ile Thr Phe Ala Phe
    50                    55                    60
Tyr Lys Lys Ala Arg Ser Met Thr Asp Val Tyr Leu Leu Asn Met Ala
65                    70                    75                    80
Ile Thr Asp Ile Leu Phe Val Leu Thr Leu Pro Phe Trp Ala Val Thr
                    85                    90                    95
His Ala Thr Asp Thr Trp Ile Phe Gly Asn Thr Met Cys Lys Leu Met
                    100                    105                    110
Lys Gly Thr Tyr Ala Val Asn Phe Asn Cys Gly Met Leu Leu Leu Ala
        115                    120                    125
Cys Ile Ser Met Asp Arg Tyr Ile Ala Ile Val Gln Ala Thr Lys Ser
    130                    135                    140

Phe Arg Val Arg Ser Arg Thr Leu Thr His Ser Lys Val Ile Cys Leu
145                 150                 155                 160
Thr Val Trp Phe Val Ser Ile Ile Ile Ser Ser Pro Thr Phe Phe Phe
                165                 170                 175
Asn Lys Gln Tyr Lys Leu Gln Gly Arg Asp Val Cys Glu Pro Gln Tyr
            180                 185                 190
Lys Leu Val Ser Glu Pro Ile Thr Trp Lys Leu Leu Gly Met Gly Leu
        195                 200                 205
Glu Leu Leu Phe Gly Phe Phe Ile Pro Leu Leu Phe Met Val Phe Cys
    210                 215                 220
Tyr Leu Phe Ile Ile Lys Thr Leu Val Gln Ala Gln Asn Ser Lys Arg
225                 230                 235                 240
His Arg Ala Ile Arg Val Val Ile Ala Val Val Leu Val Phe Leu Ala
                245                 250                 255
Cys Gln Ile Pro His Asn Met Val Leu Leu Val Thr Ala Ala Asn Thr
            260                 265                 270
Gly Lys Met Gly Arg Ser Cys Ser Ala Glu Lys Ala Leu Ala Tyr Ala
        275                 280                 285
Arg Asn Val Ala Glu Val Leu Ala Phe Leu His Cys Cys Leu Asn Pro
    290                 295                 300
Val Leu Tyr Ala Phe Ile Gly Gln Lys Phe Arg Ser Tyr Phe Met Lys
305                 310                 315                 320
Ile Met Lys Asp Val Trp Cys Met Arg Arg Lys Ser Lys Val Pro Thr
                325                 330                 335
Phe Phe Cys Ala Arg Val Tyr Ser Glu Ser Tyr Ile Ser Arg Gln Thr
            340                 345                 350
Ser Glu Thr Val Glu Asn Asp Asn Ala Ser Ser Phe Thr Met
        355                 360                 365

<210>  15
<211>  1309
<212>  DNA
<213>  Rattus norvegicus (liver)

<220>
<221>  CDS
<222>  (150)..(1250)
<223>

<400>  15
gcatctcact acccgtctct caatgagcac cgctggttgt gcctgtcaac agaatagtcc     60tctcacactt aggactggag cctggacaag
cactaaggcg ggggtacctg gccagcccac    120ttcggagctc agcgtttcct tgggaaacg atg aat ttc acc gag gcc aac tac
  173                                    Met Asn Phe Thr Glu Ala Asn Tyr
   1               5                          gga atg gaa gat tat act ggc tca gat tac tct atg ttt cca gag
acc      221Gly Met Glu Asp Tyr Thr Gly Ser Asp Tyr Ser Met Phe Pro Glu Thr            10
  15                20                          gag cca tgc tct ctg caa gag gtc aga gac ttc acc aag gtg
ttc gtg      269Glu Pro Cys Ser Leu Gln Glu Val Arg Asp Phe Thr Lys Val Phe Val          25
  30                35                  40          cca atc gcc tac tcc tta atc tgt gtc ttt ggc ctc ctt
ggc aat att      317Pro Ile Ala Tyr Ser Leu Ile Cys Val Phe Gly Leu Leu Gly Asn Ile
  45                50                  55              atg gtg gtg ata acc ttt gcc ttc tac aag aaa gcc
agg tcc atg act      365Met Val Val Ile Thr Phe Ala Phe Tyr Lys Lys Ala Arg Ser Met Thr
  60                65                  70                  gac gtc tac cta ttg aac atg gcc atc aca gac
ata ctc ttt gtc ctc      413Asp Val Tyr Leu Leu Asn Met Ala Ile Thr Asp Ile Leu Phe Val Leu
  75                80                  85                      acc cta cca ttc tgg gca gtt act cat gcc
act gac act tgg atc ttt      461Thr Leu Pro Phe Trp Ala Val Thr His Ala Thr Asp Thr Trp Ile Phe
  90                95                  100                         ggc aac acg atg tgt aaa ctg atg aaa
ggc acg tat gcg gtc aac ttt      509Gly Asn Thr Met Cys Lys Leu Met Lys Gly Thr Tyr Ala Val Asn Phe
  105               110                 115                 120         aac tgt ggg atg ctg ctc ctg gcc
tgt atc agc atg gac cgg tac att      557Asn Cys Gly Met Leu Leu Leu Ala Cys Ile Ser Met Asp Arg Tyr Ile
                    125                 130                 135             gcc atc gtc cag gcg acc aaa
tct ttc cgg gta cgc tcc aga aca ctg      605Ala Ile Val Gln Ala Thr Lys Ser Phe Arg Val Arg Ser Arg Thr
Leu                 140                 145                 150                     acg cac agt aag gtc
atc tgt ctg acg gtg tgg ttc gtt tcc atc atc      653Thr His Ser Lys Val Ile Cys Leu Thr Val Trp Phe Val
Ser Ile Ile                 155                 160                 165                         atc tca agc
ccc aca ttc ttc ttc aac aag caa tac aag ctg cag ggc      701Ile Ser Ser Pro Thr Phe Phe Phe Asn Lys Gln
Tyr Lys Leu Gln Gly             170                 175                 180                             cgt
gat gtc tgc gag cct cag tac aag ctc gtc tcg gag ccc atc acg      749Arg Asp Val Cys Glu Pro Gln Tyr Lys
Leu Val Ser Glu Pro Ile Thr         185                 190                 195                 200
  tgg aaa ctg ctg ggc atg gga ctc gag ctg ctc ttt ggc ttc ttc atc      797Trp Lys Leu Leu Gly Met Gly Leu
Glu Leu Leu Phe Gly Phe Phe Ile                 205                 210                 215
      cct ttg ctg ttt atg gtg ttc tgt tac ctg ttc atc atc aag acc ttg      845Pro Leu Leu Phe Met Val Phe
Cys Tyr Leu Phe Ile Ile Lys Thr Leu                 220                 225                 230
          gtg cag gcc cag aat tcc aag agg cac aga gcc atc cga gtc gtg att      893Val Gln Ala Gln Asn Ser
Lys Arg His Arg Ala Ile Arg Val Val Ile                 235                 240                 245
              gct gtg gtt ctc gtg ttc ctg gct tgt cag atc cct cac aac atg gtc      941Ala Val Val Leu Val
Phe Leu Ala Cys Gln Ile Pro His Asn Met Val                 250                 255                 260

ctc ctc gtg act gca gcc aac acg ggc aaa atg ggc cgc agc tgc agc 989Leu Leu Val Thr
Ala Ala Asn Thr Gly Lys Met Gly Arg Ser Cys Ser 265 270 275
280 gcc gag aaa gcc ctc gcc tac gcc agg aat gtg gct gag gtc ctg gct 1037Ala Glu Lys
Ala Leu Ala Tyr Ala Arg Asn Val Ala Glu Val Leu Ala 285 290
295 ttc ctg cac tgc tgt ctc aac ccc gtg ttg tat gcc ttc att gga cag 1085Phe Leu
His Cys Cys Leu Asn Pro Val Leu Tyr Ala Phe Ile Gly Gln 300 305
310 aaa ttc aga agc tac ttc atg aag atc atg aag gat gtg tgg tgt atg 1133Lys
Phe Arg Ser Tyr Phe Met Lys Ile Met Lys Asp Val Trp Cys Met 315 320
325 agg agg aag agc aag gtg cct acc ttc ttc tgt gcc cgg gtt tac tca
1181Arg Arg Lys Ser Lys Val Pro Thr Phe Phe Cys Ala Arg Val Tyr Ser 330 335
340 gaa agc tac atc tcc agg cag acc agt gag act gta gaa aat gac aac
1229Glu Ser Tyr Ile Ser Arg Gln Thr Ser Glu Thr Val Glu Asn Asp Asn 345 350
355 360 gca tcg tcc ttt acc atg taa cacgagagca caaagcagca
tgccccgaaa 1280Ala Ser Ser Phe Thr Met
365 gcctttgtga aacttgctat tacatgtga
1309

<210> 16
<211> 25
<212> DNA
<213> Artificial

<220>
<223> Oligonucleotide designed to act as primer for amplifying CCR6
cDNA derived from rat kidney.

<400> 16
tgtattgaag acagaacact tgtgg 25

<210> 17
<211> 28
<212> DNA
<213> Artificial

<220>
<223> Oligonucleotide designed to act as primer for amplifying CCR6
cDNA derived from rat kidney or rat liver.

<400> 17
tcacatgtaa tagcaagttt cacaaagg 28

<210> 18
<211> 21
<212> DNA
<213> Artificial

<220>
<223> Oligonucleotide designed to act as primer for amplifying CCR6
cDNA derived from rat liver.

<400> 18
gcatctcact acccgtctct c 21

<210> 19
<211> 21
<212> DNA
<213> Artificial

<220>
<223> Oligonucleotide designed to act as primer for amplifying fragment of rat CCR6 gene
transcript.

<400> 19
ggacgatgcg ttgtcatttt c 21

<210> 20
<211> 22
<212> DNA
<213> Artificial

<220>
<223> Oligonucleotide designed to act as primer for amplifying fragment of rat CCR6 gene
transcript.

<400> 20
ccgcagctgc agcgccgaga aa 22

<210>  21
<211>  20
<212>  DNA
<213>  Artificial

<220>
<223>  Oligonucleotide designed to act as primer for amplifying fragment       of rat CCR6 gene
transcript.

<400>  21
gtgcccgggt ttactcagaa                                                    20

## Description

### Field of the Invention

The present invention relates to a novel brain/nerve cell protective agent, particularly to the protective agent that is effective in the prophylaxis and treatment of cerebrovascular disorders such as cerebral infarction, cerebral hemorrhage, and subarachnoid hemorrhage, or head trauma. The present invention also relates to a diagnostic agent for brain/nerve cell injuries such as cerebrovascular disorders and head trauma. The present invention further relates to a method for screening of a substance that exhibits brain/nerve cell protective action and a kit therefor.

### Background Art

Cerebrovascular disorders represent a disease that results in large medical economic losses, being the second or third most prevalent cause of death and the first most prevalent cause of severe sequelae in Japan, USA and Europe. At present, aggressive causal treatment (tPA and the like) is performed on some patients with cerebral embolism or cerebral thrombosis, but its coverage remains narrow at several percent of the entire patient population, due to a limitation of therapeutic time window. In most cases, only maintenance therapy is administered to prevent cerebral edema and to suppress the recurrence/expansion of the disease (antithrombotic drugs), with no effective medication available for radical cure and brain protection.

Traditionally, cells of central nervous system have been well recognized as being fragile to ischemic stress; according to basic experiments using a cerebral ischemic model, nerve cells die from irreversible damage even when ischemized for only several minutes. It is undeniable that this has led to the loss of hope in clinical field for the treatment of cerebral stroke. In recent years, however, diligent studies in the neuroscience field have revealed various aspects that may be resolvable, including various stress responses at individual cell levels, crosstalks between nerve cells and glial cells, programmed cell death and the like, during ischemic loading; there is the great expectation that these findings will lead to the development of a more aggressive therapeutic strategy.

Although many attempts have been made to date to develop products based on various mechanisms of action, for example, glutamate antagonists, calcium antagonists, antioxidants and the like, all clinical studies of these products have failed to produce successful results. In Japan, RADICUT (registered trademark, Mitsubishi Pharma Corporation), an antioxidant, has been approved, but this agent has not been launched overseas; no brain-protective drug is available worldwide.

With the improvement in the intensive care system for cerebral stroke patients, brain hypothermia treatment has been reconsidered as being clinically effective for brain protection therapy. Brain hypothermia treatment is intended to maintain a lowered brain temperature at 32 to 35°C, and is gradually attracting attention for its remarkable brain-protecting effect. However, this treatment is difficult to spread as a common therapeutic approach because it necessitates 24-hour intensive management at an intensive care facility with the attendance of a plurality of medical staff members.

As the microarray method with an immobilized cDNA or oligonucleotide has been developed to enable comprehensive analysis of gene expression, technology for detecting disease-specific changes in gene expression has spread and has been recognized as being useful. For example, Affymetrix's GeneChip system is being increasingly used for the diagnosis of diseases such as cancers and for the discovery of gene targets for drug development. In fact, attempts have been made to find genes and proteins whose expression is specifically promoted due to cerebral ischemia, or genes and proteins whose expression is specifically suppressed, and to develop therefrom therapeutic drugs and diagnostic agents for cerebrovascular disorders. For example, inflammatory cytokines such as interleukin 1β (IL-1β) and tumor necrosis factor (TNFα) are known to be expressed at increased levels at infarcted sites during cerebral ischemia, and antagonists for receptors of these cytokines are being investigated as therapeutic drugs for acute stage cerebrovascular disorders.

MIP-3α (also called LARC (liver and activation-regulated chemokine), Exodus, ST38, and CCL20; herein referred to as "MIP-3α") is a kind of CC chemokine reported from more than one laboratory in 1997 (see, for example, non-patent document 1). The rat homologue of this protein was isolated as a gene expressed at an increased level in the cerebral cortex of a cerebral ischemic model, and has been reported to be expressed at increased or decreased levels correlating with onset of inflammation and therapeutic effect in an allergic encephalomyelitis model (patent document 1, non-patent document 2).

In addition, since mice knocked out for the MIP-3α receptor CCR6 exhibit decreased humoral immunity in the intestine, it has been suggested that the MIP-3α/CCR6 signal transduction system may be involved in transportation and activation of lymphocyte (patent document 2).
Patent document 1: WO 98/49309
Patent document 2: WO 01/17558
Non-patent document 1: Hieshima et al., Journal of Biological Chemistry, USA, 1997, Vol. 272, pp. 5846-5853 Non-patent document 2: Utans-Schneitz et al., Journal of Neuroimmunology, Netherlands, 1998, Vol. 92, pp. 179-190

### Disclosure of the Invention

Currently, treatment of cerebrovascular disorders need to be performed after an established diagnosis is made by X-ray CT, MRI diagnostic imaging or the like in almost all cases, and this requirement limits the therapeutic time window. Therefore, there is a strong demand for the establishment of a novel prophylactic/therapeutic means for cerebrovascular disorders, which is applicable irrespective of type of disease, and which does not require an established diagnosis.

It is an object of the present invention to provide a safe and effective prophylactic/therapeutic agent for cerebrovascular disorders. It is another object of the present invention to provide a convenient and effective method for screening of a substance having a prophylactic/therapeutic effect on cerebrovascular disorders.

With the aim of accomplishing the above objects, the present inventors tested the characteristics of hypothermic treatment using a rat model of cerebral ischemia, and searched for a target gene considered to mediate the primary mechanism of action in hypothermic treatment, using the microarray method. As a result, the inventors discovered the MIP-3α gene, a gene whose expression remarkably increases during reperfusion following transient local ischemia, and whose expression is considerably suppressed, with a brain-protecting effect, by hypothermic treatment, in the rat model of cerebral ischemia.

Furthermore, the present inventors confirmed that infarction volume was considerably reduced by administering an anti-MIP-3α antibody intracerebroventricularly to suppress the activity of MIP-3α in cerebral ischemia rats.

In addition, the present inventors newly cloned the rat CCR6 gene, and examined by the PCR method based on the base sequence of the obtained cDNA for the expression of the CCR6 gene and the presence or absence of a decrease in the expression by hypothermic treatment in brain tissue in a rat model of localized cerebral ischemia; as a result, the inventors found that the CCR6 gene had its expression remarkably increased during reperfusion following transient local ischemia, and its expression considerably suppressed, with a brain-protecting effect, by hypothermic treatment. The present inventors conducted further investigations based on these findings, and developed the present invention.

Accordingly, the present invention provides:
[1] a brain/nerve cell protective agent comprising a substance that suppresses a protein comprising the same or substantially the same amino acid sequence as the amino acid sequence of amino acid number 1 and after in the amino acid sequence represented by SEQ ID NO:2, 4 or 6, or a salt thereof,
[2] a brain/nerve cell protective agent comprising a substance that reduces the activity of a protein comprising the same or substantially the same amino acid sequence as the amino acid sequence of amino acid number 1 and after in the amino acid sequence represented by SEQ ID NO:2, 4 or 6, or a salt thereof,
[3] the brain/nerve cell protective agent described in [2] above, wherein the substance that reduces the activity is a neutralizing antibody against a protein comprising the same or substantially the same amino acid sequence as the amino acid sequence of amino acid number 1 and after in the amino acid sequence represented by SEQ ID NO:2, 4 or 6, a partial peptide thereof, or a salt thereof,
[4] a brain/nerve cell protective agent comprising a substance that inhibits the expression of the gene that encodes a polypeptide comprising the same or substantially the same amino acid sequence as the amino acid sequence represented by SEQ ID NO:2, 4 or 6,
[5] the brain/nerve cell protective agent described in [4] above, wherein the substance that inhibits the expression is a nucleic acid comprising a base sequence complementary to a base sequence that encodes a polypeptide having the same or substantially the same amino acid sequence as the amino acid sequence represented by SEQ ID NO:2, 4 or 6, or a portion thereof,
[6] a diagnostic agent for a brain/nerve cell injury, which comprises an antibody against a protein comprising the same or substantially the same amino acid sequence as the amino acid sequence of amino acid number 1 and after in the amino acid sequence represented by SEQ ID NO:2, 4 or 6, a partial peptide thereof, or a salt thereof,
[7] a diagnostic agent for a brain/nerve cell injury, which comprises a nucleic acid comprising a base sequence that encodes a polypeptide comprising the same or substantially the same amino acid sequence as the amino acid sequence represented by SEQ ID NO:2, 4 or 6, or a portion thereof,
[8] a brain/nerve cell protective agent comprising a substance that inhibits the binding of a protein comprising the same or substantially the same amino acid sequence as the amino acid sequence of amino acid number 1 and after in the amino acid sequence represented by SEQ ID NO:2, 4 or 6, or a salt thereof, and a receptor thereof,
[9] a brain/nerve cell protective agent comprising a substance that inhibits the cell stimulation activity of a receptor of a protein comprising the same or substantially the same amino acid sequence as the amino acid sequence of amino acid number 1 and after in the amino acid sequence represented by SEQ ID NO:2, 4 or 6, or a salt thereof,
[10] a brain/nerve cell protective agent comprising a substance that inhibits the expression of the gene that encodes a receptor of a protein comprising the same or substantially the same amino acid sequence as the amino acid sequence of amino acid number 1 and after in the amino acid sequence represented by SEQ ID NO:2, 4 or 6, or a salt thereof,
[11] the brain/nerve cell protective agent described in any of [8] to [10] above, wherein the receptor is a protein comprising the same or substantially the same amino acid sequence as the amino acid sequence represented by SEQ ID NO:8 or 10, or a salt thereof,
[12] a method for screening of a substance that exhibits brain/nerve cell protective action, which comprises using a protein comprising the same or substantially the same amino acid sequence as the amino acid sequence of amino acid number 1 and after in the amino acid sequence represented by SEQ ID NO:2, 4 or 6, a partial peptide thereof, or a salt thereof,
[13] a method for screening of a substance that exhibits brain/nerve cell protective action, which comprises using a protein comprising the same or substantially the same amino acid sequence as the amino acid sequence represented by SEQ ID NO:8 or 10, a partial peptide thereof, or a salt thereof,
[14] a kit for screening of a substance that exhibits brain/nerve cell protective action, which comprises a protein comprising the same or substantially the same amino acid sequence as the amino acid sequence of amino acid number 1 and after in the amino acid sequence represented by SEQ ID NO:2, 4 or 6, a partial peptide thereof, or a salt thereof,
[15] a kit for screening of a substance that exhibits brain/nerve cell protective action, which comprises a protein comprising the same or substantially the same amino acid sequence as the amino acid sequence represented by SEQ ID NO:8 or 10, a partial peptide thereof, or a salt thereof,
[16] a method for screening of a substance that exhibits brain/nerve cell protective action, which comprises using a nucleic acid comprising the base sequence that encodes a polypeptide comprising the same or substantially the same amino acid sequence as the amino acid sequence represented by SEQ ID NO:2, 4 or 6, or a portion thereof,
[17] a method for screening of a substance that exhibits brain/nerve cell protective action, which comprises using a nucleic acid comprising the base sequence that encodes a polypeptide comprising the same or substantially the same amino acid sequence as the amino acid sequence represented by SEQ ID NO:8 or 10, or a portion thereof,
[18] a kit for screening of a substance that exhibits brain/nerve cell protective action, which comprises a nucleic acid comprising the base sequence that encodes a polypeptide comprising the same or substantially the same amino acid sequence as the amino acid sequence represented by SEQ ID NO:2, 4 or 6, or a portion thereof,
[19] a kit for screening of a substance that exhibits brain/nerve cell protective action, which comprises a nucleic acid comprising the base sequence that encodes a polypeptide comprising the same or substantially the same amino acid sequence as the amino acid sequence represented by SEQ ID NO:8 or 10, or a portion thereof,
[20] a method of protecting brain or nerve cells, which comprises administering a substance that suppresses a protein comprising the same or substantially the same amino acid sequence as the amino acid sequence of amino acid number 1 and after in the amino acid sequence represented by SEQ ID NO:2, 4 or 6, or salt thereof, to a subject in need thereof,
[21] use of a substance that suppresses a protein comprising the same or substantially the same amino acid sequence as the amino acid sequence of amino acid number 1 and after in the amino acid sequence represented by SEQ ID NO:2, 4 or 6, or a salt thereof, for producing a brain/nerve cell protective agent, and the like.

### Brief Description of the Drawings

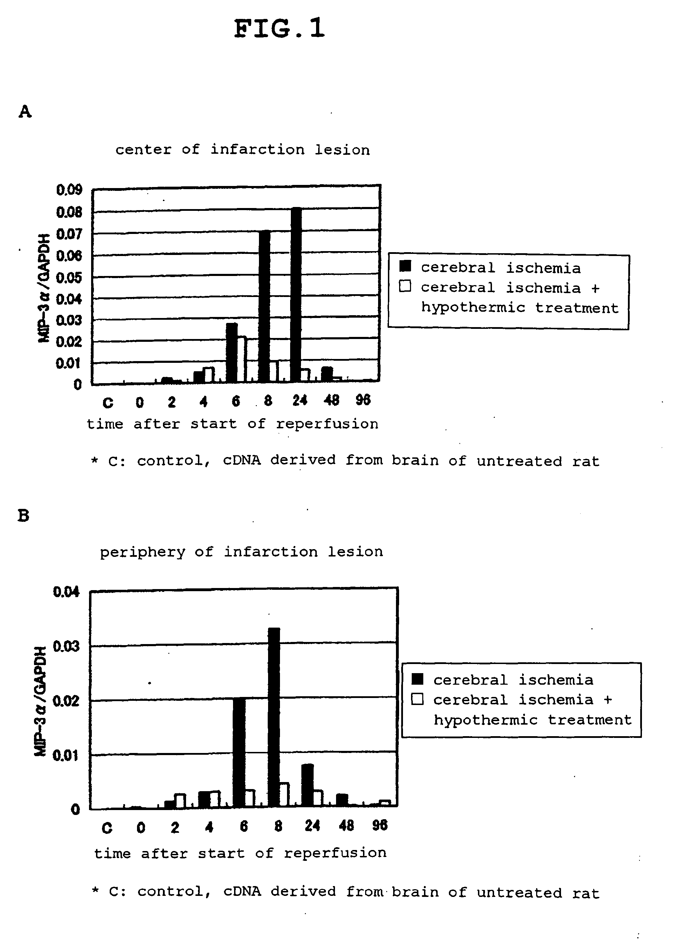

FIG. 1 is a diagram showing changes over time in the expression of the MIP-3α gene after reperfusion at the center (A) and periphery (B) of an infarction lesion in a rat model of cerebral ischemia, and effects of hypothermic treatment thereon. In the figure, C indicates a control, wherein a cDNA derived from the brain of an untreated rat was used as the template.

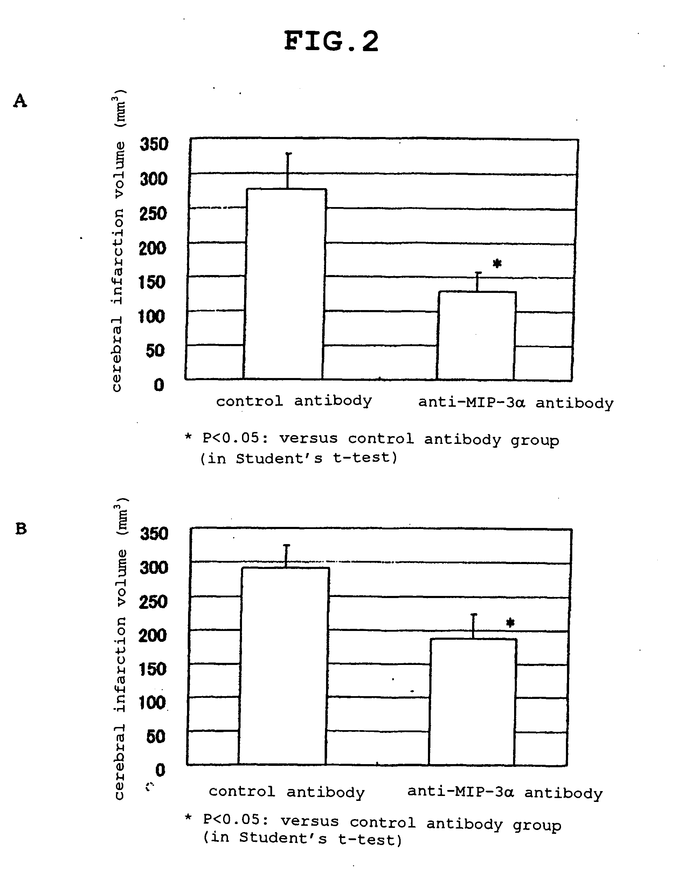

FIG. 2 is a diagram showing effects of an anti-rat MIP-3α monoclonal antibody possessing neutralizing activity on cerebral infarction volume in the rat model of cerebral ischemia. A shows the infarction volume in the brain removed at 1 day after ischemic treatment from a rat receiving the antibody just before ischemic treatment, and B shows the infarction volume in the brain removed at 2 days after ischemic treatment from a rat receiving the antibody just after reperfusion following ischemia. In the figure, * indicates p<0.05 versus the control antibody group in Student's t-test.

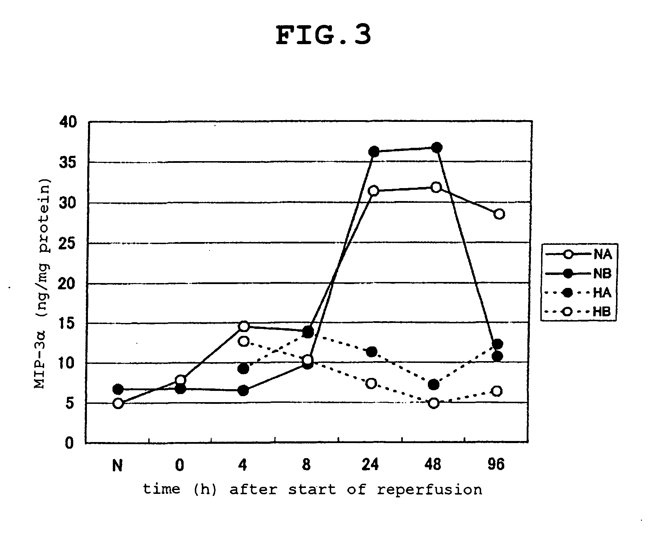

FIG. 3 is a diagram showing changes over time in the production of the MIP-3α protein after reperfusion at the center (NA) and periphery (NB) of an infarction lesion in a rat model of cerebral ischemia, and effects of hypothermic treatment thereon [infarction lesion center (HA), periphery (HB)]. In the figure, N indicates a control, wherein a solubilized fraction was derived from the brain of an untreated rat.

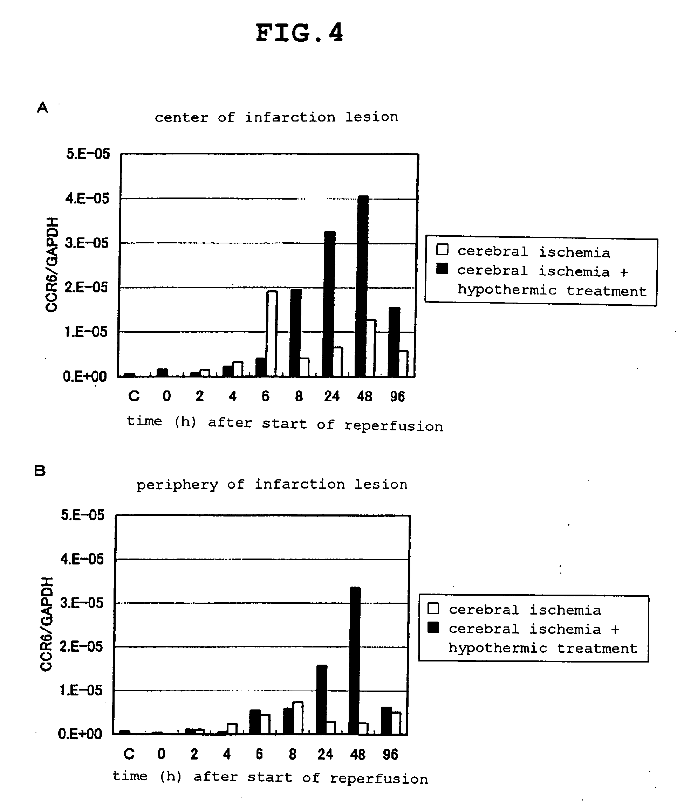

FIG. 4 is a diagram showing changes over time in the expression of the CCR6 gene after reperfusion at the center (A) and periphery (B) of an infarction lesion in a rat model of cerebral ischemia, and effects of hypothermic treatment thereon. In the figure, C indicates a control, wherein a cDNA derived from the brain of an untreated rat was used as the template.

### Best Mode for Carrying Out of the Invention

The present invention relates to a brain/nerve cell protective agent comprising an MIP-3α suppressant. Here, "MIP-3α" refers to a protein comprising the same or substantially the same amino acid sequence as the amino acid sequence of amino acid number 1 and after in the amino acid sequence represented by SEQ ID NO:2, 4 or 6, and "MIP-3α suppressant" refers to a substance that directly or indirectly reduces the expression and/or activity of MIP-3α. In addition, "brain/nerve cell protection" refers to an action to prevent (or at least delay) the cell death of brain cells and/or nerve cells that have experienced or have a risk of a cell injury, and is not particularly limited by cause of cell injury and the like.

The protein comprising the same or substantially the same amino acid sequence as the amino acid sequence of amino acid number 1 and after in the amino acid sequence represented by SEQ ID NO:2, 4 or 6 (hereinafter also referred to as "the MIP-3α of the present invention" or simply as "MIP-3α"), may be a protein derived from any cells [e.g., liver cells, splenocytes, nerve cells, glial cells, β cells of pancreas, bone marrow cells, mesangial cells, Langerhans' cells, epidermic cells, epithelial cells, goblet cells, endothelial cells, smooth muscle cells, fibroblasts, fibrocytes, myocytes, adipocytes, immune cells (e.g., macrophage, T cells, B cells, natural killer cells, mast cells, neutrophil, basophil, eosinophil, monocyte), megakaryocyte, synovial cells, chondrocytes, bone cells, osteoblasts, osteoclasts, mammary gland cells, hepatocytes or interstitial cells or the corresponding precursor cells, stem cells, cancer cells, etc.], or from any tissues where such cells are present [e.g., brain or each region of the brain (e.g., olfactory bulb, amygdaloid nucleus, basal ganglia, hippocampus, thalamus, hypothalamus, cerebral cortex, medulla oblongata and cerebellum), spinal cord, hypophysis, stomach, pancreas, kidney, liver, gonad, thyroid, gall-bladder, bone marrow, adrenal gland, skin, muscle, lung, gastrointestinal tract (e.g., large intestine and small intestine), blood vessel, heart, thymus, spleen, submandibular gland, peripheral blood, prostate, testis, ovary, placenta, uterus, bone, joint, skeletal muscle, etc.] of humans or other warm-blooded animals (e.g., guinea pigs, rats, mice, chickens, rabbits, pigs, sheep, cattle, monkeys etc.). The protein may be also a protein which is synthesized chemically or under a cell-free translation system, or a recombinant protein produced from a transformant into which polynucleotide having a base sequence encoding the above-mentioned amino acid sequence is introduced.

As substantially the same amino acid sequence as the amino acid sequence of amino acid number 1 and after in the amino acid sequence represented by SEQ ID NO:2, 4 or 6, an amino acid sequence showing a homology of about 50% or more, preferably about 60% or more, more preferably about 70% or more, still more preferably about 80% or more, particularly preferably about 90% or more, and most preferably about 95% or more, to any amino acid sequence of amino acid number 1 and after in the amino acid sequence represented by SEQ ID NO:2, 4 or 6, and the like can be mentioned.

As examples of the protein comprising substantially the same amino acid sequence as the amino acid sequence of amino acid number 1 and after in the amino acid sequence represented by SEQ ID NO:2, 4 or 6, a protein comprising substantially the same amino acid sequence as the aforementioned amino acid sequence of amino acid number 1 and after in the amino acid sequence represented by SEQ ID NO:2, 4 or 6, and having substantially the same activity as that of a protein comprising the amino acid sequence for amino acid number 1 and after in the amino acid sequence represented by SEQ ID NO:2, 4 or 6, and the like are preferred.

As examples of substantially the same activity, signal transduction activity, receptor binding activity and the like can be mentioned. Substantially the same quality means that the properties of the proteins are qualitatively (e.g., physiologically or pharmacologically) equivalent to each other. Accordingly, it is preferable that the proteins be equivalent to each other in terms of signal transduction activity or receptor binding activity (e.g., about 0.01 to 100 times, preferably about 0.1 to 10 times, more preferably 0.5 to 2 times), but quantitative factors such as the extent of activity and protein molecular weight may be different.

A measurement of signal transduction activity is conducted according to a method known per se. For example, a stimulatory activity on CCR6-expressing cells (for example, activity to promote or suppress an increase in intracellular cAMP concentration, liberation of intracellular Ca²⁺, production of inositol phosphate, changes of cell membrane potential, phosphorylation or dephosphorylation of intracellular protein, activation of c-fos, reduction of pH and the like, mediated by CCR6) is measured by a known method. Specifically, a plasmid incorporating a DNA that encodes CCR6 is introduced into, for example, Chinese hamster ovarian cells (e.g., CHO-K1 cells; described in the Journal of Experimental Medicine, Vol. 108, p. 945, 1958), CHO-K1 cells allowed to express CCR6 at a high level are cultured in the presence of MIP-3α, and signal transduction activity via the CCR6 expressed on the cell membrane (for example, activity to promote or suppress an increase or decrease in concentration of intracellular cAMP, liberation of intracellular Ca²⁺, production of inositol phosphate, changes of cell membrane potential, phosphorylation or dephosphorylation of intracellular protein, activation of c-fos, reduction of pH and the like) is measured by a known method.

A measurement of receptor binding activity can be conducted according to a method known per se. For example, receptor binding activity can be measured by (1) bringing the MIP-3α protein, previously labeled, into contact with a CCR6-expressing cell or a membrane fraction of the cell, and measuring the amount of labeled MIP-3α protein bound to the cell or the membrane fraction, or by (2) bringing a labeled MIP-3α protein into contact with the CCR6 protein expressed on the cell membrane by culturing a transformant containing a DNA that encodes the CCR6 protein, and measuring the amount of labeled MIP-3α bound to the cell or the membrane fraction.

As the DNA that encodes CCR6 used in the above-described activity measurements, a DNA that encodes a protein comprising the same or substantially the same amino acid sequence as the amino acid sequence represented by SEQ ID NO:8, 10 or 14 can be mentioned (see, for example, Baba et al., Journal of Biological Chemistry, Vol. 272, pp. 14893-14898, 1997).

In addition, examples of the MIP-3α of the present invention include proteins comprising (i) an amino acid sequence having one or two or more amino acids (preferably about 1 to 30, more preferably about 1 to 10, and still more preferably several (1 to 5) amino acids) deleted from the amino acid sequence of amino acid number 1 and after in the amino acid sequence represented by SEQ ID NO:2, 4 or 6, (ii) an amino acid sequence having one or two or more amino acids (preferably about 1 to 30, more preferably about 1 to 10, and more preferably several (1 to 5) amino acids) added to the amino acid sequence of amino acid number 1 and after in the amino acid sequence represented by SEQ ID NO:2, 4 or 6, (iii) an amino acid sequence having one or two or more amino acid (preferably about 1 to 30, more preferably about 1 to 10, and more preferably several (1 to 5) amino acids) inserted to the amino acid sequence of amino acid number 1 and after in the amino acid sequence represented by SEQ ID NO:2, 4 or 6, (iv) an amino acid sequence having one or two or more amino acids (preferably about 1 to 30, more preferably about 1 to 10, and still more preferably several (1 to 5) amino acids) substituted with other amino acids in the amino acid sequence of amino acid number 1 and after in the amino acid sequence represented by SEQ ID NO:2, 4 or 6, or (v) an amino acid sequence comprising a combination thereof, which are what is called a mutein.

When an amino acid sequence is inserted, deleted or substituted as described above, the position of the insertion, deletion or substitution is not particularly limited.

In the present specification, the proteins are represented in accordance with a common way of describing the peptides, so that the N-terminus (amino terminus) is described at the left hand and the C-terminus (carboxyl terminus) is described at the right hand. In the MIP-3α of the present invention including the protein comprising the amino acid sequence starting after the amino acid No. 1 among the amino acid sequences represented by SEQ ID NO: 2, 4 or 6, any of a carboxyl group (-COOH), a carboxylate (-COO⁻) , an amide (-CONH₂) or an ester (-COOR) may be at the C-terminus.

As used herein, R in the ester includes, for example, a C₁₋₆ alkyl group such as methyl, ethyl, n-propyl, isopropyl, n-butyl, etc.; a C₃₋₈ cycloalkyl group such as cyclopentyl, cyclohexyl, etc.; a C₆₋₁₂ aryl group such as phenyl, α-naphthyl, etc.; a phenyl-C₁₋₂ alkyl group, e.g., benzyl, phenethyl, etc., or a C₇₋₁₄ aralkyl group, e.g., an α-naphthyl-C₁₋₂-alkyl group such as α-naphthylmethyl, etc.; a pivaloyloxymethyl group and the like.

When the MIP-3α of the present invention has a carboxyl group (or carboxylate) at a position other than the C-terminus, it may be amidated or esterified and such an amide or ester is also included within the MIP-3α of the present invention. As the ester group herein, for example, the same esters as those described with respect to the above C-terminus are used.

Furthermore, examples of the protein of the present invention include those wherein the amino group of the amino acid residue (e.g., methionine residue) at the N-terminus is protected with a protecting group (e.g., a C₁₋₆ acyl group e.g., a C₁₋₆ alkanoyl group such as a formyl group, an acetyl group, etc.); those wherein the glutamyl group at the N-terminal region, which may be cleaved in vivo, is pyroglutaminated; those wherein a substituent (e.g., -OH, -SH, an amino group, an imidazole group, an indole group, a guanidino group, etc.) on the side chain of an amino acid in the molecule is protected with a suitable protecting group (e.g., a C₁₋₆ acyl group e.g., a C₁₋₆ alkanoyl group such as a formyl group, an acetyl group, etc.), and conjugated proteins such as glycoproteins having sugar chains bound thereto.

As preferable examples of the MIP-3α of the present invention, mature human MIP-3α, which comprises the amino acid sequence represented by amino acid numbers 1 to 70 in the amino acid sequence represented by SEQ ID NO:2; mature rat MIP-3α, which comprises the amino acid sequence represented by amino acid numbers 1 to 71 in the amino acid sequence represented by SEQ ID NO:4; mature mouse MIP-3α, which comprises the amino acid sequence represented by amino acid numbers 1 to 70 in the amino acid sequence represented by SEQ ID NO:6; and the like can be mentioned.

The partial peptide of the MIP-3α of the present invention may be any peptide having the aforementioned partial amino acid sequence of the MIP-3α of the present invention, preferably having substantially the same activity as the MIP-3α of the present invention. As examples of substantially the same activity, signal transduction activity, receptor binding activity, epitope activity and the like can be mentioned. "Substantially the same quality" has the same definition as above.

For example, a peptide having at least 20 or more, preferably 30 or more, more preferably 40 or more, and still more preferably 50 or more, partial amino acid sequences of the constituent amino acid sequences of the MIP-3α of the present invention, and the like, are used.

In addition, the partial peptide of the MIP-3α of the present invention may have one or two or more amino acids (preferably about 1 to 10, and more preferably several (1 to 5) amino acids) deleted from the amino acid sequence thereof, or may have one or two or more amino acids (preferably about 1 to 20, more preferably about 1 to 10, and still more preferably several (1 to 5) amino acids) added to the amino acid sequence thereof, or may have one or two or more amino acid (preferably about 1 to 20, more preferably about 1 to 10, and still more preferably several (1 to 5) amino acids) inserted to the amino acid sequence thereof, or may have one or two or more amino acids (preferably about 1 to 10, and more preferably several (1 to 5) amino acids) substituted with other amino acids in the amino acid sequence thereof.

For the partial peptide of the MIP-3α of the present invention, the C-terminus may be any of a carboxyl group (-COOH), a carboxylate (-COO⁻), an amide (-CONH₂), and an ester (-COOR). Here, as the R in the ester, the same as those mentioned above with respect to the MIP-3α of the present invention can be mentioned. When the partial peptide of the MIP-3α of the present invention has a carboxyl group (or a carboxylate) at a position other than the C terminus, a partial peptide wherein the carboxyl group is amidated or esterified is also included in the partial peptide. In this case, as the ester, for example, the C-terminal esters described above and the like are used.

Furthermore, the partial peptide also includes a peptide wherein the amino group of the N-terminal amino acid residue (e.g., methionine residue) is protected by a protecting group, a peptide wherein the N-terminal is cleaved in vivo and thus produced glutamine residue is converted to pyroglutamate, a peptide wherein a substituent on a side chain of an amino acid in the molecule is protected by an appropriate protecting group, a conjugated peptide such as what is called a glycopeptide having a sugar chain bound thereto, and the like, as with the aforementioned MIP-3α of the present invention.

The partial peptide of the MIP-3α of the present invention can be used as an antigen for preparing an antibody.

The MIP-3α of the present invention or a partial peptide thereof may be the free form or a salt. Such salts include physiologically acceptable salts with acids or bases, preferably physiologically acceptable acid addition salts. Examples of such salts include salts with inorganic acids (e.g., hydrochloric acid, phosphoric acid, hydrobromic acid and sulfuric acid), salts with organic acids (e.g., acetic acid, formic acid, propionic acid, fumaric acid, maleic acid, succinic acid, tartaric acid, citric acid, malic acid, oxalic acid, benzoic acid, methanesulfonic acid and benzenesulfonic acid), etc.

The MIP-3α of the present invention or a salt thereof can be produced from the aforementioned cell or tissue of a human or another warm-blooded animal by a method for protein purification known per se. Specifically, the MIP-3α of the present invention or a salt thereof can be purified and isolated by homogenizing a tissue or cells of a human or another warm-blooded animal, then conducting extraction with acid and the like, and subjecting the resulting extract to a combination of chromatographies such as reversed phase chromatography and ion exchange chromatography.

To synthesize the MIP-3α, a partial peptide thereof, a salt thereof or an amide thereof of the present invention, commercially available resins that are used for protein synthesis may be used. Examples of such resins include a chloromethyl resin, a hydroxymethyl resin, a benzhydrylamine resin, an aminomethyl resin, a 4-benzyloxybenzyl alcohol resin, a 4-methylbenzhydrylamine resin, a PAM resin, a 4-hydroxymethylmethylphenyl acetamidomethyl resin, a polyacrylamide resin, a 4-(2',4'-dimethoxyphenylhydroxymethyl)phenoxy resin, a 4-(2',4'-dimethoxyphenyl-Fmoc-aminoethyl)phenoxy resin, etc. Using these resins, amino acids in which α-amino groups and functional groups on the side chains are appropriately protected are condensed on the resin in the order of the sequence of the objective protein according to various condensation methods known in the art. At the end of the reaction, the protein or a partial peptide is excised from the resin and at the same time, the protecting groups are removed. Then, intramolecular disulfide bond-forming reaction is performed in a highly diluted solution to give the objective protein or a partial peptide (peptide) or amides thereof.

For condensation of the protected amino acids described above, a variety of activation reagents for protein synthesis may be used, and carbodiimides are particularly preferable. Examples of such carbodiimides include DCC, N,N'-diisopropylcarbodiimide, N-ethyl-N'-(3-dimethylaminopropyl)carbodiimide, etc. For activation by these reagents, the protected amino acids in combination with a racemization inhibitor (e.g., HOBt and HOOBt) are added directly to the resin, or the protected amino acids are previously activated in the form of symmetric acid anhydrides, HOBt esters or HOOBt esters, followed by adding the thus activated protected amino acids to the resin.

Solvents suitable for use to activate the protected amino acids or condense with the resin may be appropriately chosen from solvents known to be usable for protein condensation reactions. Examples of such solvents are acid amides such as N,N-dimethylformamide, N,N-dimethylacetamide, N-methylpyrrolidone, etc.; halogenated hydrocarbons such as methylene chloride, chloroform, etc.; alcohols such as trifluoroethanol, etc.; sulfoxides such as dimethylsulfoxide, etc.; pyridine, etc.; ethers such as dioxane, tetrahydrofuran, etc.; nitriles such as acetonitrile, propionitrile, etc.; esters such as methyl acetate, ethyl acetate, etc.; and appropriate mixtures of these solvents. The reaction temperature is appropriately chosen from the range known to be applicable to protein binding reactions and is usually selected in the range of approximately -20°C to 50°C. The activated amino acid derivatives are used generally in an excess of 1.5 to 4 times. The condensation is examined by a test using the ninhydrin reaction; when the condensation is insufficient, the condensation can be completed by repeating the condensation reaction without removal of the protecting groups. When the condensation is yet insufficient even after repeating the reaction, an influence thereof on the subsequent reactions can be prevented by acetylating the unreacted amino acids with acetic anhydride or acetylimidazole.

The protection of the functional group which should not be involved in the reaction of the starting materials and the protecting group, and elimination of the protecting group, activation of the functional group involved in the reaction, etc. can be suitably selected from known groups or known means.

Examples of the protecting groups for the amino groups of the starting materials include Z, Boc, t-pentyloxycarbonyl, isobornyloxycarbonyl, 4-methoxybenzyloxycarbonyl, Cl-Z, Br-Z, adamantyloxycarbonyl, trifluoroacetyl, phthaloyl, formyl, 2-nitrophenylsulphenyl, diphenylphosphinothioyl, Fmoc, etc.

A carboxyl group can be protected by, for example, alkyl esterification (e.g., esterification in the form of linear, branched or cyclic alkyl (e.g., methyl, ethyl, propyl, butyl, t-butyl, cyclopentyl, cyclohexyl, cycloheptyl, cyclooctyl, 2-adamantyl) esters, aralkyl esterification (e.g., esterification in the form of benzyl ester, 4-nitrobenzyl ester, 4-methoxybenzyl ester, 4-chlorobenzyl ester, benzhydryl ester), phenacyl esterification, benzyloxycarbonyl hydrazidation, t-butoxycarbonyl hydrazidation, trityl hydrazidation or the like.

The hydroxyl group of serine can be protected through, for example, its esterification or etherification. Examples of the groups appropriately used for the esterification include a lower (C₁₋₆) alkanoyl group such as an acetyl group, an aroyl group such as benzoyl group, and a group derived from carbonic acid such as a benzyloxycarbonyl group, an ethoxycarbonyl group, etc. Examples of a group appropriately used for the etherification include a benzyl group, a tetrahydropyranyl group, a t-butyl group, etc.

Examples of the groups for protecting the phenolic hydroxyl group of tyrosine include Bzl, Cl₂-Bzl, 2-nitrobenzyl, Br-Z, t-butyl, etc.

Examples of groups used to protect the imidazole moiety of histidine include Tos, 4-methoxy-2,3,6-trimethylbenzenesulfonyl, DNP, benzyloxymethyl, Bum, Boc, Trt, Fmoc, etc.

To eliminate (split off) the protecting groups, there are used a catalytic reduction under hydrogen gas flow in the presence of a catalyst such as Pd-black or Pd-carbon; an acid treatment with anhydrous hydrogen fluoride, methanesulfonic acid, trifluoromethane-sulfonic acid or trifluoroacetic acid, or a mixture solution of these acids; a treatment with a base such as diisopropylethylamine, triethylamine, piperidine or piperazine; and a reduction with sodium in liquid ammonia. The elimination of the protecting group by the acid treatment described above is performed generally at a temperature of approximately -20°C to 40°C. In the acid treatment, it is efficient to add a cation scavenger such as anisole, phenol, thioanisole, m-cresol, p-cresol, dimethylsulfide, 1,4-butanedithiol and 1,2-ethanedithiol. Furthermore, a 2,4-dinitrophenyl group used as the protecting group for the imidazole of histidine is removed by a treatment with thiophenol. A formyl group used as the protecting group of the indole of tryptophan is eliminated by the above-mentioned acid treatment in the presence of 1,2-ethanedithiol or 1,4-butanedithiol, as well as by a treatment with an alkali such as a dilute sodium hydroxide solution and dilute ammonia.

Examples of the activated carboxyl groups in the starting materials include the corresponding acid anhydrides, azides, activated esters [(esters with alcohols (e.g., pentachlorophenol, 2,4,5-trichlorophenol, 2,4-dinitrophenol, cyanomethyl alcohol, p-nitrophenol, HONB, N-hydroxysuccimide, N-hydroxyphthalimide, HOBt)]. As one in which the amino groups are activated in the starting material, the corresponding phosphoric amides are employed.

In another method for obtaining the amides of the protein or partial peptide, for example, the α-carboxyl group of the carboxy terminal amino acid is first protected by amidation, and the peptide (protein) chain at the amino group side is then extended to a desired length. Thereafter, a protein or partial peptide in which only the protecting group of the N-terminal α-amino group in the peptide chain has been eliminated and a protein or partial peptide in which only the protecting group of the C-terminal carboxyl group has been eliminated are prepared. These proteins or peptides are condensed in a mixture of the solvents described above. The details of the condensation reaction are the same as described above. After the protected protein or protected peptide obtained by the condensation is purified, all the protecting groups are eliminated by the method described above to give the desired crude protein or peptide. This crude protein or peptide is purified by various known purification means. Lyophilization of the major fraction gives the amide of the desired protein or peptide.

The esterified protein or peptide can be obtained by, for example, condensing the α-carboxyl group of the carboxy terminal amino acid with a desired alcohol to prepare an amino acid ester, which is followed by procedure similar to the preparation of the amidated protein or peptide above.

A partial peptide of the MIP-3α of the present invention or a salt thereof can be produced according to a method of peptide synthesis known per se, or by cleaving the MIP-3α of the present invention with an appropriate peptidase. The method of peptide synthesis may be any of, for example, a solid phase synthesis process and a liquid phase synthesis process. That is, a partial peptides or amino acids that may compose a partial peptide of the MIP-3α of the present invention are condensed with the residual portion. When the product has protecting groups, the desired peptide can be obtained by eliminating the protecting groups. The publicly known condensation and elimination of protecting group include the methods described in (1) - (5) below.
(1) M. Bodanszky and M.A. Ondetti: Peptide Synthesis, Interscience Publishers, New York (1966)
(2) Schroeder and Luebke: The Peptide, Academic Press, New York (1965)
(3) Nobuo Izumiya, et al.: Basics and experiments of peptide synthesis, Maruzen Co. (1975)
(4) Haruaki Yajima and Shunpei Sakakibara: Biochemical Experiment 1, Chemistry of Proteins IV, 205 (1977)
(5) Haruaki Yajima, ed.: A sequel to Development of Pharmaceuticals, Vol. 14, Peptide Synthesis, Hirokawa Shoten

After the reaction, the partial peptide can be purified and isolated using in combination ordinary methods of purification, for example, solvent extraction, distillation, column chromatography, liquid chromatography, recrystallization, and the like. When the partial peptide obtained by the above-described method is a free form, it can be converted to an appropriate salt by a known method or a method based thereon; conversely, when the protein is obtained in the form of a salt, the salt can be converted to a free form or another salt by a known method or a method based thereon.

Furthermore, the MIP-3α of the present invention or a partial peptide thereof can also be produced by culturing a transformant containing a DNA that encodes the MIP-3α of the present invention or a partial peptide thereof, and separating and purifying the MIP-3α of the present invention or a partial peptide thereof from the resulting culture. Alternatively, the MIP-3α of the present invention or a partial peptide thereof can also be synthesized by in vitro translation using a cell-free protein translation system that comprises a rabbit reticulocyte lysate, wheat germ lysate, *Escherichia coli* lysate and the like, with RNA corresponding to the DNA as the template. Alternatively, it can be synthesized using a cell-free transcription/translation system containing RNA polymerase, with the DNA as the template.

The polynucleotide that encodes the MIP-3α of the present invention or a partial peptide thereof may be any one comprising the aforementioned base sequence that encodes the MIP-3α of the present invention or a partial peptide thereof. DNA is preferred. The polynucleotide may be any of genomic DNA, a genomic DNA library, cDNA derived from the above-described cell or tissue, a cDNA library derived from the above-described cell or tissue, and synthetic DNA. The vector used for the library may be any of a bacteriophage, a plasmid, a cosmid, a phagemid and the like. The polynucleotide can also be amplified directly by the Reverse Transcriptase Polymerase Chain Reaction (hereinafter abbreviated as RT-PCR method) using total RNA or an mRNA fraction prepared from the above-described cell or tissue.

For example, the DNA that encodes the MIP-3α of the present invention may be any one of a DNA that comprises the base sequence represented by SEQ ID NO:1, 3 or 5, and a DNA that comprises a base sequence capable of hybridizing to the base sequence represented by SEQ ID NO:1, 3 or 5 under highly stringent conditions, and that encodes a protein having substantially the same activity as that of the aforementioned protein comprising the amino acid sequence for amino acid number 1 and after in the amino acid sequence represented by SEQ ID NO:2, 4 or 6.

As examples of the DNA capable of hybridizing to the base sequence represented by SEQ ID NO:1, 3 or 5 under highly stringent conditions, a DNA comprising a base sequence showing a homology of about 50% or more, preferably about 60% or more, more preferably about 70% or more, still more preferably about 80% or more, particularly preferably about 90% or more, and most preferably about 95% or more, to the base sequence represented by SEQ ID NO:1, 3 or 5, and the like, are used.

The hybridization can be performed by known methods or by modifications of these methods, for example, according to the method described in Molecular Cloning, 2nd (J. Sambrook et al., Cold Spring Harbor Lab. Press, 1989). If a commercially available library is used, hybridization can be performed according to the method described in the instructions of the attached manufacturer's protocol. More preferably, the hybridization can be performed under highly stringent conditions.

The highly stringent conditions used herein are, for example, those in a sodium concentration at about 19 mM to about 40 mM, preferably about 19 mM to about 20 mM at a temperature of about 50°C to about 70°C, preferably about 60°C to about 65°C. In particular, the hybridization condition in a sodium concentration of about 19 mM at a temperature of about 65°C is most preferable.

More preferably, the DNA that encodes the MIP-3α of the present invention is a DNA comprising the base sequence represented by SEQ ID NO:1, 3 or 5.

The DNA that encodes a partial peptide of the MIP-3_{α} of the present invention may be any DNA that comprises a base sequence that encodes the same or substantially the same amino acid sequence as a portion of the amino acid sequence of amino acid number 1 and after in the amino acid sequence represented by SEQ ID NO:2, 4 or 6, and that encodes the aforementioned peptide possessing substantially the same activity as the MIP-3α of the present invention (e.g., signal transduction activity, receptor binding activity, epitope activity and the like). In addition, the DNA may be any one of a genomic DNA, a genomic DNA library, a cDNA derived from the above-described cell or tissue, a cDNA library derived from the above-described cell or tissue, and a synthetic DNA. The vector used for the library may be any one of a bacteriophage, a plasmid, a cosmid, a phagemid and the like. The DNA can also be amplified directly by the RT-PCR method using a mRNA fraction prepared from the above-described cell or tissue.

Specifically, as examples of the DNA that encodes a partial peptide of the MIP-3α of the present invention, (1) a DNA having a partial base sequence of a DNA comprising the base sequence represented by SEQ ID NO:1, 3 or 5, or (2) a DNA that encodes a peptide comprising a base sequence capable of hybridizing to a DNA having the base sequence represented by SEQ ID NO:1, 3 or 5 under highly stringent conditions, and having substantially the same activity as a protein comprising the amino acid sequence encoded by the DNA (e.g., signal transduction activity, receptor binding activity, epitope activity and the like), and the like, are used.

The DNA capable of hybridizing to the base sequence represented by SEQ ID NO:1, 3 or 5 has the same definition as above.

The method and highly stringent conditions for hybridization are the same as those mentioned above.

A DNA that encodes the MIP-3α of the present invention or a partial peptide thereof can be cloned by amplifying by the PCR method using a synthetic DNA primer having a portion of a base sequence that encodes the protein or peptide, or hybridizing a DNA incorporated in an appropriate vector with a labeled DNA fragment or synthetic DNA that encodes a portion or the entire region of the MIP-3α of the present invention. Hybridization can be conducted according to, for example, a method described in Molecular Cloning, 2nd edition (ibidem) and the like. When a commercially available library is used, hybridization can be conducted according to the method described in the manufacturer's protocol attached thereto.

Conversion of the base sequence of DNA can be effected by per se known methods such as an ODA-LA PCR method, a Gapped duplex method and a Kunkel method or an analogue thereof using a known kit such as Mutan™-super Express Km (manufactured by Takara Shuzo Co., Ltd.) and Mutan™-K (manufactured by Takara Shuzo Co., Ltd.).

The cloned DNA can be used as it is, depending upon purpose or if desired, after digestion with a restriction enzyme or after addition of a linker thereto. The DNA may have ATG as a translation initiation codon at the 5' end thereof and may further have TAA, TGA or TAG as a translation termination codon at the 3' end thereof. These translation initiation and termination codons may also be added by using a suitable synthetic DNA adapter.

An expression vector containing a DNA that encodes the MIP-3α of the present invention or a partial peptide thereof can be produced by, for example, (a) cleaving the desired DNA fragment from the DNA that encodes the MIP-3α of the present invention or a partial peptide thereof, and (b) ligating the DNA fragment to downstream of a promoter in an appropriate expression vector.

Examples of the expression vector include plasmids derived from *E. coli* (e.g., pBR322, pBR325, pUC12, pUC13); plasmids derived from *Bacillus subtilis* (e.g., pUB110, pTP5, pC194); plasmids derived from yeast (e.g., pSH19, pSH15); bacteriophages such as a λ phage; animal virus vectors such as retrovirus, vaccinia virus and baculovirus; pA1-11, pXT1, pRc/CMV, pRc/RSV, pcDNAI/Neo; or the like.

The promoter may be any promoter if it matches well with a host to be used for gene expression.

When animal cells are used as the host, examples of the promoter include an SR α promoter, SV40 promoter, an LTR promoter, a CMV (cytomegalovirus) promoter, an HSV-TK promoter, etc., and preferably a CMV promoter, an SRα promoter, etc.

When the host is bacteria of the genus Escherichia, preferred examples of the promoter include a trp promoter, a lac promoter, a recA promoter, a λP_{L} promoter, an Ipp promoter, a T7 promoter etc.

When bacteria of the genus Bacillus are used as the host, preferred example of the promoter are an SPO1 promoter, an SPO2 promoter, a penP promoter, etc.

When yeast is used as the host, preferred examples of the promoter are a PHO5 promoter, a PGK promoter, a GAP promoter, an ADH promoter, etc.

When insect cells are used as the host, preferred examples of the promoter include polyhedrin prompter, P10 promoter, etc.

The expression vector may further optionally contain an enhancer, a splicing signal, a poly A-addition signal, a selection marker, an SV40 replication origin (hereinafter, sometimes abbreviated as SV40 ori), etc. in addition to the foregoing examples. Examples of the selection marker include a dihydrofolate reductase gene (hereinafter, sometimes abbreviated as dhfr) [methotrexate (MTX) resistance], an ampicillin resistant gene (hereinafter, sometimes abbreviated as amp^{r}), a neomycin resistant gene (hereinafter, sometimes abbreviated as neo^{r}, G418 resistance), etc. In particular, when dhfr gene is used as the selection marker together with dhfr genedeficient Chinese hamster cells, the objective genes can also be selected on a thymidine free medium.

In addition, as necessary, the signal sequence matching with the host may be added to the N-terminus side of the MIP-3α of the present invention or a partial peptide thereof, in place of a native signal sequence (for example, the amino acid sequence before amino acid number 1 in the amino acid sequence represented by SEQ ID NO:2, 4 or 6 and the like) . When the host is a bacterium of the genus *Escherichia,* the PhoA/signal sequence, the OmpA/signal sequence and the like can be utilized; when the host is a bacterium of the genus *Bacillus,* the α-amylase/signal sequence, the subtilisin/signal sequence and the like can be utilized; when the host is a yeast, the MFα/signal sequence, the SUC2/signal sequence and the like can be utilized; when the host is an animal cell, the insulin/signal sequence, the α-interferon/signal sequence, antibody molecule/signal sequences and the like can be utilized.

A transformant comprising the thus-obtained "DNA that encodes the MIP-3α of the present invention or a partial peptide thereof" can be produced by transforming the host with an expression vector containing the DNA according to a known method. Here, as the expression vector, those mentioned above can be mentioned.

As examples of the host, a bacterium of the genus *Escherichia,* a bacterium of the genus *Bacillus,* a yeast, an insect cell, an insect, an animal cell and the like can be used.

As the bacteria belonging to the genus Escherichia, there can be used, for example, *Escherichia coli* K12 DH1 [Proceedings of the National Academy of Sciences of the USA, Vol. 60, 160 (1968)], *Escherichia coli* JM103 (Nucleic Acids Research, Vol. 9, 309 (1981)), *Escherichia coli* JA221 (Journal of Molecular Biology, Vol. 120, 517 (1978)), *Escherichia coli* HB101 (Journal of Molecular Biology, Vol. 41, 459 (1969)), *Escherichia coli* C600 (Genetics, Vol. 39, 440 (1954)), etc.

As the bacteria belonging to the genus Bacillus, there can be used, for example, *Bacillus subtilis* MI114 (Gene, Vol. 24, 255 (1983)), *Bacillus subtilis* 207-21 (Journal of Biochemistry, Vol. 95, 87 (1984)), etc.

As the yeast, there can be used, for example, Saccharomyces cerevisiae AH22, AH22R⁻, NA87-11A, DKD-5D, 20B-12, Schizosaccharomyces pombe NCYC1913, NCYC2036, Pichia pastoris KM71, etc.

As the insect cells, there can be used, for example, for the virus AcNPV, established cell line derived from cabbage armyworm (Spodoptera frugiperda cells; Sf cells), MG1 cells derived from mid-intestine of Trichoplusia ni; High Five™ cells derived from egg of Trichoplusia ni, cells derived from Mamestra brassicae, cells derived from Estigmena acrea, etc.; and for the virus BmNPV, established cell line derived from Bombyx mori (Bombyx mori N cells; BmN cells), etc. As the Sf cells, there can be used, for example, Sf9 cells (ATCC CRL1711) and Sf21 cells (both cells are described in Vaughn, J. L. et al., In Vivo, 13, 213-217 (1977), etc.

As the insect, there can be used, for example, a larva of Bombyx mori (Maeda, et al., Nature, Vol. 315, 592 (1985)) .

As the animal cells, there can be used, for example, monkey COS-7 cells, Vero cells, Chinese hamster cells CHO (hereinafter, referred to as CHO cells), dhfr genedeficient Chinese hamster cells CHO (hereinafter, abbreviated as CHO (dhfr⁻) cell), mouse L cells, mouse AtT-20, mouse myeloma cells, mouse ATDC5 cells, rat GH3 cells, human FL cells, etc.

Transformation can be performed according to a known method depending on the kinds of the host.

Bacteria belonging to the genus Escherichia can be transformed, for example, by the method described in Proceedings of the National Academy of Sciences of the USA, Vol. 69, 2110 (1972), Gene, Vol. 17, 107 (1982), etc.

Bacteria belonging to the genus Bacillus can be transformed, for example, by the method described in Molecular & General Genetics, Vol. 168, 111 (1979).

Yeast can be transformed, for example, by the method described in Methods in Enzymology, Vol. 194, 182-187 (1991), Proceedings of the National Academy of Sciences of the USA, Vol. 75, 1929 (1978), etc.

The insect cells or the insects can be transformed, for example, according to the method described in Bio/Technology, Vol. 6, 47-55 (1988), etc.

The animal cells can be transformed, for example, according to the method described in Saibo Kogaku (Cell Engineering), Extra issue 8, Shin Saibo Kogaku Jikken Protocol (New Cell Engineering Experimental Protocol), 263-267 (1995), published by Shujunsha, or Virology, Vol. 52, 456 (1973).

Cultivation of a transformant can be performed according to a known method depending on the kinds of the host.

For example, when the host is a bacterium belonging to the genus Escherichia or the genus Bacillus, a preferred medium for culture is a liquid medium. Preferably the medium contains materials required for growth of the transformant such as carbon sources, nitrogen sources, inorganic materials, etc. Examples of the carbon sources include glucose, dextrin, soluble starch, sucrose, etc. Examples of the nitrogen sources include inorganic or organic materials such as ammonium salts, nitrate salts, corn steep liquor, peptone, casein, meat extract, soybean cake, potato extract, etc. Examples of the inorganic materials include calcium chloride, sodium dihydrogenphosphate, magnesium chloride, etc. In addition, yeast extract, vitamins, growth promoting factors etc. may also be added to the medium. Preferably, pH of the medium is adjusted to about 5 to about 8.

The medium for cultivation of a transformant when the host is the bacterium belonging to the genus Escherichia, is preferably, for example, M9 medium supplemented with glucose and Casamino acids (Miller, Journal of Experiments in Molecular Genetics, 431-433, Cold Spring Harbor Laboratory, New York, 1972). If necessary, chemicals such as 3β-indolylacrylic acid can be added to the medium thereby to function the promoter efficiently.

When a bacterium belonging to the genus Escherichia is used as the host, the transformant is usually cultivated at about 15°C to about 43°C for about 3 hours to about 24 hours. If necessary, the culture may be aerated or agitated.

When a bacterium belonging to the genus Bacillus is used as the host, the transformant is cultivated usually at about 30°C to about 40°C for about 6 hours to about 24 hours. If necessary, the culture can be aerated or agitated.

When yeast is used as the host, the transformant is cultivated, for example, in Burkholder's minimal medium [Bostian, K. L. et al., Proceedings of the National Academy of Sciences of the USA, Vol. 77, 4505 (1980)] or in SD medium supplemented with 0.5% Casamino acids [Bitter, G. A. et al., Proceedings of the National Academy of Sciences of the USA, Vol. 81, 5330 (1984)). Preferably, pH of the medium is adjusted to about 5 to about 8. The transformant is usually cultivated at about 20°C to about 35°C for about 24 hours to about 72 hours. If necessary, the culture can be aerated or agitated.

When an insect cell or an insect is used as the host, the transformant is cultivated in, for example, Grace's Insect Medium (Grace, T. C. C., Nature, vol. 195, 788 (1962)) to which a suitable additive such as 10% inactivated bovine serum is added. Preferably, pH of the medium is adjusted to about 6.2 to about 6.4. The transformant is usually cultivated at about 27°C to about 50°C for about 3 days to about 5 days and, if necessary and desired, the culture can be aerated or agitated.

When an animal cell is employed as the host, the transformant is cultivated in, for example, MEM medium containing about 5% to about 20% fetal bovine serum (Science, vol. 122, 501 (1952)), DMEM medium; Virology, vol. 8, 396 (1959)), RPMI 1640 medium (The Journal of the American Medical Association, vol. 199, 519 (1967)), 199 medium (Proceeding of the Society for the Biological Medicine, vol. 73, 1 (1950)), etc. Preferably, pH of the medium is adjusted to about 6 to about 8. The transformant is usually cultivated at about 30°C to about 40°C for about 15 hours to about 60 hours and, if necessary and desired, the culture can be aerated or agitated.

As described above, the MIP-3α of the present invention or a partial peptide thereof can be produced in the cells, in the cell membrane, or outside the cells of the transformant.

Separation and purification of the MIP-3α of the present invention or a partial peptide thereof from the above-described culture can, for example, be conducted by the following method.

In extracting the MIP-3α of the present invention or a partial peptide thereof from cultured bacteria or cells, a method wherein bacteria or cells are harvested by a known method after completion of cultivation, this is suspended in an appropriate buffer solution, the bacteria or cells are disrupted by ultrasound, lysozyme and/or freeze-drying and the like, after which a crude extract of protein (peptide) is obtained by centrifugation or filtration, and the like are appropriately used. In the buffer solution, a protein denaturant such as urea or guanidine hydrochloride and a surfactant such as Triton X-100™ may be contained. When the protein (peptide) is secreted in the culture medium, bacteria or cells and the supernatant are separated by a method known per se after completion of cultivation, and the supernatant is harvested.

Purification of the protein (peptide) contained in the thus-obtained culture supernatant or extract can be conducted by appropriately combining separation/purification methods known per se. As these known separation and purification methods, methods utilizing solubility, such as salting-out and solvent precipitation method, methods mainly utilizing differences in molecular weight, such as dialysis method, ultrafiltration method, gel filtration method and SDS-polyacrylamide gel electrophoresis method, methods utilizing differences in electric charge, such as ion exchange chromatography, methods utilizing specific affinity, such as affinity chromatography, methods utilizing differences in hydrophobicity, such as reversed-phase high performance liquid chromatography, methods utilizing differences in isoelectric point, such as isoelectric focusing, and the like can be used.

When the thus-obtained protein (peptide) is the free form, it can be converted to a salt by a method known per se or a method based thereon; when it has been conversely obtained as a salt, it can be converted to the free form or another salt by a method known per se or a method based thereon.

Note that by allowing an appropriate protein modification enzyme to act on the protein (peptide) produced by the transformant, before purification or after purification, it is also possible to optionally add a modification or partially remove a polypeptide. As examples of the protein modification enzyme, trypsin, chymotrypsin, arginyl endopeptidase, protein kinase, glycosidase and the like can be used.

The presence of the thus-formed MIP-3α of the present invention or a partial peptide thereof can be determined by enzyme immunoassay, Western blotting or the like using a specific antibody thereto.

Furthermore, the MIP-3α of the present invention or a partial peptide thereof can also be synthesized by in vitro translation using a cell-free protein translation system that comprises a rabbit reticulocyte lysate, wheat germ lysate, *Escherichia coli* lysate and the like, with an RNA corresponding to the above-described DNA that encodes the MIP-3α of the present invention or a partial peptide thereof as the template. Alternatively, it can be synthesized using a cell-free transcription/translation system containing RNA polymerase, with the DNA that encodes the protein of the present invention or a partial peptide thereof as the template.

As shown in the Examples below, the gene expression of MIP-3α remarkably increases during cerebral ischemia, and considerably decreases in proportion to therapeutic effect of hypothermic treatment. Furthermore, as infarction volume in a cerebral ischemia model remarkably decreases with administration of a neutralizing antibody against MIP-3α, the MIP-3α suppressant of the present invention has a brain/nerve cell-protecting effect, especially in cerebrovascular disorders and head trauma. Therefore, the substance is useful in the prophylaxis/treatment for diseases against which protection of brain cells and nerve cells against cell injury is prophylactically/therapeutically effective. As examples of such diseases, cerebrovascular disorders (e.g., cerebral infarction, cerebral hemorrhage, subarachnoid hemorrhage and the like), head trauma, brain disorders during resuscitation following cardiac arrest resulting from brain/nerve cell disorder, nervous system disorders resulting from brain hypofunction before and after brain surgery, hypoxia, hypoglycemia, brain or spine trauma, drug poisoning, gas poisoning, diabetes mellitus, administration of antitumor agent, alcohol and the like; senile dementias such as Alzheimer's disease; Parkinson's disease; Huntington's disease; prion disease; amyotrophic lateral sclerosis; spinocerebellar degeneration; and AIDS can be mentioned, but these are not to be construed as limiting. In particular, the MIP-3α suppressant of the present invention is useful in the prophylaxis/treatment for cerebrovascular disorders (e.g., cerebral infarction, cerebral hemorrhage, subarachnoid hemorrhage and the like) and head trauma.

Accordingly, MIP-3α suppressants (these substances may have formed a salt; as specific examples of the salt, the same as those mentioned with respect to the aforementioned salt of the MIP-3α of the present invention can be mentioned) may be used as brain/nerve cell protective agents after being mixed with a pharmacologically acceptable carrier into a pharmaceutical composition as required.

As examples of substances that reduce the activity of MIP-3α out of MIP-3α suppressants, a neutralizing antibody against MIP-3α or a partial peptide thereof, an antagonist for MIP-3α or a receptor thereof, a substance that inhibits signal transduction, a substance that inhibits the expression of receptor, a substance that stimulates the inactivation mechanism of MIP-3α, a substance that promotes the decomposition or metabolism of MIP-3α, or a substance that activates them, and the like can be mentioned, but are not to be construed as limiting.

In a preferred embodiment, the substance that reduces the activity of MIP-3α is the above-described antibody against the MIP-3α of the present invention, a partial peptide thereof, or a salt thereof.

The antibody against the MIP-3α of the present invention, a partial peptide thereof, or a salt thereof may be any one of a polyclonal antibody and a monoclonal antibody, as long as it is an antibody capable of recognizing them. In addition, the antibody may be the antibody molecule as is, and may be the F(ab')₂, Fab', or Fab fraction of the antibody molecule, or a single-chain antibody (scFv) joined with the light chain and the heavy chain of the variable region via a linker, and the like.

An antibody against the MIP-3α of the present invention, a partial peptide thereof, or a salt thereof (in the description of antibody below, these are also combinedly simply abbreviated as "the MIP-3α of the present invention") can be produced according to a method of antibody or antiserum production known per se using the MIP-3α of the present invention as the antigen.

### [Preparation of monoclonal antibody]

### (a) Preparation of monoclonal antibody-producing cells

The MIP-3α of the present invention is administered to non-human warm-blooded animals either solely or together with carriers or diluents to the site where the production of antibody is possible by the administration. In order to potentiate the antibody productivity upon the administration, complete Freund's adjuvants or incomplete Freund's adjuvants may be administered. The administration is usually performed once in every 2 to 6 weeks and approximately 2 to 10 times in total. Examples of the applicable non-human warm-blooded animals include monkeys, rabbits, dogs, guinea pigs, mice, rats, sheep, goats and chicken, with mice and rats being preferred.

In the preparation of monoclonal antibody-producing cells, from warm-blooded animals, e.g., mice, immunized with an antigen, one wherein the antibody titer is noted is selected, then the spleen or lymph node is collected after 2 to 5 days from the final immunization and antibody-producing cells contained therein are fused with myeloma cells derived from homologous or heterologous animal to give monoclonal antibody-producing hybridomas. Measurement of the antibody titer in antisera may be made, for example, by reacting labeled protein, which will be described later, with the antiserum followed by assaying the activity of the labeling agent bound to the antibody. The fusion may be operated by the known method, for example, Koehler and Milstein method [Nature, vol. 256, 495 (1975)]. Examples of the fusion accelerator include polyethylene glycol (PEG), Sendai virus, etc., of which PEG is preferably employed.

Examples of the myeloma cells are myeloma cells derived from warm-blooded animals such as NS-1, P3U1, SP2/0. In particular, P3U1 is preferably employed. A preferred ratio of the count of the antibody-producing cells used (spleen cells) to the count of myeloma cells is within a range of approximately 1: 1 to 20: 1. When PEG (preferably, PEG 1000 to PEG 6000) is added in a concentration of approximately 10 to 80% followed by incubating at 20°C to 40°C, preferably at 30°C to 37°C for 1 to 10 minutes, an efficient cell fusion can be performed. Various methods can be used for screening of a monoclonal antibody-producing hybridoma. Examples of such methods include a method which comprises adding the culture supernatant of a hybridoma to a solid phase (e.g., microplate) adsorbed with the antigen protein (peptide) directly or together with a carrier, adding an anti-immunoglobulin antibody (when mouse cells are used for the cell fusion, anti-mouse immunoglobulin antibody is used) labeled with a radioactive substance or an enzyme, or Protein A and detecting the monoclonal antibody bound to the solid phase; a method which comprises adding the culture supernatant of a hybridoma to a solid phase adsorbed with an anti-immunoglobulin antibody or Protein A, adding the protein (peptide) labeled with a radioactive substance or an enzyme and detecting the monoclonal antibody bound to the solid phase; etc.

The monoclonal antibody can be selected by known methods or by analogues of these methods. In general, the selection can be effected in a medium for animal cells supplemented with HAT (hypoxanthine, aminopterin and thymidine). Any selection and growth medium can be employed as far as the hybridoma can grow therein. For example, RPMI 1640 medium containing 1% to 20%, preferably 10% to 20% fetal calf serum, GIT medium (Wako Pure Chemical Industries, Ltd.) containing 1% to 10% fetal calf serum, a serum free medium for culture of a hybridoma (SFM-101, Nissui Seiyaku Co., Ltd.), etc. may be used for the selection and growth medium. The cultivation is performed generally at 20°C to 40°C, preferably at about 37°C, for 5 days to 3 weeks, preferably 1 to 2 weeks. The cultivation may be performed normally in 5% CO₂. The antibody titer of the culture supernatant of hybridomas can be determined as in the assay for the antibody titer in the antisera described above.

### (b) Purification of monoclonal antibody

Separation and purification of the monoclonal antibody can be performed by per se known methods such as methods applied to separation and purification of immunoglobulins [e.g., salting-out, alcohol precipitation, isoelectric point precipitation, electrophoresis, adsorption and desorption with ion exchangers (e.g., DEAE), ultracentrifugation, gel filtration, or a specific purification method which comprises collecting only an antibody with an activated adsorbent such as an antigen-binding solid phase, Protein A, Protein G, etc. and dissociating the binding to give the antibody].

### [Preparation of polyclonal antibody]

The polyclonal antibody against the MIP-3α of the present invention can be manufactured by per se known methods or analogues thereof. For example, an immunogen (a protein or peptide) or a complex of the immunogen and a carrier protein is prepared, and a warm-blooded animal is immunized with the immunogen or the complex in a manner similar to the method described above for the manufacture of monoclonal antibodies. The product containing the antibody to the protein of the present invention is collected from the immunized animal followed by separation and purification of the antibody.

In the complex of an immunogen and a carrier protein used to immunize a warm-blooded animal, the type of carrier protein and the mixing ratio of the carrier to hapten may be of any type in any ratio, as long as the antibody is efficiently produced to the hapten immunized by crosslinking to the carrier. For example, bovine serum albumin, bovine thyroglobulins, hemocyanin, etc. is coupled to hapten with the weight ratio of approximately 0.1 to 20, preferably about 1 to about 5, per one hapten. A variety of condensing agents can be used for the coupling of a carrier to hapten. Glutaraldehyde, carbodiimide, maleimide activated ester, activated ester reagents containing thiol group or dithiopyridyl group, etc. are used for the coupling.

The condensation product is administered to a warm-blooded animal either solely or together with carriers or diluents to the site in which the antibody may be produced by the administration. In order to potentiate the antibody productivity upon the administration, complete Freund's adjuvant or incomplete Freund's adjuvant may be administered. The administration is usually made once in approximately every 2 to 6 weeks and about 3 to about 10 times in total.

The polyclonal antibody can be collected from the blood, ascites, etc., preferably from the blood of a warm-blooded animal immunized by the method described above.

The polyclonal antibody titer in antiserum can be assayed by the same procedure as that for the determination of serum antibody titer described above. The separation and purification of the polyclonal antibody can be performed, following the method for the separation and purification of immunoglobulins performed as applied to the separation and purification of monoclonal antibodies described hereinabove.

A brain/nerve cell protective agent comprising the antibody of the present invention is of low toxicity, and can be administered orally or non-orally (e.g., intraarticular administration) to a human or another mammal (e.g., rat, rabbit, sheep, swine, bovine, cat, dog, monkey and the like), as is in the form of a solution, or after being mixed with a pharmacologically acceptable carrier into an appropriate dose form of pharmaceutical composition.

The pharmacologically acceptable carrier includes various kinds of organic or inorganic carriers which are conventionally used as pharmaceutical materials, such as excipient, lubricant, binder, and disintegrator for solid preparations; or the solvent, solubilizer, suspending agent, isotonizing agent, buffer, and soothing agent for liquid preparations. Further, additives such as antiseptics, antioxidant, colorant, sweetener, etc. can also be incorporated, if necessary.

The excipient preferably includes lactose, sucrose, D-mannitol, D-sorbitol, starch, α-starch, dextrin, crystalline cellulose, low-substituted hydroxypropylcellulose, carboxymethylcellulose sodium, gum arabic, dextrin, pullulan, light silicic anhydride, synthetic aluminum silicate, magnesium metasilicate aluminate, etc.

The lubricant includes magnesium stearate, calcium stearate, talc, colloidal silica, etc.

The binder includes α-starch, cane sugar, gelatin, gum arabic, methylcellulose, carboxymethylcellulose, carboxymethylcellulose sodium, crystalline cellulose, sucrose, D-mannitol, trehalose, dextrin, pullulan, hydroxypropylcellulose, hydroxypropylmethylcellulose, polyvinylpyrrolidone, etc.

The disintegrator includes lactose, sucrose, starch, carboxymethylcellulose, carboxymethylcellulose calcium, croscarmellose sodium, carboxymethylstarch sodium, light silicic anhydride, low- substituted hydroxypropylcellulose, etc.

The solvent includes water for injection, physiological saline, Ringer's solution, alcohol, propylene glycol, polyethylene glycol, sesame oil, corn oil, olive oil, cottonseed oil, etc.

The solubilizer includes polyethylene glycol, propylene glycol, D-mannitol, trehalose, benzyl benzoate, ethanol, trisaminomethane, cholesterol, triethanolamine, sodium carbonate, sodium citrate, sodium salicylate, sodium acetate, etc.

The suspending agent includes surfactants such as stearyltriethanolamine, sodium lauryl sulfate, laurylaminopropionic acid, lecithin, benzalkonium chloride, benzethonium chloride, glyceryl monostearate, etc.; and hydrophilic polymer such as polyvinyl alcohol, polyvinylpyrrolidone, carboxymethylcellulose sodium, methylcellulose, hydroxymethylcellulose, hydroxyethylcellulose, hydroxypropylcellulose, etc.; polysorbates, polyoxyethylene hydrogenated castor oil, etc.

The isotonizing agent includes sodium chloride, glycerin, D-mannitol, D-sorbitol, glucose, etc.

The buffer includes phosphate, acetate, carbonate, citrate, etc.

The soothing agent includes benzyl alcohol, etc.

The antiseptic includes p-oxybenzoic acid esters, chlorobutanol, benzyl alcohol, phenethyl alcohol, dehydroacetic acid, sorbic acid, etc.

The antioxidant includes sulfites, ascorbic acid salts, etc.

The colorant includes water-soluble food tar colors (e.g., Food Color Red No.2 and 3, Food Color Yellow No. 4 and No. 5, and Food Color Blue No. 1 and No. 2; and water-insoluble lake colors (e.g., aluminum salt of the above-mentioned water-soluble food tar colors), natural colors (e.g., β-carotene, chlorophyll, Bengala and the like), etc.

The sweetener includes saccharin sodium, dipotassium glycyrrhizinate, aspartame, stevia, etc.

Formulation of the pharmaceutical composition includes, for example, oral preparations such as tablets, capsules (including soft capsule and microcapsule), granules, powders, syrups, emulsion, suspension, etc., or non-oral preparations such as injections (e.g., subcutaneous injections, intracutanuous injections, intravenous injections, intramuscular injections, peritoneal injections, intrajoint injections, etc.), external preparations (e.g., nasal preparations, transdermal preparations, ointments, etc.) and suppositories (e.g., rectal suppositories, vaginal suppositories, etc.), pellet, drops, sustained-release preparations (e.g., sustained-release microcapsule, etc.), which can be safely administered orally or parenterally.

These pharmaceutical compositions can be produced according to a conventional method in the technical field of the drug formulation, for example, the method described in the Japanese Pharmacopoeia. Specific methods of preparing the preparations will be described below. While the content of the transcription regulating factor of the present invention in the pharmaceutical composition varies depending on formulation, dose of the compound, etc., it is, for example, about 0.1 to 100 % by weight.

The oral preparation can be produced by adding an excipient (e.g., lactose, sucrose, starch, D-mannitol, etc.), a disintegrator (e.g., carboxymethylcellulose calcium, etc.), a binder (e.g., α-starch, gum arabic, carboxymethylcellulose, hydroxypropylcellulose, polyvinylpyrrolidone, etc.), a lubricant (e.g., talc, magnesium stearate, polyethylene glycol 6000, etc.), etc. to active ingredients, followed by compressing it and, coating the formulated product with a coating base for the purpose of taste masking, enteric dissolution or sustained release according to a per se known method, if necessary.

The coating base includes, for example, sugar-coating base, water-soluble film-coating base, enteric film-coating base, sustained-release film-coating base and the like.

The sugar-coating base includes, for example, sucrose, which may be used in combination with one or more of talc, precipitated calcium carbonate, gelatin, gum arabic, pullulan, Carnauba Wax, etc.

The water-soluble film-coating base includes, for example, cellulose polymers such as hydroxypropylcellulose, hydroxypropylmethylcellulose, hydroxyethylcellulose and methylhydroxyethylcellulose; synthetic polymers such as polyvinylacetal diethylaminoacetate, aminoalkylmethacrylate copolymer E [Eudragit E (trademark), Roehm Pharma GmbH], polyvinylpyrrolidone; polysaccharides such as pullulan and the like.

The enteric film-coating base includes, for example, cellulose polymers such as hydroxypropylmethylcellulose phthalate, hydroxypropylmethylcellulose acetatosuccinate, carboxymethylethylcellulose and cellulose acetate phthalate; acrylate polymers such as methacrylate copolymer L [Eudragit L (trademark), Roehm Pharma GmbH], methacrylate copolymer LD [Eudragit L-30D55 (trademark), Roehm Pharma GmbH] and methacrylate copolymer S [Eudragit S (trademark), Roehm Pharma GmbH]; natural substances such as Shellac and the like.

The sustained-release film-coating base includes, for example, cellulose polymers such as ethylcellulose; acrylate polymers such as aminoalkylmethacrylate copolymer RS [Eudragit RS (trademark), Roehm Pharma GmbH], ethyl acrylate/methyl methacrylate copolymer suspension [Eudragit NE (trademark), Roehm Pharma GmbH], etc.

The above-mentioned coating bases may be used in a suitable mixture of two or more. In addition, a light-blocking agent such as titanium oxide and iron sesquioxide may be used in coating.

The injections can be produced by dissolving, suspending or emulsifying active ingredients in aqueous solvent (e.g., distilled water, physiological saline, Ringer's solution, etc.) or oily solvent (e.g., vegetable oils such as olive oil, sesame oil, cottonseed oil and corn oil, propylene glycol, etc.) with a dispersing agent (e.g., polysorbate 80, polyoxyethylene hydrogenated castor oil 60, polyethylene glycol, carboxymethylcellulose, and sodium alginate, etc.), a preservative (e.g., methylparaben, propylparaben and benzyl alcohol, chlorobutanol, phenol, etc.), an isotonizing agent (e.g., sodium chloride, glycerin, D-mannitol, D-sorbitol, glucose, etc), etc. If desired, additives such as a solubilizer (e.g., sodium salicylate, sodium acetate, etc.), a stabilizer (e.g., human serum albumin, etc.), soothing agents (e.g., benzyl alcohol, etc.) may be used. The injection is usually filled in suitable ampoules.

The dose of a brain/nerve cell protective agent comprising the antibody of the present invention varies depending on subject of administration, target disease, symptoms, route of administration and the like; when using for the treatment/prophylaxis of an adult patient with a cerebrovascular disorder, for example, the brain/nerve cell protective agent is advantageously administered in the form of a powder inhalant at normally about 0.01 to 20 mg/kg body weight, preferably about 0.1 to 10 mg/kg body weight, and more preferably about 0.1 to 5 mg/kg body weight, based on the amount of the antibody of the present invention per dose, about 1 to 5 times a day, preferably about 1 to 3 times a day. In the case of other forms of non-oral administration and oral administration, a dose based on the above-described doses can be administered. If the symptom is especially severe, the dose may be increased according to the symptom.

As the substance that inhibits the expression of MIP-3α, a nucleic acid comprising a base sequence complementary to a base sequence that encodes the MIP-3α of the present invention or a portion thereof can be preferably mentioned. The nucleic acid comprising a base sequence complementary to a base sequence that encodes the MIP-3α of the present invention or a portion thereof (hereinafter also abbreviated as "the antisense nucleic acid of the present invention") may be any one having a base sequence completely or substantially complementary to the base sequence of the MIP-3α of the present invention, or a portion of the complementary base sequence, and acting to suppress the translation of the protein from the RNA that encodes the MIP-3α of the present invention. As "the substantially complementary base sequence", a base sequence capable of hybridizing to the base sequence that encodes the MIP-3α of the present invention under physiological conditions for cells that express the protein, more specifically a base sequence showing a homology of about 70% or more, preferably about 80% or more, more preferably about 90% or more, and most preferably about 95% or more, to a complementary chain of the base sequence that encodes the MIP-3α of the present invention or a partial base sequence thereof, and the like can be mentioned.

The antisense nucleic acid of the present invention can be designed and synthesized on the basis of information on the cloned or determined base sequence of a nucleic acid that encodes the MIP-3α of the present invention. Such a nucleic acid is capable of inhibiting the replication or expression of the gene that encodes the MIP-3α of the present invention. Hence, the antisense nucleic acid of the present invention is capable of hybridizing to the RNA transcribed from the gene that encodes the MIP-3α of the present invention, thus inhibiting the synthesis (processing) or function (translation into protein) of mRNA.

The target region of the antisense nucleic acid of the present invention is not particularly limited as to the length thereof, as long as hybridization of the antisense nucleic acid results in the inhibition of the translation of the MIP-3α of the present invention, and can be the entire sequence or a partial sequence of the RNA that encodes the MIP-3α of the present invention; a partial sequence of about 15 bases for the shortest, and the entire sequence of the mRNA or initial transcription product for the longest, can be mentioned. Considering the ease of synthesis and the issue of antigenicity, an oligonucleotide consisting of about 15 to about 30 bases is preferred, which, however, is not to be construed as limiting. Specifically, for example, the 5'-end hairpin loop, the 5'-end 6-base-pair repeat, the 5'-end untranslated region, the polypeptide translation initiation codon, the protein-coding region, the ORF translation initiation codon, the 3'-end untranslated region, the 3'-end palindrome region, and the 3'-end hairpin loop of the gene that encodes the MIP-3α of the present invention can be selected as the target region, but any region within the gene can be selected as the target. For example, it is also preferable that the intron portion of the gene be the target region.

Furthermore, the antisense nucleic acid of the present invention may be one not only capable of hybridizing to the mRNA or initial transcription product that encodes the MIP-3α of the present invention to inhibit the translation to the protein, but also capable of binding to the gene that encodes the MIP-3α of the present invention, which is a double-stranded DNA, to form a triple strand (triplex) and inhibiting the transcription of RNA.

As the antisense nucleic acid, a deoxyribonucleotide containing 2-deoxy-D-ribose, a ribonucleotide containing D-ribose, another type of nucleotide that is an N-glycoside of the purine or pyrimidine base, or another polymer having a non-nucleotide backbone (for example, commercially available protein nucleic acids and synthetic sequence specific-nucleic acid polymers) or another polymer having a special bond (however, this polymer comprises a nucleotide having a sequence that allows base pairing or base attachment as found in DNA or RNA) and the like can be mentioned. These may be double-stranded DNAs , single-stranded DNAs, double-stranded RNAs or single-stranded RNAs, or DNA:RNA hybrids, and may also be non-modified polynucleotides (or non-modified oligonucleotides), those having a known modification added thereto, for example, those with a labels known in the art, those with a cap, those methylated, those having 1 or more naturally occurring nucleotides substituted with analogues, those modified with an intramolecular nucleotide, for example, those having a non-charge bond (for example, methylphosphonate, phospho triester, phosphoramidate, carbamate and the like), those having a charged bond or a sulfur-containing bond (for example, phosphorothioate, phosphorodithioate and the like), for example, those having a side chain group of a protein (nuclease, nuclease inhibitor, toxin, antibody, signal peptide, poly-L-lysine and the like), a sugar (for example, monosaccharide and the like) and the like, those having a intercalator (for example, acridine, psoralen and the like), those containing a chelate compound (for example, metals, radioactive metals, boron, oxidizing metals and the like), or those containing an alkylating agent, those having a modified bond (for example, α anomer type nucleic acid and the like) . Here, "nucleoside", "nucleotide" and "nucleic acid" may include not only those containing the purine and pyrimidine bases, but also those containing another modified heterocyclic base. These modified products may contain a methylated purine or pyrimidine, an acylated purine or pyrimidine, or another heterocycle. The modified nucleotide and the modified nucleotide may also have their sugar portion modified by, for example, substitution of 1 or more hydroxyl groups by a halogen, an aliphatic group and the like, or conversion to a functional group such as an ether or an amine.

The antisense nucleic acid is RNA, DNA or a modified nucleic acid (RNA, DNA). As specific examples of the modified nucleic acid, sulfur derivatives and thiophosphate derivatives of nucleic acids, and those resistant to decomposition by polynucleosideamide or oligonucleosideamide can be mentioned, which, however, are not to be construed as limiting. The antisense nucleic acid of the present invention can preferably be designed to accomplish one of the following purposes: to make the antisense nucleic acid more stable in the cell, to increase the cell permeability of the antisense nucleic acid, to increase the affinity for the desired sense chain, and to reduce the toxicity, if any, of the antisense nucleic acid. Many such modifications are known in the art, and disclosed in, for example J. Kawakami et al., Pharm Tech Japan, Vol. 8, p. 247, 1992; Vol. 8, p. 395, 1992; S. T. Crooke et al. ed., Antisense Research and Applications, CRC Press, 1993 and the like.

The antisense nucleic acid may be altered, and may contain an modified sugar, base or bond, and can be supplied in a special form like liposome or microspheres, can be applied for gene therapy, and can be given in an adduct form. As such an adduct form used, a polycation like polylysine, which acts to neutralize the charge of the phosphate backbone, and a hydrophobic compound like a lipid that enhances the interaction with cell membrane or increases nucleic acid uptake (for example, phospholipid, cholesterol and the like) can be mentioned. As lipids preferred for addition, cholesterol and derivatives thereof (for example, cholesterylchloroformate, cholic acid and the like) can be mentioned. These can be attached to the 3' end or the 5' end of nucleic acid, and can be attached via a base, a sugar or an intramolecular nucleoside bond. As other groups, a capping group specifically arranged at the 3' end or 5' end of nucleic acid to prevent degradation by a nuclease such as exonuclease or RNase can be mentioned. As such a capping group, hydroxyl protecting groups known in the art, including glycols such as polyethylene glycol and tetraethylene glycol, can be mentioned, which, however, are not to be construed as limiting.

A ribozyme capable of specifically cleaving the mRNA or initial gene transcription product that encodes the MIP-3α of the present invention within the coding region (including the intron portion in the case of the initial transcription product) can also be encompassed in the antisense nucleic acid of the present invention. "Ribozyme" refers to an RNA possessing an enzyme activity to cleave a nucleic acid, and is herein understood to be used as a concept encompassing DNA, as long as it possesses sequence-specific nucleic acid cleavage activity, since it has recently been found that an oligo DNA having the base sequence of the enzyme activity portion also possesses nucleic acid cleavage activity. One of the most versatile ribozymes is a self-splicing RNA found in infectious RNAs such as viroid and virusoid, and the hammerhead type, the hairpin type and the like are known. The hammerhead type exhibits enzyme activity with about 40 bases in length, and it is possible to specifically cleave the target mRNA by making several bases at both ends adjoining to the hammerhead structure portion (about 10 bases in total) to be a sequence complementary to the desired cleavage site of the mRNA. Because this type of ribozymes has RNA only as the substrate, it offers an additional advantage that there is no attack on genomic DNA. Provided that the mRNA encoding the MIP-3α of the present invention takes a double-stranded structure by itself, the target sequence can be made single-stranded, using a hybrid ribozyme prepared by joining an RNA motif derived from a viral nucleic acid that can specifically bind to RNA helicase [Proc. Natl. Acad. Sci. USA, 98(10): 5572-5577 (2001)]. Furthermore, when the ribozyme is used in the form of an expression vector containing a DNA that encodes it, the ribozyme may be a hybrid ribozyme prepared by further joining a sequence modified from the tRNA to promote the migration of the transcription product to cytoplasm [Nucleic Acids Res., 29(13): 2780-2788 (2001)].

A double-stranded oligo RNA complementary to a partial sequence (including the intron portion in the case of the initial transcription product) within the coding region of the mRNA or initial gene transcription product that encodes the MIP-3α of the present invention (small interfering RNA; siRNA) can also be encompassed in the scope of the antisense nucleic acid of the present invention. What is called the RNA interference (RNAi) phenomenon, in which introducing a short double-stranded RNA in a cell results in the decomposition of an mRNA complementary to the RNA, has long been known in nematodes, insects, plants and the like; since this phenomenon has recently been confirmed as also occurring in mammalian cells [Nature, 411(6836): 494-498 (2001)], it is attracting attention as a substitute for ribozyme.

The antisense oligonucleotide and ribozyme of the present invention can be prepared by determining the target region of the mRNA or initial transcription product on the basis of information on the cDNA sequence or genomic DNA sequence that encodes the MIP-3α of the present invention, and synthesizing a sequence complementary thereto using a commercially available DNA/RNA synthesizer (Applied Biosystems, Beckman Instruments, and the like). siRNA can be prepared by synthesizing each of a sense strand and an antisense strand using a DNA/RNA synthesizer, denaturing the strands in an appropriate annealing buffer at, for example, about 90 to about 95°C for about 1 minute, and then annealing the strands at about 30 to about 70°C for about 1 to about 8 hours. It is also possible to prepare a longer double-stranded polynucleotide by synthesizing complementary oligonucleotide strands in alternative overlaps, annealing the strands, and then ligating the strands using ligase.

A brain/nerve cell protective agent comprising the antisense nucleic acid of the present invention can be formulated into a preparation by a technique known per se in the same manner as the above-described case of the antibody of the present invention.

In addition, for example, the antisense nucleic acid of the present invention can be administered orally or parenterally to a human or another mammal (e.g., rat, rabbit, sheep, swine, bovine, cat, dog, monkey and the like) according to a conventional method, alone or after insertion into an appropriate vector such as a retrovirus vector, adenovirus vector, or adenovirus associated virus vector. The antisense nucleic acid can be administered using a gene gun or a catheter such as a hydrogel catheter, as is or after being formulated into a preparation along with a physiologically acceptable carrier such as an auxiliary agent for promoting the ingestion thereof. Alternatively, it can also be administered locally into the trachea as an inhalant after conversion to an aerosol.

Furthermore, the antisense nucleic acid of the present invention may be administered intravenously, subcutaneously or intraarticularly and the like, alone or after being formulated into a preparation (injection) along with a carrier such as liposome, for the purpose of improving its movement in the body, extending its half-life, and improving its intracellular uptake efficiency.

The dose of the antisense nucleic acid varies depending on target disease, subject of administration, route of administration and the like; for the treatment of a cerebrovascular disorder in the acute stage, for example, the antisense nucleic acid of the present invention is normally administered at about 0.1 to 100 mg per day, in an adult patient (60 kg body weight).

Furthermore, the antisense nucleic acid of the present invention can also be used as a diagnostic nucleic acid probe for examining the presence of a nucleic acid that encodes the MIP-3α of the present invention in tissue and cells and the expression thereof.

The present invention also provides a brain/nerve cell protective agent comprising a substance that inhibits the binding of MIP-3α and a receptor thereof, or a substance that inhibits the signal transduction activity of an MIP-3α receptor. As examples of such substances, an antagonist of MIP-3α or a receptor thereof and the like can be mentioned.

The MIP-3α receptor is not particularly limited, as long as it binds specifically to MIP-3α to exhibit signal transduction action; for example, CCR6, a known MIP-3α receptor, can be mentioned. "The CCR6 of the present invention" is a protein comprising the same or substantially the same amino acid sequence as the amino acid sequence represented by SEQ ID NO:8 or 10 or a salt thereof. Here, the "protein comprising substantially the same amino acid sequence" has the same definition as that described in detail above with respect to the MIP-3α of the present invention.

A substance that inhibits the binding of MIP-3α and a receptor thereof can be obtained by, for example, the screening method described below, which uses the MIP-3α of the present invention, a partial peptide thereof, or a salt thereof, and/or an MIP-3α receptor (for example, the CCR6 of the present invention), a partial peptide thereof, or a salt thereof.

### [Screening for substances exhibiting brain/nerve cell protective action]

The present invention provides a method for screening of a compound that inhibits an activity of the MIP-3α of the present invention (for example, signal transduction activity, receptor binding activity and the like) or a salt thereof, which comprises using the MIP-3α of the present invention, a partial peptide thereof, or a salt thereof (in the description of this screening below, these are also combinedly simply abbreviated as "the MIP-3α of the present invention") and/or an MIP-3α receptor (for example, the CCR6 of the present invention), a partial peptide thereof, or a salt thereof (in the description of this screening below, these are also combinedly simply abbreviated as "the CCR6 of the present invention").

More specifically, for example, a method for screening of a compound that inhibits an activity of the MIP-3α of the present invention or a salt thereof, is used, which comprises (i) measuring and comparing the binding activity between the MIP-3α of the present invention and the CCR6 of the present invention in the presence and absence of a test compound, and (ii) measuring and comparing the signal transduction activity in a cell capable of producing the CCR6 of the present invention in the presence of the MIP-3α of the present invention and in the presence of the MIP-3α of the present invention and the test compound.

In the above-described screening method, in both cases (i) and (ii), for example, signal transduction activity and receptor binding activity are measured by a method known per se. Specifically, in the presence and absence of a test compound, CCR6-expressing cells [transformant cells obtained by introducing a DNA that encodes the CCR6 of the present invention, for example, a DNA that comprises the base sequence represented by SEQ ID NO:7 or 9, a DNA comprising the base sequence represented by base numbers 343 to 1440 in the base sequence represented by SEQ ID NO:13, or a DNA capable of hybridizing to a DNA comprising the base sequence represented by SEQ ID NO:7 or 9 or to a DNA comprising the base sequence represented by base numbers 343 to 1440 in the base sequence represented by SEQ ID NO:13, under highly stringent conditions, and that encodes a protein having substantially the same activity as the protein comprising the amino acid sequence represented by SEQ ID NO:8, 10 or 14 (here, "highly stringent conditions", "substantially the same activity" and the like have the same definitions as those given above), into one of the expression vectors mentioned as examples in the description of the MIP-3α of the present invention, and introducing the expression vector into one of the host cells mentioned as examples in the description of the MIP-3α of the present invention (e.g., CHO-K1 cells and the like) using the same method of transformation], for example, are cultured in the presence of the MIP-3α of the present invention, and a signal transduction activity via the CCR6 expressed on the cell membrane (for example, activity to promote or suppress increase or decrease in concentration of intracellular cAMP, liberation of intracellular Ca²⁺, production of inositol phosphate, changes of cell membrane potential, phosphorylation or dephosphorylation of intracellular protein, activation of c-fos, reduction of pH and the like) is measured by a known method.

Alternatively, in the presence and absence of a test compound, (1) the MIP-3α of the present invention, previously labeled, is brought into contact with the CCR6 of the present invention, and the amount of labeled MIP-3α bound to the receptor is measured and compared, (2) the MIP-3α of the present invention, previously labeled, is brought into contact with cells containing the CCR6 of the present invention (for example, immune cells such as lymphocytes, dendritic cells, astrocytes and the like known to express CCR6) or a membrane fraction of the cells, and the amount of labeled MIP-3α bound to the cells or membrane fraction is measured and compared, or (3) the MIP-3α of the present invention, previously labeled, is brought into contact with the protein CCR6 expressed on the cell membrane by culturing a transformant containing a DNA that encodes CCR6 (for example, the above-described transformant cell and the like), and the amount of labeled MIP-3α bound to the cells or membrane fraction is measured and compared.

The MIP-3α of the present invention may be added as an isolated protein from outside to a system containing the CCR6 of the present invention or cells capable of producing the same, or a host (transformant) transformed with the aforementioned vector containing a DNA that encodes the MIP-3α of the present invention may be used. As examples of the host, animal cells such as mouse ATDC5 cells and CHO cells are preferably used. For the screening, a transformant that extracellularly secretes the MIP-3α of the present invention when cultured by the aforementioned method, for example, is preferably used. The method for culturing a cell capable of expressing the MIP-3α of the present invention is the same as the aforementioned method for culturing the transformant of the present invention.

As examples of the test compound, a peptide, a protein, a non-peptide compound, a synthetic compound, a fermentation product, a cell extract, a plant extract, an animal tissue extract, and the like can be mentioned.

For example, if the signal transduction activity or receptor binding activity is decreased by about 20% or more, preferably about 30% or more, more preferably about 50% or more, in the presence of a test compound, compared to the absence of the test compound, the test compound can be selected as a compound that inhibits the activity of the MIP-3α of the present invention.

MIP-3α receptor antagonists can be screened for in the same manner as above, using a receptor, preferably the CCR6 of the present invention, and another ligand of the receptor, for example, a compound (e.g., synthetic low molecular compound) selected as a CCR6 agonist in the above-described screening method.

In addition, inverse agonists of the MIP-3α receptor are also preferred as substances exhibiting the brain/nerve cell protective action of the present invention, and such substances can be screened for using any one of the above-described screening methods, and also by applying the CCR6 of the present invention alone to method (i) or (ii) above.

The kit used for the above-described screening method of the present invention comprises the MIP-3α of the present invention and/or the CCR6 of the present invention. When screening is conducted using signal transduction activity as the index, the CCR6 of the present invention is supplied as cells capable of producing the same. The MIP-3α of the present invention may be supplied as an isolated protein, or supplied as cells capable of producing the same. In addition, when using receptor binding activity as the index, the MIP-3α of the present invention is preferably labeled.

The compound obtained using the screening method of the present invention or screening kit or a salt thereof is a compound that inhibits the receptor binding activity and/or signal transduction activity of the MIP-3α of the present invention, or a salt thereof selected from among the above-described test compounds, for example, a peptide, a protein, a non-peptide compound, a synthetic compound, a fermentation product, a cell extract, a plant extract, an animal tissue extract, plasma and the like. As the salt of the compound, the same as the aforementioned salt of the MIP-3α of the present invention is used.

A brain/nerve cell protective agent comprising a compound that inhibits the receptor binding activity and/or signal transduction activity of the MIP-3α of the present invention or a salt thereof can be formulated into a preparation in the same manner as in the above-described case of the antibody of the present invention.

The dose of the brain/nerve cell protective agent varies depending on subject of administration, target disease, symptoms, route of administration and the like; for the treatment/prophylaxis of an adult patient with a cerebrovascular disorder, for example, the brain/nerve cell protective agent is advantageously administered in the form of a powder inhalant at normally about 0.01 to 20 mg/kg body weight, preferably about 0.1 to 10 mg/kg body weight, and more preferably about 0.1 to 5 mg/kg body weight, based on the amount of compound that inhibits the receptor binding activity and/or signal transduction activity of the MIP-3α of the present invention or a salt thereof per dose, about 1 to 5 times a day, preferably about 1 to 3 times a day. In the case of other forms of parenteral administration and oral administration, a dose based on these doses can be administered. If the symptom is especially severe, the dose may be increased according to the symptom.

Because the gene that encodes the MIP-3α of the present invention can have the expression thereof increased in cerebral ischemia, a substance that inhibits the expression of the gene that encodes the MIP-3α of the present invention can also be used as a substance exhibiting brain/nerve cell protective action.

Therefore, a nucleic acid comprising the base sequence that encodes the MIP-3α of the present invention or a portion thereof is useful as a reagent for screening for a substance that inhibits the expression of the gene that encodes the MIP-3α of the present invention.

Accordingly, the present invention also provides a method for screening of a substance exhibiting brain/nerve cell protective action, which comprises using a nucleic acid comprising the base sequence that encodes the MIP-3α of the present invention or a portion thereof.

The screening method comprises culturing cells capable of producing the MIP-3 of the present invention in the presence and absence of a test compound, and measuring and comparing the MIP-3α mRNA contents under the both conditions.

As the test compound and the cells capable of producing the MIP-3α of the present invention, the same as those mentioned above can be mentioned.

A measurement of mRNA content can be conducted by a known method, for example, Northern hybridization using as the probe a nucleic acid comprising the base sequence that encodes the MIP-3α of the present invention or a portion thereof, or a PCR method using as the primer a nucleic acid comprising the base sequence that encodes the MIP-3α of the present invention or a portion thereof, or a method based on these methods.

For example, a test compound that inhibits the gene expression amount in the absence of the test compound by about 20% or more, preferably about 30% or more, and more preferably about 50% or more, can be selected as a compound that inhibits the expression of the gene that encodes the MIP-3α of the present invention, hence as a compound that exhibits brain/nerve cell protective action.

The expression amount of the gene that encodes the MIP-3α of the present invention can also be measured using an antibody against the MIP-3α of the present invention.

Specifically, after the aforementioned cells capable of producing the MIP-3α of the present invention are cultured in the presence and absence of a test compound, the MIP-3α protein content in the resulting cell supernatant or cell extract can be measured using an antibody against the MIP-3α of the present invention by a known method, for example, Western analysis, ELISA method and the like or a method based on these methods.

For example, a test compound that inhibits the protein production in the absence of the test compound by about 20% or more, preferably about 30% or more, and more preferably about 50% or more, can be selected as a compound that inhibits the expression of the gene that encodes the MIP-3α of the present invention, hence as a compound that exhibits brain/nerve cell protective action.

Because MIP-3α exhibits its physiological activities by binding to a receptor thereof (for example, CCR6) to transmit signal information to cells expressing the receptor, its activities decrease when the expression of the receptor thereto is inhibited. Therefore, a substance that inhibits the expression of the receptor is also encompassed in the scope of the MIP-3α suppressant in the present invention.

Accordingly, the present invention also provides a method for screening of a substance exhibiting brain/nerve cell protective action, which comprises using a nucleic acid comprising the base sequence that encodes the CCR6 of the present invention or a portion thereof, or an antibody against the CCR6 of the present invention, a partial peptide thereof, or a salt thereof.

As the screening method, a method comprising culturing cells capable of producing the CCR6 of the present invention in the presence and absence of a test compound, and measuring and comparing the CCR6 mRNA contents or protein contents under the two conditions.

A measurement of mRNA content can be conducted by a known method, for example, Northern hybridization using as the probe a nucleic acid comprising the base sequence that encodes the CCR6 of the present invention or a portion thereof, or a PCR method using as the primer a nucleic acid comprising the base sequence that encodes the CCR6 of the present invention or a portion thereof, or a method based on these methods.

A measurement of protein content can be conducted by culturing cells capable of producing the CCR6 of the present invention in the presence and absence of a test compound, then isolating a cell membrane fraction or cell extract, and using an antibody against the CCR6 of the present invention, by a known method, for example, Western analysis, ELISA method and the like or a method based on these methods. An antibody against CCR6 can be prepared in the same manner as with the above-described antibody against MIP-3α.

The kit used for the above-described screening method of the present invention comprises a nucleic acid comprising the base sequence that encodes the MIP-3α of the present invention or a portion thereof or an antibody against the MIP-3α of the present invention, cells capable of producing the MIP-3α of the present invention and the like, or comprises a nucleic acid comprising the base sequence that encodes the CCR6 of the present invention or a portion thereof or an antibody against the CCR6 of the present invention, cells capable of producing the CCR6 of the present invention and the like.

A compound obtained using the screening method of the present invention or screening kit, or a salt thereof, is a compound selected from among the above-described test compounds, for example, a peptide, a protein, a non-peptide compound, a synthetic compound, a fermentation product, a cell extract, a plant extract, an animal tissue extract, plasma and the like or a salt thereof, and a compound that inhibits the expression of the MIP-3α of the present invention or the CCR6 of the present invention or a salt thereof. As the salt of the compound, the same as the aforementioned salt of the MIP-3α of the present invention is used.

A brain/nerve cell protective agent comprising the above-described compound that inhibits the expression of the MIP-3α of the present invention or the CCR6 of the present invention or a salt thereof can be formulated into a preparation in the same manner as in the above-described case of the antibody of the present invention.

The dose of the brain/nerve cell protective agent varies depending on subject of administration, target disease, symptoms, route of administration and the like; for the treatment/prophylaxis of an adult patient with a cerebrovascular disorder, for example, the brain/nerve cell protective agent is advantageously administered in the form of a powder inhalant at normally about 0.01 to 20 mg/kg body weight, preferably about 0.1 to 10 mg/kg body weight, and more preferably about 0.1 to 5 mg/kg body weight, based on the amount of compound that inhibits the expression of the MIP-3α of the present invention or the CCR6 of the present invention or a salt thereof per dose, about 1 to 5 times a day, preferably about 1 to 3 times a day. In the case of other forms of parenteral administration and oral administration, a dose based on these doses can be administered. If the symptom is especially severe, the dose may be increased according to the symptom.

### [Quantitation of the MIP-3α of the present invention, a partial peptide thereof, or a salt thereof]

Because an antibody against the MIP-3α of the present invention is capable of specifically recognizing the MIP-3α of the present invention, it can be used for the quantitation of the MIP-3α of the present invention in a test liquid, especially for the quantitation by the sandwich immunoassay, and the like.

Accordingly, the present invention provides:
(i) a method of quantifying the MIP-3α of the present invention in a test liquid, which comprises competitively reacting an antibody against the MIP-3α of the present invention, the test liquid and the MIP-3α of the present invention, previously labeled, and measuring the ratio of labeled MIP-3α of the present invention bound to the antibody, and
(ii) a method of quantifying the MIP-3α of the present invention in a test liquid, which comprises simultaneously or serially reacting the test liquid, an antibody against the MIP-3α of the present invention, previously insolubilized on a carrier, and another antibody against the MIP-3α of the present invention, previously labeled, and then measuring the activity of the labeling agent on the insolubilizing carrier.

In quantitation method (ii), if one antibody is an antibody that recognizes the N-terminus portion of the MIP-3α of the present invention, it is desirable that the other antibody be an antibody that recognizes another portion of the MIP-3α of the present invention.

In addition, using a monoclonal antibody against the MIP-3α of the present invention, it is possible to quantify the MIP-3α of the present invention, and also to detect the MIP-3α by tissue staining and the like. For these purposes, the antibody molecule itself may be used, or F(ab')2, Fab' or Fab fractions of the antibody molecule may be used as well.

The methods of the present invention for quantifying the MIP-3α of the present invention using the antibody to MIP-3α of the present invention are not to be limited particularly. Any method can be used, so long as the amount of antibody, antigen, or antibody-antigen complex corresponding to the amount of antigen (e.g., the amount of the protein) in a test liquid can be detected by chemical or physical means and can be calculated from a standard curve prepared from standard solutions containing known amounts of the antigen. For example, nephrometry, competitive method, immunometric method, and sandwich method are advantageously used, among which the sandwich method described below is particularly preferable in terms of sensitivity and specificity.

As labeling agents used for the assay methods using labeled substances, there are employed, for example, radioisotopes, enzymes, fluorescent substances, luminescent substances, etc. As the radioisotopes, there are employed, for example, [¹²⁵I] , [¹³¹I] , [³H] , [¹⁴C] , etc. As the enzymes described above, stable enzymes with a high specific activity are preferred; for example, β-galactosidase, β-glucosidase, alkaline phosphatase, peroxidase, malate dehydrogenase, etc. are used. Examples of the fluorescent substance used are fluorescamine, fluorescein isothiocyanate, etc. As the luminescent substances, there are employed, for example, luminol, luminol derivatives, luciferin, lucigenin, etc. Furthermore, the biotin-avidin system may also be used for binding of an antibody or antigen to the labeling agent.

For insolubilization of the antigen or antibody, physical adsorption may be used. Chemical binding methods conventionally used for insolubilization or immobilization of proteins, enzymes, etc. may be used as well. For the carriers, examples include insoluble polysaccharides such as agarose, dextran, cellulose, etc.; synthetic resin such as polystyrene, polyacrylamide, silicone, etc., or glass, etc.

In the sandwich method, the insolubilized monoclonal antibody to MIP-3α of the present invention is reacted with a test liquid (primary reaction), then with a labeled form of another monoclonal antibody to MIP-3α of the present invention (secondary reaction), and the activity of the labeling agent on the insolubilized carrier is assayed, whereby the amount of the MIP-3α of the present invention in the test liquid can be quantified. The order of the primary and secondary reactions may be reversed, and the reactions may be performed simultaneously or with some time intervals. The labeling agent and the methods for insolubilization can be performed by modifications of those methods described above. In the immunoassay by the sandwich method, the antibody used for immobilized antibody or labeled antibody is not necessarily from one species, but a mixture of two or more species of antibodies may be used to increase the measurement sensitivity.

In the methods of assaying the MIP-3α of the present invention by the sandwich method, antibodies that bind to different sites of the protein of the present invention are preferably used as the monoclonal antibodies to MIP-3α of the present invention for the primary and secondary reactions. That is, in the antibodies used for the primary and secondary reactions, for example, when the antibody used in the secondary reaction recognizes the C-terminus region of the MIP-3α of the invention, it is preferable to use the antibody capable of recognizing the region other than the C-terminus region (e.g., the antibody capable of recognizing the N-terminus region) for the primary reaction.

A monoclonal antibody against the MIP-3α of the present invention can be used for the assay systems other than the sandwich method, for example, the competitive method, immunometric method, nephrometry, etc.

In the competitive method, an antigen in a test liquid and a labeled antigen are competitively reacted with an antibody, and the unreacted labeled antigen (F) and the labeled antigen bound to the antibody (B) are separated (B/F separation). The amount of the labeled antigen in B or F is measured, and the amount of the antigen in the test liquid is quantified. This reaction method includes a liquid phase method using a soluble antibody as an antibody, polyethylene glycol and a secondary antibody to the soluble antibody for B/F separation, etc. and an immobilized method either using an immobilized antibody as the primary antibody, or using a soluble antibody as the primary antibody and an immobilized antibody as the secondary antibody.

In the immunometric method, an antigen in a test liquid and an immobilized antigen are competitively reacted with a definite amount of labeled antibody, the solid phase is separated from the liquid phase, or an antigen in a test liquid is reacted with an excess amount of labeled antibody, the immobilized antigen is then added to bind the unreacted labeled antibody to the solid phase, and the solid phase is separated from the liquid phase. Then, the amount of the labeled antibody in either phase is measured to quantify an amount of the antigen in the test liquid.

In the nephrometry, an amount of insoluble precipitates produced after the antigen-antibody reaction in gel or solution are measured. Even when the amount of an antigen in a test liquid is small and only a small amount of precipitates is obtained, laser nephrometry utilizing scattering of laser can be advantageously employed.

For applying these individual immunological assay methods to the quantification methods of the present invention, any particular conditions, and setting of procedures and the like are not required. The assay systems for the protein of the present invention may be constructed by adding ordinary technical consideration in the art to conventional conditions and procedures in the respective methods. For the details of these general technical means, reference can be made to the following reviews and texts. For example, references can be made to Hiroshi Irie, ed., "Radioimmunoassay" (Kodansha Ltd., published in 1974), Hiroshi Irie, ed., "Sequel to the Radioimmunoassay" (Kodansha Ltd., published in 1979), Eiji Ishikawa, et al., ed., "Enzyme immunoassay" (Igakushoin, published in 1978), Eiji Ishikawa, et al., ed., "Enzyme immunoassay" (2nd ed.) (Igakushoin, published in 1982), Eiji Ishikawa, et al., ed., "Enzyme immunoassay" (3rd ed.) (Igakushoin, published in 1987), Methods in ENZYMOLOGY, Vol. 70 (Immunochemical Techniques (Part A)), ibid., Vol. 73 (Immunochemical Techniques (Part B)), ibid., Vol. 74 (Immunochemical Techniques (Part C)), ibid., Vol. 84 (Immunochemical Techniques (Part D: Selected Immunoassays)), ibid., Vol. 92 (Immunochemical Techniques (Part E: Monoclonal Antibodies and General Immunoassay Methods)), ibid., Vol. 121 (Immunochemical Techniques (Part I: Hybridoma Technology and Monoclonal Antibodies))(all published by Academic Press Publishing), and the like.

The MIP-3α of the present invention can be quantified with high sensitivity using an antibody against the MIP-3α of the present invention as described above.

Furthermore, if an increase or decrease in the concentration of the MIP-3α of the present invention is detected by quantifying the concentration of MIP-3α of the present invention using an antibody against the MIP-3α of the present invention, the subject can be diagnosed as having a brain/nerve cell injury, for example, a brain/nerve cell injury in a cerebrovascular disorder such as cerebral infarction, cerebral hemorrhage, or subarachnoid hemorrhage, or in head trauma, or as being highly likely to have a cell injury in the future.

In addition, an antibody against the MIP-3α of the present invention can be used to detect the MIP-3α of the present invention present in a test sample such as of body fluid or tissue. The same can also be used for the preparation of an antibody column for purifying the MIP-3α of the present invention, detection of the MIP-3α of the present invention in each fraction during purification, for analysis of the behavior of the MIP-3α of the present invention in the test cell, and the like.

### [Genetic diagnostic agent]

Because the DNA that encodes the MIP-3α of the present invention (hereinafter also abbreviated as "the DNA of the present invention" in this section (genetic diagnostic agent) and the following section for DNA transgenic animals and knock-out animals) is capable of detecting an abnormality of the DNA or mRNA (gene abnormality) that encodes the MIP-3α of the present invention or a partial peptide thereof in a human or another warm-blooded animal (for example, rat, mouse, guinea pig, rabbit, bird, sheep, swine, bovine, horse, cat, dog, monkey, chimpanzee and the like) when used as, for example, a probe, it is useful as, for example, a genetic diagnostic agent for injuries, mutations or decreased expression of the DNA or mRNA, increased expression or overexpression of the DNA or mRNA, and the like.

The above-described genetic diagnosis using the DNA of the present invention can be performed by, for example, a method known per se, such as Northern hybridization, the PCR-SSCP method (Genomics, Vol. 5, pp. 874-879 (1989), Proceedings of the National Academy of Sciences of the United States of America, Vol. 86, pp. 2766-2770 (1989)) and the like.

For example, if overexpression is detected by Northern hybridization, or if a DNA mutation is detected by the PCR-SSCP method, the subject can be diagnosed as having a brain/nerve cell injury, for example, a brain/nerve cell injury due to a cerebrovascular disorder such as cerebral infarction, cerebral hemorrhage, or subarachnoid hemorrhage, or head trauma, or as being likely to have a cell injury in the future.

### [DNA transfected animals]

The present invention provides a non-human mammal bearing an exogenous DNA encoding the MIP-3α of the present invention (hereinafter, abbreviated as the exogenous DNA of the present invention) or its variant DNA (sometimes abbreviated as the exogenous variant DNA of the present invention).

That is, the present invention provides:
1) A non-human mammal bearing the exogenous DNA of the present invention or its variant DNA;
2) The mammal according to 1), wherein the non-human mammal is a rodent;
3) The mammal according to 2), wherein the rodent is mouse or rat; and,
4) A recombinant vector bearing the exogenous DNA of the present invention or its variant DNA and capable of expressing in a mammal.

The non-human mammal bearing the exogenous DNA of the present invention or its variant DNA (hereinafter, abbreviated as the DNA transgenic animal of the present invention) can be prepared by transferring a desired DNA into an unfertilized egg, a fertilized egg, a spermatozoon, a germinal cell containing a primordial germinal cell thereof, or the like, preferably in the embryogenic stage in the development of a non-human mammal (more preferably in the single cell or fertilized cell stage and generally before the 8-cell phase), by the calcium phosphate method, the electric pulse method, the lipofection method, the aggregation method, the microinjection method, the particle gun method, the DEAE-dextran method or the like. In addition, it is possible to transfer the desired exogenous DNA of the present invention into a somatic cell, a living organ, a tissue cell, or the like by the DNA transfer methods, and utilize them for cell culture, tissue culture, etc. In addition, these cells may be fused with the above-described germinal cell by a per se known cell fusion method to create the DNA transgenic animal of the present invention.

Examples of the non-human mammal that can be used include bovine, swine, sheep, goats, rabbits, dogs, cats, guinea pigs, hamsters, mice, rats, and the like. Above all, preferred are rodents, especially mice (e.g., C57BL/6 strain, DBA2 strain, etc. for a pure line and, B6C3F₁ strain, BDF₁ strain, B6D2F₁ strain, BALB/c strain, ICR strain, etc. for a cross line) or rats (e.g., Wistar, SD, etc.), since they are relatively short in ontogeny and life cycle from a standpoint of creating disease model animals and are easy to breed.

"Mammals" in a recombinant vector that can be expressed in the mammals include the aforementioned non-human mammals and human, etc.

The exogenous DNA of the present invention refers to the DNA of the present invention that is once isolated/extracted from mammals, not the DNA of the present invention inherently possessed by the non-human mammals .

The variant DNA of the present invention includes those resulting from variation (e.g., mutation, etc.) in the base sequence of the original DNA of the present invention, specifically DNAs resulting from base addition, deletion, substitution with other bases, etc. and further including abnormal DNA.

The abnormal DNA is intended to mean such a DNA that expresses the abnormal MIP-3α of the present invention and exemplified by the DNA that expresses a protein to suppress the functions of the normal MIP-3α of the present invention.

The exogenous DNA of the present invention may be any one of those derived from a mammal of the same species as, or a different species from, the mammal as the target animal. In transferring the DNA of the present invention into the target animal, it is generally advantageous to use the DNA as a DNA construct in which the DNA is ligated downstream from a promoter capable of expressing the DNA in the animal cells. For example, in the case of transferring the human DNA of the present invention, a DNA-introduced mammal that expresses the DNA of the present invention to a high level, can be prepared by microinjecting a DNA construct (e.g., vector, etc.) ligated with the human DNA of the present invention into a fertilized egg of the target mammal, for example mouse, downstream various promoters, which are capable of expressing the DNA derived from various mammals (e.g., rabbits, dogs, cats, guinea pigs, hamsters, rats, mice, etc.) bearing the DNA of the present invention highly homologous to the human DNA.

As expression vectors for the MIP-3α of the present invention, there are *Escherichia coli*-derived plasmids, *Bacillus* subtilis-derived plasmids, yeast-derived plasmids, bacteriophages such as λ phage, retroviruses such as Moloney leukemia virus, and animal viruses such as vaccinia virus, baculovirus, etc. Of these vectors, *Escherichia coli*-derived plasmids, *Bacillus subtilis*-derived plasmids, or yeast-derived plasmids, etc. are preferably used.

Examples of these promoters for regulating the DNA expression include 1) promoters for the DNA derived from viruses (e.g., simian virus, cytomegalovirus, Moloney leukemia virus, JC virus, breast cancer virus, poliovirus, etc.), and 2) promoters derived from various mammals (human, rabbits, dogs, cats, guinea pigs, hamsters, rats, mice, etc.), for example, promoters of albumin, insulin II, uroplakin II, elastase, erythropoietin, endothelin, muscular creatine kinase, glial fibrillary acidic protein, glutathione S-transferase, platelet-derived growth factor β, keratins K1, K10 and K14, collagen types I and II, cyclic AMP-dependent protein kinase β I subunit, dystrophin, tartrate-resistant alkaline phosphatase, atrial natriuretic factor, endothelial receptor tyrosine kinase (generally abbreviated as Tie2), sodium-potassium adenosine triphosphorylase (Na, K-ATPase), neurofilament light chain, metallothioneins I and IIA, metalloproteinase I tissue inhibitor, MHC class I antigen (H-2L), H-ras, renin, dopamine β-hydroxylase, thyroid peroxidase (TPO), peptide chain elongation factor 1α (EF-1α) , β actin, α and β myosin heavy chains, myosin light chains 1 and 2, myelin base protein, thyroglobulins, Thy-1, immunoglobulins, H-chain variable region (VNP), serum amyloid component P, myoglobin, troponin C, smooth muscle α actin, preproencephalin A, vasopressin, etc. Among them, cytomegalovirus promoters, human peptide chain elongation factor 1α (EF-1α) promoters, human and chicken β actin promoters etc., which enable high expression systemically in the whole body, are preferred.

It is preferred that the vectors described above have a sequence for terminating the transcription of the desired messenger RNA in the DNA-introduced animal (generally referred to as a terminator); for example, a sequence of each DNA derived from viruses and various mammals. SV40 terminator of the simian virus and the like, are preferably used.

In addition, for the purpose of increasing the expression of the desired exogenous DNA to a higher level, the splicing signal and enhancer region of each DNA, a portion of the intron of a eukaryotic DNA may also be ligated at the 5' upstream of the promoter region, or between the promoter region and the translational region, or at the 3' downstream of the translational region, depending upon purposes.

The normal translational region in the MIP-3α of the present invention can be acquired as whole genomic DNA or portion thereof from liver-, kidney-, thyroid cell-, or fibroblast-derived DNA of human or various mammals (e.g., rabbits, dogs, cats, guinea pigs, hamsters, rats, mice, etc.) and commercially available various genomic DNA libraries, or from a complementary DNA as a source, which is prepared by per se known methods from liver-, kidney-, thyroid cell-, or fibroblast-derived RNA. Alternatively, the exogenous abnormal DNA can be prepared by mutating the translational region in the normal protein, which is obtained from the above cells or tissues, to variant translational region using point mutagenesis.

The translational region can be prepared as a DNA construct that can be expressed in the transgenic animal by an ordinary DNA engineering method, wherein the DNA is ligated downstream from the above-mentioned promoters (and if desired, upstream transcription termination site).

The exogenous DNA of the present invention is transferred at the fertilized egg cell stage in a manner such that the DNA is certainly present in all the germinal cells and somatic cells of the target mammal. The fact that the exogenous DNA of the present invention is present in the germinal cells of the animal prepared by DNA transfer means that all offspring of the prepared animal will carry the exogenous DNA of the present invention in all of the germinal cells and somatic cells thereof. The offspring of the animal that inherits the exogenous DNA of the present invention also have the exogenous DNA of the present invention in all of the germinal cells and somatic cells thereof.

The non-human mammal in which the normal exogenous DNA of the present invention has been transferred can be passed as the DNA-bearing animal under ordinary breeding environment, by confirming the fact that the exogenous DNA is stably retained by mating.

By transferring the exogenous DNA of the present invention at the fertilized egg cell stage, the DNA is retained to be excess in all of the germinal and somatic cells of target mammal. The fact that the exogenous DNA of the present invention is excessively present in the germinal cells of the prepared animal after transfer means that the exogenous DNA of the present invention is excessively present in all of the germinal cells and somatic cells of offspring of the prepared animal. The offspring of the animal that inherits the exogenous DNA of the present invention have excessively the exogenous DNA of the present invention in all of the germinal cells and somatic cells thereof.

By obtaining a homozygous animal having the transferred DNA in both of homologous chromosomes and mating a male and female of the animal, all offspring can be passed to retain the DNA in excess.

A non-human mammal having the normal DNA of this invention highly expresses the normal DNA of this invention, and by promoting the function of endogenous normal DNA, the hyperfunction of the MIP-3α of this invention may ultimately occur. Thus, it can be used as a pathological model animal. For example, using a transgenic animal of the present invention, to which the normal DNA has been transferred, it is possible to elucidate the pathological mechanism of the hyperfunction of the MIP-3α of this invention and the disease involved by the MIP-3α of this invention, and to study the therapeutic method of these diseases.

In addition, because a mammal, to which the exogenous abnormal DNA of this invention is transferred, has a symptom of an increase in the free form of the MIP-3α of the present invention, it can also be utilized for a screening test for a prophylactic/therapeutic agent for a disease associated with the MIP-3α of the present invention, for example, a prophylactic/therapeutic agent for cerebrovascular disorders such as cerebral infarction, cerebral hemorrhage, and subarachnoid hemorrhage, for head trauma, and for a variety of inflammatory diseases.

In the meantime, the non-human mammal which has the exogenous abnormal DNA of this invention can be bred over generations in typical breeding environment as an animal retaining the exogenous DNA, upon confirmation of stable retention of the exogenous DNA by mating. Moreover, upon incorporation of the objective exogenous DNA in the aforementioned plasmid, it can be used as a starting material. A DNA construct containing a promoter can be prepared according to general DNA engineering technique. The transfer of the abnormal DNA of this invention in the stage of fertilized ovum is ensured to be present in every germinal cell and every somatic cell of the target mammal. The existence of abnormal DNA of this invention in the germinal cell of created animal after DNA transfer means that every germinal cell and every somatic cell in every progeny of the created animal retain the abnormal DNA of the present invention. The offspring of this kind of animal that inherited the exogenous DNA of this invention has the abnormal DNA of this invention in every germinal cell and every somatic cell. By obtaining homozygote animals having the introduced DNA in both the homologous chromosomes and mating male and female of the animals, every offspring can be bred over generations such that the DNA is retained.

A non-human mammal having the abnormal DNA of this invention highly expresses the abnormal DNA of this invention, and inhibition of the function of the endogenous normal DNA sometimes causes ultimately functionally inactive adiaphoria to the MIP-3α of this invention. Thus, it can be utilized as a pathological model animal. For example, using an animal, to which the abnormal DNA of this invention has been transferred, elucidation of the pathological mechanism of functionally inactive adiaphoria to the MIP-3α of this invention and consideration of the treatment method of these diseases can be afforded.

As a concrete possibility of use, an animal that highly expresses the abnormal DNA of this invention can be a model to clarify the functional inhibition (dominant negative action) of normal protein by abnormal protein of this invention in functionally inactive adiaphoria to the MIP-3α of this invention.

In addition, because a mammal, to which the exogenous abnormal DNA of this invention is transferred, has a symptom of an increase in the free form of the MIP-3α of the present invention, it can also be utilized for a screening test for the MIP-3α of the present invention or a prophylactic/therapeutic agent for functionally inactive adiaphoria, for example, a prophylactic/therapeutic agent for cerebrovascular disorders such as cerebral infarction, cerebral hemorrhage, and subarachnoid hemorrhage, for head trauma, and for a variety of inflammatory diseases and the like.

As a possibility of other use of the above-mentioned two kinds of the DNA transgenic animals of this invention, for example,
1) use as cell source for tissue cultivation,
2) analysis of relationship with a peptide that is specifically expressed or activated due to the MIP-3α of this invention, by a direct analysis of DNA or RNA in the tissue of the DNA transgenic animals of this invention or by analysis of peptide tissue expressed by the DNA in the tissue,
3) study of function of cell from tissue generally difficult to cultivate, by cultivating cells of tissue having a DNA by general tissue cultivation technique and using them,
4) screening of a pharmaceutical agent that enhances the function of cells by the use of the cell described in the above-mentioned 3), and
5) isolation and purification of mutant protein of this invention and production of antibody thereto, and the like.

Furthermore, clinical condition of the diseases relating to the MIP-3α of this invention, including functionally inactive adiaphoria to the MIP-3α of this invention can be investigated using the transgenic animal of this invention, and detailed pathological findings in each organ of the disease model relating to the MIP-3α of this invention can be obtained, thus contributing to the development of a new therapeutic method, and study and therapy of secondary disease due to said disease.

Establishment of cultivated cells is also possible by removing each organ from the DNA transgenic animal of this invention, followed by dicing, liberating DNA-transferred cells by a protease such as trypsin, and cultivation thereof. Moreover, characterization of a cell producing the MIP-3α of this invention, relationship with apoptosis, differentiation or propagation, or signal transduction mechanism thereof can be examined to look for abnormality therein and the like, thus providing effective research material for the elucidation of the MIP-3α of this invention and its action.

Furthermore, for the development of a therapeutic agent of diseases relating to the MIP-3α of this invention, including functionally inactive adiaphoria to the MIP-3α of this invention, by the use of the DNA transgenic animal of this invention, an effective and rapid screening method of a said therapeutic agent of the disease can be provided, using the aforementioned test method, quantification method and the like. In addition, using the DNA transgenic animal of this invention or an exogenous DNA expression vector of this invention, a DNA therapy of diseases relating to the MIP-3α of this invention can be studied and developed.

### [Knockout animal]

The present invention further provides a non-human mammal embryonic stem cell where the DNA of this invention is inactivated and non-human mammal deficient in expression of DNA of this invention.

Accordingly, the present invention provides:
1) A non-human mammal embryonic stem cell in which the DNA of the present invention is inactivated;
2) The embryonic stem cell according to 1), wherein the DNA is inactivated by introducing a reporter gene (e.g., β-galactosidase gene derived from *Escherichia coli*) *;*
3) The embryonic stem cell according to 1), which is resistant to neomycin;
4) The embryonic stem cell according to 1), wherein the non-human mammal is a rodent;
5) The embryonic stem cell according to 4), wherein the rodent is mouse;
6) A non-human mammal deficient in expressing the DNA of the present invention, wherein the DNA of the present invention is inactivated;
7) The non-human mammal according to 6), wherein the DNA is inactivated by introducing a reporter gene (e.g., β-galactosidase derived from *Escherichia coli*) therein and the reporter gene is capable of being expressed under control of the promoter to the DNA of the present invention;
8) The non-human mammal according to 6), which is a rodent;
9) The non-human mammal according to 8), wherein the rodent is mouse; and
10) A method for screening of a compound or salt thereof that promotes or inhibits the promoter activity for the DNA of the present invention, which comprises administering a test compound to the animal of 7) and detecting expression of the reporter gene.

The non-human mammal embryonic stem cell where the DNA of this invention is inactivated means an embryonic stem cell (hereinafter to be briefly referred to as ES cell) of a non-human mammal, wherein the DNA does not substantially have the expression capability of the MIP-3α of this invention (hereinafter sometimes to be referred to as knockout DNA of the present invention), which is achieved by artificially introducing a mutation to the DNA of this invention possessed by the non-human mammal to suppress expression capability of DNA, or by substantially obliterating the activity of the MIP-3α of this invention that the DNA codes for.

As the non-human mammal, those similar to the aforementioned can be used.

As a method for artificially introducing a mutation to the DNA of the present invention, for example, a part or the entire DNA sequence can be deleted, or different DNA can be inserted or substituted by genetic engineering technique. With these mutations, a knockout DNA of this invention can be prepared, for example, by shifting the reading frame of codon or destroying the function of promoter or exon.

Specific examples of the non-human mammal embryonic stem cell where the DNA of this invention is inactivated (hereinafter to be briefly referred to as DNA-inactivated ES cell of this invention or knockout ES cell of this invention) can be obtained as follows. First, the DNA of this invention that the objective non-human mammal possesses is isolated, and a drug resistant gene represented by neomycin resistant gene and hygromycin resistant gene, a reporter gene represented by lacZ (β-galactosidase gene) and cat (chloramphenicol acetyl transferase gene) or the like is inserted into the exon portion of the DNA to destroy its function, or a DNA sequence (e.g., poly A-adding signal and the like) that terminates transcription of gene into an intron portion between the exons, in order to construct a DNA chain (hereinafter to be briefly referred to as targeting vector) having a DNA sequence constructed to consequently destroy gene by preventing synthesis of complete messenger RNA. Then, the DNA chain is transferred into the chromosome of the animal by, for example, homologous recombination. The knockout ES cell of the present invention is selected by analyzing the obtained ES cell by southern hybridization analysis using the DNA sequence on the DNA of this invention or in the vicinity thereof as a probe, or by PCR using the DNA sequence on a targeting vector, and a DNA sequence in the vicinity of the DNA that is other than the DNA of the present invention and is used for preparation of the targeting vector, as primers.

The original ES cell in which the DNA of this invention is inactivated by homologous recombination and the like, may be already established one as aforementioned, or even a new ES cell established according to the known method of Evans and Kaufma. For example, in the case of mouse ES cell, ES cell of 129 strain is generally used at present. However, since the immunological background of the cell of 129 strain is unclear, the ES cell which is established by the use of C57BL/6 mouse, BDF1 mouse (F1 of C57BL/6 and DBA/2) that has been established by crossing C57BL/6 with DBA/2 to increase the number of eggs obtained from C57BL/6, and the like can be also alternatively used, with the aim of obtaining an ES cell from pure strain and having clear immunologically and genetic background, and the like. BDF1 mouse advantageously produces many eggs that are strong, and in addition, it is derived from C57BL/6 mouse. Therefore, when a pathological model mouse is created from ES cell obtained from the BDF1 mouse, it is advantageous that the genetic background of the BDF1 mouse can be changed to that of C57BL/6 mouse by backcrossing with C57BL/6 mouse.

For the establishment of ES cell, blastocyst at day 3.5 after fertilization is generally used. In addition, many early embryos can be efficiently obtained by collecting an 8 cell embryo and cultivating it up to blastocyst.

While either male or female ES cell can be used, generally, male ES cell is more convenient for creating a chimera of germ line than a female one. For eliminating complicated cultivation procedure, moreover, it is desirable to judge the sexuality of the cell as early as possible.

A method for judging sexuality of ES cells comprises, for example, a method comprising amplifying and detecting a gene in the sex determining region on Y chromosome by PCR. Using this method, the number of ES cells as small as one colony (about 50) is enough for karyotype analysis, though conventional method required about 10⁶ cells. Thus, primary selection of ES cells in the early stage of cultivation can be made based on judgment of sexuality. The selection of male cells in the early stage drastically reduces labor in the early stage of the cultivation.

The secondary selection can be made by, for example, confirmation of the number of chromosomes by G-binding method and the like. Although the number of chromosomes in the obtained ES cells is desirably 100% of the normal number, if it is difficult to achieve 100% due to physiological manipulation for establishment and the like, the gene of ES cell is desirably re-cloned into a normal cell (e.g., cell wherein number of chromosome is 2n = 40 for mouse) after the knocking out.

Although the embryonic stem cell line obtained in this manner generally shows highly superior proliferation performance, careful subculture thereof is necessary, because it easily loses the ability of ontogeny. For example, the cell is cultivated according to a method comprising cultivation on a suitable feeder cell such as STO fibroblast in the presence of LIF (1 - 10000 U/ml) in a CO₂ culture vessel (preferably 5% CO₂, 95% air or 5% oxygen, 5% CO₂, 90% air) at about 37°C, or other method, and for subculture, for example, a method is employed which comprises a treatment with a trypsin/EDTA solution (generally 0.001 - 0.5% trypsin/0.1 - 5 mM EDTA, preferably about 0.1% trypsin/l mM EDTA) to give a single cell, and seeded on a newly prepared feeder cell, and the like. Such subculture is done generally every 1 to 3 days. On this occasion, the cells are observed and when a morphologically abnormal cell is found, the cultivated cell is desirably discarded.

ES cells can be differentiated to various types of cells of, for example, vertex muscle, visceral muscle, cardiac muscle and the like by single layer cultivation until they reach high density or by float cultivation until cell agglomeration is formed under suitable conditions [M. J. Evans and M. H. Kaufman, Nature, vol. 292, p. 154 (1981); G. R. Martin, Proceedings of the National Academy of Sciences. U.S.A., vol. 78, p. 7634 (1981); T. C. Doetschman et. al., Journal of Embryology and Experimental Morphology, vol. 87, p. 27 (1985)]. The cell deficient in expression of the DNA of the present invention, which is obtained by differentiating the ES cell of the present invention, is useful for the cell biological investigation of the MIP-3α of this invention *in vitro.*

Non-human mammal deficient in expression of the DNA of this invention can be distinguished from normal animals by measuring and indirectly comparing the expression level of the mRNA of said animal by a known method.

As the non-human mammal, those similar to the aforementioned can be used.

The non-human mammal which is deficient in the expression of DNA of the present invention can be produced as follows. For example, a targeting vector prepared as mentioned above is introduced into a mouse embryonic stem cell or mouse ovum, and as a result of the introduction, a DNA sequence in the targeting vector in which the DNA of this invention is inactivated, is replaced with the DNA of this invention on the chromosome of the mouse embryonic stem cell or mouse ovum, by homologous recombination, whereby the DNA of this invention can be knocked out.

The cell wherein the DNA of this invention is knocked out can be judged by southern hybridization analysis using a DNA sequence on the DNA of this invention or in the vicinity thereof as a probe, or by PCR using, as primers, the DNA sequence on a targeting vector and a DNA sequence in the vicinity that is other than the DNA of the present invention derived from mouse and was used as the targeting vector. When a non-human mammal embryonic stem cell is used, a cell line wherein the DNA of this invention is inactivated by gene homologous recombination is cloned, and the cells are injected at a suitable stage, for example, into 8 cell embryo or blastocyst of non-human mammal, and the chimeric embryo prepared is transplanted into the uterus of the pseudopregnant non-human mammal. The created animal is a chimeric animal consisting of cells having a normal locus of the DNA of the present invention and cells having locus of artificially mutated DNA of the present invention. When part of the germ cells of the chimeric animal has the locus of mutant DNA of the present invention, such chimeric individual and a normal individual are mated to give individual group, from which an individual whose entire tissues consist of cells having the locus of DNA of the present invention in which artificial mutation was added, can be obtained by, for example, judgment of coat color and the like. The thus-obtained individual is generally a heterozygote which is deficient in the expression of the MIP-3α of this invention. The heterozygotes, which is deficient in the expression of the MIP-3α of this invention are mated each other and the homozygote which is deficient in the expression of the MIP-3α of this invention, can be obtained from their offspring.

When an ovum is used, for example, a transgenic non-human mammal incorporating a targeting vector in chromosome can be obtained by injecting a DNA solution into an ovum nucleus by a microinjection method, and selecting one that has a mutation in the locus of DNA of this invention by gene homologous recombination, as compared to such transgenic non-human mammal.

An individual in which the DNA of this invention is knocked-out can be bred over generations in ordinary breeding environment, upon confirmation of knocked-out of said DNA in the individual animal obtained by mating.

Moreover, establishment and maintenance of germ line can be performed by following the conventional methods. That is, a homozygote animal having said inactivated DNA in both the homologous chromosomes can be obtained by mating male and female animal retaining said inactivated DNA. The homozygote animal thus obtained can be reproduced efficiently by breeding in the state where normal individual is 1 and homozygote are plural relative to a mother animal. By mating male and female heterozygote animals, homozygote and heterozygote animals having said inactivated DNA can be bred over generations.

Non-human mammal embryonic stem cell wherein the DNA of this invention is inactivated is highly useful for creating the non-human mammal deficient in expression of DNA of the present invention.

In addition, the non-human mammal deficient in expression of the DNA of this invention lacks various biological activities that can be induced by the MIP-3α of this invention. Since it can be a model of the disease caused by inactivation of the biological activity of the MIP-3α of this invention, it is useful for the investigation of the cause of such disease and consideration of the treatment methods.

### (a) A method for screening of compounds having therapeutic and/or prophylactic effects for diseases caused by deficiency, damages, etc. of the DNA of the present invention

The non-human mammal deficient in expression of the DNA of the present invention can be used to screen the compounds having therapeutic and/or prophylactic effects for diseases caused by deficiency, damages, and the like of the DNA of the present invention.

That is, the present invention provides a method for screening of a compound or salt thereof having therapeutic and/or prophylactic effects for diseases caused by deficiency, damages, etc. of the DNA of the present invention which comprises administering a test compound to the non-human mammal deficient in expression of the DNA of the present invention, and observing and measuring a change occurred in the animal.

As the non-human mammal deficient in expression of the DNA of the present invention used for the screening method, the same examples as given hereinabove can be used.

Examples of the test compounds include, for example, peptides, proteins, non-peptide compounds, synthetic compounds, fermentation products, cell extracts, vegetable extracts, animal tissue extracts, blood plasma, etc. and these compounds may be novel compounds or per se known compounds.

Specifically, the non-human mammal deficient in the expression of the DNA of the present invention is treated with a test compound, comparison is made with an untreated animal for control and a change in each organ, tissue, disease conditions, etc. of the animal is used as an indicator to assess the therapeutic and/or prophylactic effects of the test compound.

For treating a test animal with a test compound, for example, oral administration, intravenous injection, etc. can be applied and the treatment can be appropriately selected depending upon conditions of the test animal, properties of the test compound, etc. Furthermore, the amount of a test compound administered can be appropriately selected depending on administration route, nature of the test compound, or the like.

For example, when screening for a compound having a therapeutic/prophylactic effect on cerebrovascular disorders such as cerebral infarction, cerebral hemorrhage, and subarachnoid hemorrhage, on head trauma, and on various inflammatory diseases and the like, a test compound is administered to a non-human mammal deficient in the expression of the DNA of the present invention, the animal is examined for behavior, infarction volume and the like over time, and for symptoms of the above-described diseases. In the screening method, when a test compound is administered to a test animal, the test compound can be selected as a compound having the prophylactic and/or therapeutic effect against the above-mentioned diseases if the conditions thereof of the test animal improved about 10% or more, preferably about 30% or more, more preferably about 50% or more.

The compound obtained using the screening methods is a compound selected from the test compounds described above and can be used as safe and less toxic medicines such as a therapeutic and/or prophylactic agent for the diseases caused by deficiencies, damages, etc. of the MIP-3α of the present invention. Furthermore, compounds derived from such a compound obtained by the above screening can be used as well.

The compound obtained by the screening method may be in the form of salts. As the salts of the compound, there may be used salts with physiologically acceptable acids (e.g., inorganic acids, organic acids, etc.) or bases (e.g., alkali metal, etc.), preferably physiologically acceptable acid addition salts. Examples of such salts are salts with inorganic acids (e.g., hydrochloric acid, phosphoric acid, hydrobromic acid, sulfuric acid, etc.), salts with organic acids (e.g., acetic acid, formic acid, propionic acid, fumaric acid, maleic acid, succinic acid, tartaric acid, citric acid, malic acid, oxalic acid, benzoic acid, methanesulfonic acid, benzenesulfonic acid, etc.) and the like.

A medicine comprising the compound or salts thereof obtained by the screening methods can be formulated in the same manner as in pharmaceutical agents containing the MIP-3α of the present invention. Since the preparation thus obtained is safe and less toxic, it can be administered to humans and mammals (e.g., rats, mice, guinea pigs, rabbits, sheep, pigs, bovines, horses, cats, dogs, monkeys, etc.).

The dose of the compound or a salt thereof varies depending on target disease, subject of administration, route of administration and the like; when given by oral administration for the treatment of a cerebrovascular disorder, for example, the compound is normally administered at about 0.1 to 100 mg, preferably about 1.0 to 50 mg, and more preferably about 1.0 to 20 mg, per day, in an adult patient (60 kg body weight) . In the case of parenteral administration, the dose of the compound per dose varies depending on subject of administration, target disease and the like; when given in the form of an injection, for example, to an adult patient (60 kg body weight) with a cerebrovascular disorder, the compound is advantageously administered at normally about 0.01 to 30 mg, preferably about 0.1 to 20 mg, and more preferably about 0.1 to 10 mg, per day, by intravenous injection. In the case of other animals, a dose converted per 60 kg body weight can be administered.

### (b) A method for screening of a compound that promotes or inhibits the activities of a promoter to the DNA of the present invention

The present invention provides a method for screening of a compound or salt thereof that promotes or inhibits the activities of a promoter to the DNA of the present invention, which comprises administering a test compound to a non-human mammal deficient in expression of the DNA of the present invention and detecting expression of the reporter gene.

In the screening method described above, the non-human mammal deficient in expression of the DNA of the present invention is selected from the above-mentioned non-human mammal deficient in expression of the DNA of the present invention for an animal, in which the DNA of the present invention is inactivated by introducing a reporter gene and the reporter gene can be expressed under control of a promoter to the DNA of the present invention.

The same examples given above for the test compound apply to the test compound.

As the reporter gene, the same specific examples given above apply to the reporter gene, with β-galactosidase (lacZ), soluble alkaline phosphatase gene, luciferase gene, etc. being preferred.

In the non-human mammal deficient in expression of the DNA of the present invention wherein the DNA of the present invention is substituted with a reporter gene, the reporter gene is present under control of a promoter to the DNA of the present invention. Thus, the activity of the promoter can be detected by tracing the expression of a substance encoded by the reporter gene.

For example, when a part of the DNA region encoding the MIP-3α of the present invention is substituted with β-galactosidase gene (lacZ) derived from *Escherichia coli,* β-galactosidase is expressed in a tissue where the MIP-3_{α} of the present invention should originally be expressed, in place of the MIP-3α of the present invention. Thus, the in vivo expression state of the MIP-3α of the present invention can be readily observed in an animal, by staining with a reagent, e.g., 5-bromo-4-chloro-3-indolyl-β-galactopyranoside (X-gal), which is a substrate for β-galactosidase. Specifically, a mouse deficient in the MIP-3α of the present invention, or its tissue section is fixed with glutaraldehyde, etc. After washing with phosphate buffered saline (PBS), the system is reacted with a staining solution containing X-gal at room temperature or about 37°C for approximately 30 minutes to an hour. After the β-galactosidase reaction is terminated by washing the tissue sample with 1 mM EDTA/PBS solution, the developed color is observed. Alternatively, mRNA encoding lacZ may be detected in a conventional manner.

The compound or salts thereof obtained using the screening methods described above are compounds selected from the test compounds described above, which promote or inhibit the promoter activity for the DNA of the present invention.

The compound obtained by the screening methods may be in the form of salts. The salts of the compound are salts with physiologically acceptable acids (e.g., inorganic acids, etc.) or bases (e.g., organic acids, etc.), and physiologically acceptable acid addition salts are preferred. Examples of such salts are salts with inorganic acids (e.g., hydrochloric acid, phosphoric acid, hydrobromic acid, sulfuric acid, etc.), salts with organic acids (e.g., acetic acid, formic acid, propionic acid, fumaric acid, maleic acid, succinic acid, tartaric acid, citric acid, malic acid, oxalic acid, benzoic acid, methanesulfonic acid, benzenesulfonic acid, etc.) and the like.

Because a compound that promotes or inhibits the promoter activity on the DNA of the present invention or a salt thereof is capable of regulating the expression of the MIP-3α of the present invention, and regulating the function of the protein, it is useful as, for example, a prophylactic/therapeutic agent for cerebrovascular disorders such as cerebral infarction, cerebral hemorrhage, and subarachnoid hemorrhage, for head trauma, and for various inflammatory diseases.

Furthermore, a compound derived from a compound obtained by the above-described screening can also be used in the same manner.

A medicament containing a compound obtained by the screening method or a salt thereof can be produced in the same manner as the aforementioned medicament containing the MIP-3α of the present invention or a salt thereof.

Because the preparation thus obtained is safe and of low toxicity, it can be administered to, for example, a human or mammal (for example, rat, mouse, guinea pig, rabbit, sheep, swine, bovine, horse, cat, dog, monkey, and the like) .

The dose of the compound or a salt thereof varies depending on target disease, subject of administration, route of administration and the like; when given by oral administration, for example, the compound that inhibits the promoter activity on the DNA of the present invention is administered at about 0.1 to 100 mg, preferably about 1.0 to 50 mg, and more preferably about 1.0 to 20 mg, per day, in an adult patient (60 kg body weight) with cerebral stroke. In the case of parenteral administration, one dose of the compound varies depending on subject of administration, target disease and the like; when given in the form of an injection to an adult patient (60 kg body weight) with cerebral stroke, for example, the compound that inhibits the promoter activity of the DNA of the present invention is advantageously administered by intravenous injection at normally about 0.01 to 30 mg, preferably about 0.1 to 20 mg, and more preferably about 0.1 to 10 mg, per day. In the case of other animals, a dose converted per 60 kg body weight can be administered.

As described above, the non-human mammal deficient in expression of the DNA of the present invention is extremely useful for screening a compound or salt thereof that promotes or inhibits the promoter activity to the DNA of the present invention. Therefore, it can greatly contribute for searching causes of, or developing prophylactic and/or therapeutic agents for various diseases caused by deficiency in expression of the DNA of the present invention.

Further, where so-called transgenic animal (gene-introduced animal) is prepared by using DNA, which contains a promoter region for the MIP-3α of the present invention, ligating genes encoding a variety of proteins to downstream thereof and injecting this DNA to animal's egg cell, the protein can be synthesized specifically, so that it will allow to investigate its intravital function. Furthermore, where the cell line expressing an appropriate reporter gene, which binds to the above-mentioned promoter region, leads to establish, it can be used as a screening system of low molecular weight compound having a function that specifically promotes or inhibits intravital producing ability of the MIP-3α of the present invention itself.

The MIP-3α suppressant of the present invention may be used in combination with other active ingredients. As examples of such active ingredients, thrombolytic agents (e.g., tissue plasminogen activator, urokinase and the like), anticoagulants (e.g., argatroban, warfarin and the like), factor X inhibitors, thromboxane synthase inhibitors (e.g., ozagrel and the like), antioxidants (e.g., edaravone and the like), anti-edema agents (e.g., glycerol, mannitol and the like), nerve neogenesis/regeneration promoters (e.g., Akt/PKB activators, GSK-3β inhibitors and the like), acetylcholine esterase inhibitors (e.g., donepezil, ribastigmine, galanthamine, zanapezil and the like), β amyloid protein production, secretion, accumulation, aggregation and/or sedimentation suppressants [β secretase inhibitors (e.g., compound described in WO 98/38156, compounds described in WO 02/2505, WO 02/2506, and WO 02/2512, OM99-2 (WO 01/00663)) , γ secretase inhibitory agents, β amyloid protein aggregation inhibitory agents (e.g., PTI-00703, ALZHEMED (NC-531), PPI-368 (Japanese National Phase PCT Laid-Open Publication No. 11-514333), PPI-558 (Japanese National Phase PCT Laid-Open Publication No. 2001-500852), SKF-74652 (Biochem. J. (1999), 340 (1), 283-289)), β amyloid vaccine, β amyloid decomposing enzyme and the like], brain function enhancers (e.g., aniracetam, nicergorine and the like), other therapeutic drugs for Parkinson's disease [(e.g., dopamine receptor agonists (L-dopa, bromocriptine, pergolide, talipexole, pramipexole, cabergoline, adamantadine and the like), monoamine oxidase (MAO) inhibitors (deprenyl, selegiline, remacemide, riluzole and the like), cholinolytic agents (e.g., trihexyphenidyl, biperiden and the like),), COMT inhibitors (e.g., entacapon and the like)], therapeutic drugs for amyotrophic lateral sclerosis (e.g., riluzole and the like, neurotrophic factor and the like), therapeutic drugs for hyperlipemia such as cholesterol decreasing drugs [statins (e.g., pravastatin sodium, atorvastatin, simvastain, Rosuvastatin and the like), fibrates (e.g., clofibrate and the like), squalene synthase inhibitors], therapeutic drugs for abnormal behavior, poriomania and the like associated with progression of dementia (e.g., sedatives, anti-anxiety agents), apoptosis inhibitors (e.g., CPI-1189, IDN-6556, CEP-1347 and the like), nerve differentiation/regeneration promoters (Leteprinim, Xaliproden; SR-57746-A), SB-216763 and the like), hypotensive agents, therapeutic drugs for diabetes mellitus, antidepressants, anti-anxiety drugs, non-steroidal anti-inflammatory drugs (e.g., meloxicam, tenoxicam, indomethacin, ibuprofen, celecoxib, rofecoxib, aspirin, indomethacin and the like), disease-modifying antirheumatic drugs (DMARDs), anticytokine drugs (TNF inhibitors, MAP kinase inhibitors and the like), steroidal drugs (e.g., dexamethasone, hexestrol, cortisone acetate and the like), sex hormones or derivatives thereof (e.g., progesterone, estradiol, estradiol benzoate, and the like), parathyroid hormone (PTH), calcium receptor antagonists, and the like can be mentioned. Another active ingredient and the MIP-3α suppressant of the present invention may be used in combination after being mixed according to a method known per se and formulated into one pharmaceutical composition (e.g., tablets, powders, granules, capsules (including soft capsules), solutions, injections, suppositories, sustained-release preparations and the like), or may be prepared into separate preparations and administered to the same subject simultaneously or after a time lag.

In addition, the MIP-3α suppressant of the present invention can be used in combination with other therapies, as well as with other drugs. For example, for cerebrovascular disorders, the same can be used in combination with hypothermic or low-brain-temperature therapy, surgery for removal of cerebral thrombosis/embolism, and the like, and for nerve degenerative diseases such as Alzheimer's disease and Parkinson's disease, the same can be used in combination with therapies such as nerve stem cell transplantation, but these are not to be construed as limiting.

In the description, the codes of bases and amino acids are denoted in accordance with the IUPAC-IUB Commission on Biochemical Nomenclature or by the common codes in the art, examples of which are shown below. For amino acids that may have the optical isomer, L form is presented unless otherwise indicated.
- DNA: : deoxyribonucleic acid
- cDNA: : complementary deoxyribonucleic acid
- A: : adenine
- T: : thymine
- G: : guanine
- C: : cytosine
- RNA: : ribonucleic acid
- mRNA: : messenger ribonucleic acid
- dATP: : deoxyadenosine triphosphate
- dTTP: : deoxythymidine triphosphate
- dGTP: : deoxyguanosine triphosphate
- dCTP: : deoxycytidine triphosphate
- ATP: : adenosine triphosphate
- EDTA: : ethylenediamine tetraacetic acid
- SDS: : sodium dodecyl sulfate
- Gly: : glycine
- Ala: : alanine
- Val: : valine
- Leu: : leucine
- Ile: : isoleucine
- Ser: : serine
- Thr: : threonine
- Cys: : cysteine
- Met: : methionine
- Glu: : glutamic acid
- Asp: : aspartic acid
- Lys: : lysine
- Arg: : arginine
- His: : histidine
- Phe: : phenylalanine
- Tyr: : tyrosine
- Trp: : tryptophan
- Pro: : proline
- Asn: : asparagine
- Gln: : glutamine
- pGlu: : pyroglutamic acid
- Sec: : selenocysteine

Furthermore, substituents, protecting groups and reagents often used in the present description are denoted using the following symbols.
- Me: : methyl group

- Et: : ethyl group
- Bu: : butyl group
- Ph: : phenyl group
- TC: : thiazolidin-4(R)-carboxamide group
- Tos: : p-toluenesulfonyl
- CHO: : formyl
- Bzl: : benzyl
- Cl₂Bzl: : 2,6-dichlorobenzyl
- Bom: : benzyloxymethyl
- Z: : benzyloxycarbonyl
- Cl-Z: : 2-chlorobenzyloxycarbonyl
- Br-Z: : 2-bromobenzyloxycarbonyl
- Boc: : t-butoxycarbonyl
- DNP: : dinitrophenol
- Trt: : trityl
- Bum: : t-butoxymethyl
- Fmoc: : N-9-fluorenyl methoxycarbonyl
- HOBt: : 1-hydroxybenztriazole
- HOOBt: : 3,4-dihydro-3-hydroxy-4-oxo-1,2,3-benztriazine
- HONB: : 1- hydroxy-5-norbornene-2,3-dicarboximide
- DCC: : N,N'-dicyclohexylcarbodiimide

The sequence identification numbers in the sequence listing herein show the following sequences.

### [SEQ ID NO: 1]

Shows the base sequence of human MIP-3α cDNA.

### [SEQ ID NO:2]

Shows the amino acid sequence of human MIP-3α precursor polypeptide.

### [SEQ ID NO:3]

Shows the base sequence of rat MIP-3α cDNA.

### [SEQ ID NO:4]

Shows the amino acid sequence of rat MIP-3α precursor polypeptide.

### [SEQ ID NO:5]

Shows the base sequence of mouse MIP-3α cDNA.

### [SEQ ID NO:6]

Shows the amino acid sequence of mouse MIP-3α precursor polypeptide.

### [SEQ ID NO:7]

Shows the base sequence of human CCR6 cDNA.

### [SEQ ID NO:8]

Shows the amino acid sequence of human CCR6.

### [SEQ ID NO:9]

Shows the base sequence of mouse CCR6 cDNA.

### [SEQ ID NO:10]

Shows the amino acid sequence of mouse CCR6.

### [SEQ ID NO:11]

Shows the base sequence of an oligonucleotide designed to function as a primer for amplifying a fragment of the rat MIP-3α gene transcription product.

### [SEQ ID NO:12]

Shows the base sequence of an oligonucleotide designed to function as a primer for amplifying a fragment of the rat MIP-3α gene transcription product.

### [SEQ ID NO: 13]

Shows the base sequence of rat kidney-derived CCR6 cDNA.

### [SEQ ID NO:14]

Shows the amino acid sequence of rat CCR6.

### [SEQ ID NO:15]

Shows the base sequence of rat liver-derived CCR6 cDNA.

### [SEQ ID NO:16]

Shows the base sequence of an oligonucleotide designed to function as a primer for amplifying rat kidney-derived CCR6 cDNA.

### [SEQ ID NO:17]

Shows the base sequence of an oligonucleotide designed to function as a primer for amplifying rat kidney or rat liver-derived CCR6 cDNA.

### [SEQ ID NO:18]

Shows the base sequence of an oligonucleotide designed to function as a primer for amplifying rat liver-derived CCR6 cDNA.

### [SEQ ID NO:19]

Shows the base sequence of an oligonucleotide designed to function as a primer for amplifying a fragment of the rat CCR6 gene product.

### [SEQ ID NO:20]

Shows the base sequence of an oligonucleotide designed to function as a primer for amplifying a fragment of the rat CCR6 gene product.

### [SEQ ID NO:21]

Shows the base sequence of an oligonucleotide designed to function as a primer for amplifying a fragment of the rat CCR6 gene product.

The present invention is hereinafter described in more detail by means of the following Examples, which examples, however, are not to be construed as limiting the scope of the present invention.

### Example 1 Increase in the expression of the MIP-3α gene and decrease in the expression by hypothermic treatment in rat model of cerebral ischemia

The presence or absence of an increase in the expression of the MIP-3α gene and a decrease in the expression by hypothermic treatment in brain tissue was examined in a rat model of localized cerebral ischemia. For the cerebral ischemic model, a middle cerebral artery occlusion model was developed using 8-week-old male SD strain rats (Charles River Japan Inc.) (Kiyota et al., Experimental Brain Research, Vol. 95, pp. 388-396, 1993). Specifically, a silicone-coated plug was inserted from the right common carotid artery to the middle cerebral artery origin under halothane anesthesia, and the middle cerebral artery was occluded for 120 minutes. Subsequently, at 0, 2, 4, 6, 8, 24, 48, and 96 hours after the start of reperfusion by plug removal, five rats per group were killed, their whole brains were removed, and the infarction centers and peripheries were collected; thereafter, these specimens were combined together into one sample for each group. For the hypothermic treatment group as well, local cerebral ischemia was followed by low-temperature treatment (the inside the cage was cooled with cold wind to keep a brain temperature of 35°C) started simultaneously with the start of reperfusion, and as with the ischemia group, their whole brains were removed at 2, 4, 6, 8, 24, 48, and 96 hours after the start of reperfusion, and these specimens were combined together into one sample for each group. After each brain tissue sample described above was milled under cooling with liquid nitrogen, total RNA was purified from the milled wet tissue using ISOGEN (Nippon Gene) as directed in the attached manual. After contaminating genomic DNA was removed using a Message Clean kit (GenHunter), single-stranded cDNA was synthesized using a Thermoscript RT-PCR kit (GIBCO BRL) . The expression amount of the MIP-3α gene was analyzed by quantitative PCR on the basis of the thus-obtained cDNA. Specifically, a quantitative PCR primer set for the MIP-3α gene was designed using the Primer Express (Applied Biosystems), and two selected kinds of oligonucleotides (SEQ ID NO:11: 5'-AGAATGGCCTGCAAGCATCT-3'; SEQ ID NO:12: 5'-TGCAGAGGTAAGCCAGCAGTA-3') were synthesized (outsourced to Proligo Japan). An MIP-3α gene fragment amplified using the above-described primer set was used as the reference sequence for quantitation. The quantitative PCR reaction system used comprised the above-described primer set and a PCR QuantiTect SYBR Green PCR master kit (QIAGEN). Analysis was performed using the ABI Prism 7000 (Applied Biosystems) with the aforementioned rat cDNA per reaction derived from 0.8 ng of polyA⁺ RNA, or 0 to 10⁶ copies of the standard MIP-3α gene as a template. The expression amount of the MIP-3α gene was determined by calculating the MIP-3α cDNA copy number within each sample from a working curve generated using the standard MIP-3α gene. To make a correction for sample-to-sample variation, the MIP-3α gene copy number per copy of GAPDH in the template cDNA was calculated with the GAPDH copy number within the same sample calculated using a TaqMan Rodent GAPDH Control Reagent (VIC probe) (Applied Biosystems) as control. As a result, as shown in FIG. 1, both for the infarction center and for the periphery, the expression amount of the MIP-3α gene increased 2 hours after the start of reperfusion. The expression amount of the MIP-3α gene maximized at 24 hours after the start of reperfusion for the infarction center, and at 8 hours for the periphery, and thereafter decreased. In addition, it was found that the expression thereof, both for the infarction center and the periphery, is suppressed by hypothermic treatment. From the results above, MIP-3α was shown to be a gene whose expression is remarkably induced by reperfusion following ischemia and remarkably suppressed by hypothermic treatment.

### Example 2 Brain-protecting effect of anti-MIP-3α antibody in rat model of cerebral ischemia

The suppressive effect of an anti-rat MIP-3α monoclonal antibody possessing neutralizing activity (hereinafter referred to as anti-MIP-3α antibody) on cerebral infarction volume in a rat model of localized cerebral ischemia was examined by intraventricular administration. For the cerebral ischemic model, a middle cerebral artery occlusion model was developed using 8-week-old male SD strain rats (Charles River Japan Inc.) (Kiyota et al., Experimental Brain Research, Vol. 95, pp. 388-396, 1993). Specifically, a silicone-coated plug was inserted from the right common carotid artery to the middle cerebral artery origin under halothane anesthesia, and the middle cerebral artery was occluded for 120 minutes. In experiment 1, the rat was immobilized to a stereotaxic apparatus just before occlusion of the middle cerebral artery under halothane anesthesia, and anti-MIP-3α antibody (IgG1: Genzyme Techno, catalog number: 43540; 20 µg/10 µl) or control antibody (IgG1: Genzyme Techno, catalog number: 43002; 20 µg/10 µl) was injected to the lateral ventricle on the ischemia side (AP: -0.8, ML: - 1.6, DV: -4.0, from dura mater) . One day after ischemia treatment, the rat brain was removed, a frontal plane slice 2 mm in thickness was prepared, and cerebral infarction volume was determined by image analysis on a TTC stained image; the infarction volume for the anti-MIP-3α antibody administration group was significantly (P<0.05) lower than the control group (FIG. 2A). In experiment 2, control antibody or anti-MIP-3α antibody was injected intraventricularly in the same manner as above just after reperfusion from occlusion of the middle cerebral artery, and cerebral infarction volume was determined two days later; the cerebral infarction volume was significantly (P<0.05) lower for the anti-MIP-3α antibody administration group (FIG. 2B) . The above results show that cerebral infarction is suppressed by suppressing the function of MIP-3α.

### Example 3 Increase in production of MIP-3α protein and decrease in the production by hypothermic treatment in rat model of cerebral ischemia

The presence or absence of an increase in the production of the MIP-3α protein and a decrease in the production by hypothermic treatment in brain tissue was examined in a rat model of localized cerebral ischemia. For the cerebral ischemic model, a middle cerebral artery occlusion model was developed using 8-week-old male SD strain rats (Charles River Japan Inc.) (Kiyota et al., Experimental Brain Research, Vol. 95, pp. 388-396, 1993). Specifically, a silicone-coated plug was inserted from the right common carotid artery to the middle cerebral artery origin under halothane anesthesia, and the middle cerebral artery was occluded for 120 minutes. Subsequently, at 0, 4, 8, 24, 48, and 96 hours after the start of reperfusion by plug removal, five rats per group were killed, their whole brains were removed, and the infarction centers and peripheries were collected; thereafter, these specimens were combined together into one sample for each group. For the hypothermic treatment group as well, local cerebral ischemia was followed by low-temperature treatment (the inside the cage was cooled with cold wind to keep a brain temperature of 35°C) started simultaneously with the start of reperfusion, and as with the ischemia group, their whole brains were removed at 4, 8, 24, 48, and 96 hours after the start of reperfusion, and these specimens were combined together into one sample for each group. After each brain tissue sample described above was milled under cooling with liquid nitrogen, 1 ml of a lytic solution comprising CellLytic-MT Mammalian Tissue Lysis/Extraction Reagent (Sigma Ltd.) and a one-hundredth volume of a protease inhibitor cocktail (P8340; Sigma Ltd.) added thereto was added to 50 mg of each milled brain tissue, and the tissue was solubilized using Polytron. Subsequently, each solubilized tissue liquid was centrifuged at 4°C and 12,000 rpm for 5 minutes, the insoluble fraction was removed, and the supernatant liquid was used as the sample liquid. This sample liquid was subjected to the rat MIP-3α sandwich ELISA described below; the MIP-3α protein content per mg of brain tissue protein is shown in FIG. 3. The protein content in the sample liquid was quantified using a BCA protein assay kit (Pierce) as directed in the attached manual. Note that rat MIP-3α sandwich ELISA was performed using a commercially available anti-rat MIP-3α antibody (43540; Genzyme Techne) and a biotin-labeled anti-rat MIP-3α polyclonal antibody (44540; Genzyme Techne). Specifically, the anti-rat MIP-3α antibody (43540) was diluted with phosphate buffer solution (PBS) to obtain a concentration of 2 µg/ml, and this was dispensed to a 96-well ELISA plate at 100 µl per well. After the plate was allowed to stand at room temperature for 2 hours and then washed three times with a washing buffer solution (PBS containing 0.05% Tween 20, pH 7.2), a blocking buffer solution (PBS containing 1% fetal calf serum, 5% sucrose, and 0.05% sodium azide) was added at 300 µl per well, and the plate was allowed to stand at room temperature for 1 hour. Subsequently, the plate was washed three times with a washing buffer solution, 100 µl of each diluted sample liquid and 100 µl of rat MIP-3α standard liquid were added, and the plate was allowed to stand at room temperature for 2 hours. After the plate was washed three times with the washing buffer solution, a biotin-labeled anti-rat MIP-3α polyclonal antibody (44540) diluted with PBS to 50 ng/ml was added at 100 µl per well, and the plate was to stand at room temperature for 2 hours. After plate washing, streptoavidin-horseradish peroxidase was coupled to the biotin-labeled anti-rat MIP-3α polyclonal antibody as directed in the attached manual of a protein detector ELISA kit (KPL), thereafter the reaction substrate was added to each well to cause the reaction, and absorbance at 405 nm was determined. The results are shown in FIG. 3.

It was found that for both the infarction center and the periphery, the MIP-3α protein content in brain tissue maximized at 24 hours after the start of reperfusion. It was also shown that the production thereof was considerably suppressed by hypothermic treatment. From the results above, MIP-3α protein was shown to be a protein whose production is remarkably induced by reperfusion following ischemia and remarkably suppressed by hypothermic treatment.

### Example 4 Cloning and base sequencing of cDNAs that encode CC chemokine receptor 6 (CCR6)

The cDNAs that encode rat kidney-derived CCR6 and rat liver-derived CCR6, respectively, were cloned, and the base sequences thereof were determined. The cloning and base sequencing procedures for each CCR6 are shown below.

First, a PCR reaction was carried out using two kinds of primers, i.e., primer 1 (see below) and primer 2 (see below), with rat kidney Marathon-Ready cDNA (Clontech) as the template. Regarding the reaction liquid composition, 5 µl of the above-described cDNA was used as the template, and 4 µl of Advantage 2 Polymerase Mix (Clontech), 0.4 µM of each of primer 1 and primer 2, 200 µM of dNTP mixture, and 20 µl of 10x cDNA PCR Reaction Buffer (Clontech) were added to make a final volume of 200 µl. The PCR reaction was carried out by heat treatment at 94°C for 1 minute, followed by 35 repeated cycles of 94°C for 30 seconds, 67°C for 30 seconds, and 68°C for 2 minutes. After the amplified fragment was confirmed by agarose gel electrophoresis, the PCR reaction product was subcloned into the plasmid vector pCR 2.1-TOPO (Invitrogen) according to the operating directions for the TOPO TA Cloning Kit (Invitrogen). *Escherichia coli* TOP10 was transformed with this plasmid, and clones incorporating the cDNA were selected on a kanamycin-containing LB agar medium. As a result of an analysis of the base sequences of the individual clones, the cDNA sequence that encodes rat CCR6 (SEQ ID NO:13) was obtained, and the plasmid DNA was designated rCCR6-kidney. Furthermore, *Escherichia coli* DH5α (Invitrogen) was transformed with the plasmid DNA, and the resulting transformant was designated DH5α/rCCR6-kidney.

Subsequently, a PCR reaction was carried out using two kinds of primers, i.e., primer 2 (see below) and primer 3 (see below), with rat liver Marathon-Ready cDNA (Clontech) as the template. Regarding the reaction liquid composition, 5 µl of the above-described cDNA was used as the template, and 1 µl of Pyrobest DNA Polymerase (Takara Bio Inc.), 0.4 µM of each of primer 2 and primer 3, 200 µM of dNTP mixture, and 20 µl of 10x PyrobestII buffer (Takara Bio Inc.) were added to make a final volume of 200 µl. The PCR reaction was carried out by heat treatment at 94°C for 30 seconds, followed by 40 repeated cycles of 94°C for 30 seconds, 65°C for 30 seconds, and 72°C for 2 minutes. After the amplified fragment was confirmed by agarose gel electrophoresis, the PCR reaction product was subcloned into the plasmid vector pCR-BluntII-TOPO (Invitrogen) according to the operating directions for the Zero Blunt TOPO PCR Cloning Kit (Invitrogen). *Escherichia coli* TOP10 was transformed with this plasmid, and clones incorporating the cDNA were selected on a kanamycin-containing LB agar medium. As a result of an analysis of the base sequences of the individual clones, the cDNA sequence that encodes rat CCR6 (SEQ ID NO:15) was obtained, and the plasmid DNA was designated rCCR6-liver. Furthermore, *Escherichia coli* DH5_{α} (Invitrogen) was transformed with the plasmid DNA, and the resulting transformant was designated DH5α/rCCR6-liver.

The rat kidney derived CCR6 cDNA and the rat liver derived CCR6 cDNA agreed completely with each other in terms of the base sequence of the protein-coding region, and encoded a protein having an amino acid sequence consisting of 366 amino acids (SEQ ID NO: 14) . The results of a hydrophobicity plot analysis of the amino acid sequence suggested that this protein might have a seven transmembrane domain.
Primer 1: 5'-TGTATTGAAGACAGAACACTTGTGG-3' (SEQ ID NO:16)
Primer 2: 5'-TCACATGTAATAGCAAGTTTCACAAAGG-3' (SEQ ID NO:17)
Primer 3: 5'-GCATCTCACTACCCGTCTCTC-3' (SEQ ID NO:18)

### Example 5 Increase in expression of the CCR6 gene and decrease in the expression by hypothermic treatment in rat model of cerebral ischemia

The presence or absence of an increase in the expression of the CCR6 gene and a decrease in the expression by hypothermic treatment in brain tissue was examined in a rat model of localized cerebral ischemia. For the cerebral ischemic model, a middle cerebral artery occlusion model was developed using 8-week-old male SD strain rats (Charles River Japan Inc.) (Kiyota et al., Experimental Brain Research, Vol. 95, pp. 388-396, 1993). Specifically, a silicone-coated plug was inserted from the right common carotid artery to the middle cerebral artery origin under halothane anesthesia, and the middle cerebral artery was occluded for 120 minutes. Subsequently, at 0, 2, 4, 6, 8, 24, 48, and 96 hours after the start of reperfusion by plug removal, five rats per group were killed, their whole brains were removed, and the infarction centers and peripheries were collected; thereafter, these specimens were combined together into one sample for each group. For the hypothermic treatment group as well, local cerebral ischemia was followed by low-temperature treatment (the inside the cage was cooled with cold wind to keep a brain temperature of 35°C) started simultaneously with the start of reperfusion, and as with the ischemia group, their whole brains were removed, at 2, 4, 6, 8, 24, 48, and 96 hours after the start of reperfusion, and these specimens were combined together into one sample for each group. After each brain tissue sample described above was milled under cooling with liquid nitrogen, total RNA was purified from the milled wet tissue using ISOGEN (Nippon Gene) as directed in the attached manual. After contaminating genomic DNA was removed using a Message Clean kit (GenHunter), single-stranded cDNA was synthesized using a Thermoscript RT-PCR kit (GIBCO BRL) . The expression amount of the CCR6 gene was analyzed by quantitative PCR with the thus-obtained cDNA as a template. Specifically, a rat CCR6 gene fragment was amplified on the basis of the rat CCR6 gene sequence cloned in Example 4 (previous section) using two kinds of oligonucleotides (SEQ ID NO:19: 5'-GGACGATGCGTTGTCATTTTC-3'; SEQ ID NO:20: 5'-CCGCAGCTGCAGCGCCGAGAAA-3', for both, synthesis was outsourced to Proligo Japan), and was used as the reference sequence for quantitation. The quantitative PCR reaction system used comprised a quantitative PCR primer set designed using Primer Express (Applied Biosystems) (SEQ ID NO:19: 5'-GGACGATGCGTTGTCATTTTC-3', SEQ ID NO:21: 5'-GTGCCCGGGTTTACTCAGAA-3', both were synthesized on an outsourcing basis by Proligo Japan) and a PCR QuantiTect SYBR Green PCR master kit (QIAGEN). Analysis was performed using the ABI Prism 7000 (Applied Biosystems) with each rat cDNA sample in an amount equivalent to 0.8 ng of polyA⁺ RNA per reaction, or 0 to 10⁶ copies of the standard CCR6 gene fragment as the template. The expression amount of the CCR6 gene was standardized by the expression amount of the GAPDH gene within the same sample. Specifically, the CCR6 cDNA copy number within each sample was calculated from a working curve generated using the standard CCR6 gene, and at the same time the GAPDH cDNA copy number within the same sample was calculated using a TaqMan Rodent GAPDH Control Reagent (VIC probe) (Applied Biosystems), and the CCR6 cDNA copy number per copy of GAPDH cDNA was calculated. As a result, as shown in FIG. 5, the expression amount of the CCR6 gene began increasing at 4 hours after the start of reperfusion for the infarction center, and at 6 hours for the periphery. In addition, the expression amount of the CCR6 gene maximized at 48 hours after the start of reperfusion, and thereafter decreased, for both the infarction center and the periphery. Furthermore, the expression thereof was found to begin being suppressed by hypothermic treatment at 8 hours after the start of reperfusion for the infarction center, and at 24 hours after the start of reperfusion for the periphery. From the results above, the CCR6 gene was shown to be a gene whose expression is remarkably induced by reperfusion following ischemia and remarkably suppressed by hypothermic treatment.

### Industrial Applicability

Because the MIP-3α of the present invention is a diagnostic marker for brain/nerve cell injuries, for example, cerebrovascular disorders, a substance that inhibits the activity of the protein, for example, a neutralizing antibody against the protein, and a substance that inhibits the expression of the gene for the protein, for example, the antisense nucleic acid of the present invention, are useful as brain/nerve cell protective agents, especially as brain/nerve cell protective agents for cerebrovascular disorders such as cerebral infarction, cerebral hemorrhage, and subarachnoid hemorrhage, or for head trauma.

## Claims

1. A brain/nerve cell protective agent comprising a substance that suppresses a protein comprising the same or substantially the same amino acid sequence as the amino acid sequence of amino acid number 1 and after in the amino acid sequence represented by SEQ ID NO:2, 4 or 6, or a salt thereof.

2. A brain/nerve cell protective agent comprising a substance that reduces the activity of a protein comprising the same or substantially the same amino acid sequence as the amino acid sequence of amino acid number 1 and after in the amino acid sequence represented by SEQ ID NO:2, 4 or 6, or a salt thereof.

3. The brain/nerve cell protective agent of claim 2, wherein the substance that reduces the activity is a neutralizing antibody against a protein comprising the same or substantially the same amino acid sequence as the amino acid sequence of amino acid number 1 and after in the amino acid sequence represented by SEQ ID NO:2, 4 or 6, a partial peptide thereof, or a salt thereof.

4. A brain/nerve cell protective agent comprising a substance that inhibits the expression of the gene that encodes a polypeptide comprising the same or substantially the same amino acid sequence as the amino acid sequence represented by SEQ ID NO:2, 4 or 6.

5. The brain/nerve cell protective agent of claim 4, wherein the substance that inhibits the expression is a nucleic acid comprising a base sequence complementary to a base sequence that encodes a polypeptide having the same or substantially the same amino acid sequence as the amino acid sequence represented by SEQ ID NO:2, 4 or 6, or a portion thereof.

6. A diagnostic agent for a brain/nerve cell injury, which comprises an antibody against a protein comprising the same or substantially the same amino acid sequence as the amino acid sequence of amino acid number 1 and after in the amino acid sequence represented by SEQ ID NO:2, 4 or 6, a partial peptide thereof, or a salt thereof.

7. A diagnostic agent for a brain/nerve cell injury, which comprises a nucleic acid comprising a base sequence that encodes a polypeptide comprising the same or substantially the same amino acid sequence as the amino acid sequence represented by SEQ ID NO:2, 4 or 6, or a portion thereof.

8. A brain/nerve cell protective agent comprising a substance that inhibits the binding of a protein comprising the same or substantially the same amino acid sequence as the amino acid sequence of amino acid number 1 and after in the amino acid sequence represented by SEQ ID NO:2, 4 or 6, or a salt thereof, and a receptor thereof.

9. A brain/nerve cell protective agent comprising a substance that inhibits the cell stimulation activity of a receptor of a protein comprising the same or substantially the same amino acid sequence as the amino acid sequence of amino acid number 1 and after in the amino acid sequence represented by SEQ ID NO:2, 4 or 6, or a salt thereof.

10. A brain/nerve cell protective agent comprising a substance that inhibits the expression of the gene that encodes a receptor of a protein comprising the same or substantially the same amino acid sequence as the amino acid sequence of amino acid number 1 and after in the amino acid sequence represented by SEQ ID NO:2, 4 or 6, or a salt thereof.

11. The brain/nerve cell protective agent described in claims 8 to 10, wherein the receptor is a protein comprising the same or substantially the same amino acid sequence as the amino acid sequence represented by SEQ ID NO:8 or 10, or a salt thereof.

12. A method for screening of a substance that exhibits brain/nerve cell protective action, which comprises using a protein comprising the same or substantially the same amino acid sequence as the amino acid sequence of amino acid number 1 and after in the amino acid sequence represented by SEQ ID NO:2, 4 or 6, a partial peptide thereof, or a salt thereof.

13. A method for screening of a substance that exhibits brain/nerve cell protective action, which comprises using a protein comprising the same or substantially the same amino acid sequence as the amino acid sequence represented by SEQ ID NO:8 or 10, a partial peptide thereof, or a salt thereof.

14. A kit for screening of a substance that exhibits brain/nerve cell protective action, which comprises a protein comprising the same or substantially the same amino acid sequence as the amino acid sequence of amino acid number 1 and after in the amino acid sequence represented by SEQ ID NO:2, 4 or 6, a partial peptide thereof, or a salt thereof.

15. A kit for screening of a substance that exhibits brain/nerve cell protective action, which comprises a protein comprising the same or substantially the same amino acid sequence as the amino acid sequence represented by SEQ ID NO:8 or 10, a partial peptide thereof, or a salt thereof.

16. A method for screening of a substance that exhibits brain/nerve cell protective action, which comprises using a nucleic acid comprising the base sequence that encodes a polypeptide comprising the same or substantially the same amino acid sequence as the amino acid sequence represented by SEQ ID NO:2, 4 or 6, or a portion thereof.

17. A method for screening of a substance that exhibits brain/nerve cell protective action, which comprises using a nucleic acid comprising the base sequence that encodes a polypeptide comprising the same or substantially the same amino acid sequence as the amino acid sequence represented by SEQ ID NO:8 or 10, or a portion thereof.

18. A kit for screening of a substance that exhibits brain/nerve cell protective action, which comprises a nucleic acid comprising the base sequence that encodes a polypeptide comprising the same or substantially the same amino acid sequence as the amino acid sequence represented by SEQ ID NO:2, 4 or 6, or a portion thereof.

19. A kit for screening of a substance that exhibits brain/nerve cell protective action, which comprises a nucleic acid comprising the base sequence that encodes a polypeptide comprising the same or substantially the same amino acid sequence as the amino acid sequence represented by SEQ ID NO:8 or 10, or a portion thereof.

20. A method of protecting brain or nerve cells, which comprises administering a substance that suppresses a protein comprising the same or substantially the same amino acid sequence as the amino acid sequence of amino acid number 1 and after in the amino acid sequence represented by SEQ ID NO:2, 4 or 6, or salt thereof, to a subject in need thereof.

21. Use of a substance that suppresses a protein comprising the same or substantially the same amino acid sequence as the amino acid sequence of amino acid number 1 and after in the amino acid sequence represented by SEQ ID NO:2, 4 or 6, or a salt thereof, for producing a brain/nerve cell protective agent.
